# EUROPEAN PATENT APPLICATION

(11) **EP 3 693 736 A2**
(43) Date of publication of application: **12.08.2020**
(21) Application number: 19211956.8
(22) Date of filing: 16.10.2015
(51) Int. Cl.: G01N 33/50, G01N 33/68, C12N 15/113, A61P 1/00, A61K 31/7088

(54) **METHODS FOR DOSING AND MONITORING SMAD7 ANTISENSE OLIGONUCLEOTIDE TREATMENT USING BIOMARKER LEVELS**

(30) Priority: 17.10.2014 US 201462065586 P; 17.10.2014 US 201462065596 P; 01.12.2014 US 201462085949 P
(62) Divisional of application: 15785081.9
(71) Applicant: Nogra Pharma Limited, Dublin 2 (IE)
(72) Inventor: MONTELEONE, Giovanni, Grottaferrata 00046 (IT)
(74) Representative: Lau, Sarah Jane

(57) **Abstract**

Methods of treating IBD in a subject using an anti-SMAD7 therapy, such as a SMAD7 antisense oligonucleotide, to reduce CCL20, IL8, or TNFα levels are disclosed. Methods of treating and managing IBD in a subject using an anti-SMAD7 therapy, such as a SMAD7 antisense oligonucleotide, based on CCL20, IL8, or TNFα levels are also disclosed. Also disclosed are methods of determining whether a subject with IBD is responsive or likely to be responsive to treatment an anti-SMAD7 therapy. Reduction of CCL20, IL8, or TNFα levels may correlated with IBD remission or decreases in CDAI score.

## Description

This application claims the benefit of U.S. Provisional Application No. 62/065,586, filed October 17, 2014, U.S. Provisional Application No. 62/065,596, filed October 17, 2014, and U.S. provisional application 62/085,949, filed December 1, 2014, the entire contents of each of which are herein incorporated by reference.

### FIELD OF THE INVENTION

The present invention relates to methods of monitoring effectiveness of treatment or management of inflammatory bowel diseases (IBD) using a SMAD7 antisense oligonucleotide, as well as methods of regulating SMAD7 antisense oligonucleotide treatment, based on analysis of Chemokine (C-C motif) ligand 20 (CCL20) levels, Interleukin 8 (IL8) levels, and/or Tumor Necrosis Factor α (TNFα) levels.

### BACKGROUND

Inflammatory bowel disease (IBD) is a chronic inflammatory disorder of the gastrointestinal tract suffered by approximately one million patients in the United States. The two most common forms of IBD are Crohn's disease (CD) and ulcerative colitis (UC). Although CD can affect the entire gastrointestinal tract, it primarily affects the ileum (the distal or lower portion of the small intestine) and the large intestine. UC primarily affects the colon and the rectum. Current treatment for both CD and UC include aminosalicylates (*e.g.,* 5-aminosalicylic acid, sulfasalazine and mesalamine), antibiotics (*e.g.,* ciprofloxacin and metronidazole), corticosteroids (*e.g.,* budesonide or prednisone), immunosuppressants *(e.g.,* azathioprine or methotrexate) and tumor necrosis factor (TNF) antagonists (*e.g.,* infliximab (Remicade®)). Patient response to these therapies varies with disease severity and it can vary over cycles of active inflammation and remission. Moreover, many of the current therapies for IBD are associated with undesirable side effects.

Although the etiologies of CD and UC are unknown, both are considered inflammatory diseases of the intestinal mucosa. Recent studies have demonstrated that TGF-β1 acts as a potent immunoregulator able to control mucosal intestinal inflammation. TGF-β1 binds a heterodimeric transmembrane serine/threonine kinase receptor containing two subunits, TGF-β1 R1 and TGF-β1 R2. Upon ligand binding, the TGF-β1 R1 receptor is phosphorylated by the constitutively active TGF-β1 R2 receptor and signal is propagated to the nucleus by proteins belonging to the SMAD family. Activated TGF-β1 R1 directly phosphorylates SMAD2 and SMAD3 proteins, which then interact with SMAD4. The complex of SMAD2/SMAD3/SMAD4 translocates to the nucleus and modulates the transcription of certain genes.

Additional studies have demonstrated that another SMAD protein, SMAD7, also plays a role in inflammation. SMAD7, an intracellular protein, has been shown to interfere with binding of SMAD2/SMAD3 to the TGF-β1 R1 preventing phosphorylation and activation of these proteins. Further, increased expression of SMAD7 protein is associated with an inhibition of TGF-β1 mediated-signaling. Mucosal samples from IBD patients are characterized by high levels of SMAD7 and reduced levels of phosphorylated-SMAD3 indicating that TGF-β1-mediated signaling is compromised in these patients.

Recent studies have focused on SMAD7 as a target for treating patients suffering from IBD. Such therapies include anti-SMAD7 antisense therapies. As such, there is a need for methods based on predictive biomarkers that can be used to identify patients that are likely (or unlikely) to respond to treatment with anti-SMAD7 therapies and methods of evaluating treatment success.

### SUMMARY

The invention is that levels of CCL20, IL8, and/or TNFα in a patient with IBD correlate with IBD disease state and can be used as a means for monitoring disease state and managing responsiveness to IBD treatment with an anti-SMAD7 therapy. The invention is also that one can use monitoring and analysis of CCL20, IL8, and/or TNFα levels in a patient with IBD to determine appropriate levels of SMAD7 antisense oligonucleotide administration and to regulate and adjust SMAD7 antisense oligonucleotide treatment.

It will be appreciated that it is advantageous to be able to determine shortly after commencing treatment, shortly before stopping treatment, or shortly after stopping treatment, whether an IBD patient is responsive to treatment with an anti-SMAD7 therapy, in particular, a SMAD7 antisense oligonucleotide. Modulation of CCL20, IL8, and/or TNFα levels in a patient with IBD, as described herein, is useful for evaluating the efficacy of and responsiveness to treatment with an anti-SMAD7 therapy in a subject having IBD. Furthermore, it will be appreciated that it is advantageous to be able to evaluate and modulate administration of an anti-SMAD7 therapy in a patient with IBD based on levels, or changes in levels, of a biomarker, *e.g.,* CCL20, IL8, and/or TNFα, that correlate with disease state. Thus, the invention provides methods for analyzing levels of CCL20, IL8, and/or TNFα in a patient being treated with or who has been administered an anti-SMAD7 therapy, *e.g.,* a SMAD7 antisense oligonucleotide, and adjusting dosage levels based on CCL20, IL8, and/or TNFα levels or changes in CCL20, IL8, and/or TNFα levels determined by an analyzing step, following a dose of the anti-SMAD7 therapy. Advantageously, the methods of the invention will ultimately assist physicians in choosing effective therapies and monitoring and adjusting treatment with said therapies. Furthermore, methods of the invention will lead to improvements in IBD treatment efficacy for patients, with reduction in overall patient costs.

In a first aspect, the invention provides methods for treating or managing inflammatory bowel disease in a patient having IBD. In one embodiment, the method includes the following steps: (a) of administering to the patient an initial dose of a SMAD7 antisense oligonucleotide; (b) analyzing the level of CCL20, IL8, or TNFα in the patient; and (c) if the level of CCL20, IL8, or TNFα is above normal levels of CCL20, IL8, or TNFα, then administering to the patient a subsequent dose that is greater than or equal to the initial dose. Alternatively, if in step (c), the level of CCL20, IL8, or TNFα is below normal levels of CCL20, IL8, or TNFα as determined in step (b), then step (c) includes administering to the patient a subsequent dose that is equal to or smaller than the initial dose.

In some embodiments, the invention may comprise a SMAD7 antisense oligonucleotide for use in a method of treating or managing IBD. For instance, in some embodiments, the invention comprises a SMAD7 antisense-oligonucleotide for use in a method for treating or managing inflammatory bowel disease (IBD) in a patient having IBD, wherein the method comprises analyzing the level of CCL20, IL8, or TNFα in the patient to determine appropriate levels of SMAD7 antisense oligonucleotide administration. In some embodiments, the invention comprises a SMAD7 antisense-oligonucleotide for this use, wherein the method comprises the steps of: (a) administering to the patient an initial dose of the SMAD7 antisense-oligonucleotide; (b) analyzing the level of CCL20, IL8, or TNFα in the patient; and (c) if the level of CCL20, IL8, or TNFα is above normal levels of CCL20, IL8, or TNFα, then administering to the patient a subsequent dose of the SMAD7 antisense-oligonucleotide that is greater than or equal to the initial dose, or, if the level of CCL20, IL8, or TNFα is below normal levels of CCL20, IL8, or TNFα, then administering to the patient a subsequent dose of the SMAD7 antisense-oligonucleotide that is equal to or smaller than the initial dose.

In another aspect of the invention, the invention provides methods for treating or managing IBD in a patient having IBD with respect to administration of an initial dose of a SMAD7 antisense oligonucleotide. In one embodiment, the invention provides a method for treating or managing IBD in a patient having IBD, where the method includes the following steps:
(a) analyzing the level of CCL20, IL8, or TNFα in the patient; and (b) if the level of CCL20, IL8, or TNFα is above normal levels of CCL20, IL8, or TNFα, then administering to the patient an initial dose of a SMAD7 antisense oligonucleotide. In a particular embodiment, the invention provides a method for treating or managing IBD in a patient having IBD, where the method includes the following steps: (a) analyzing the level of CCL20, IL8, or TNFα in the patient; and
(b) if the level of CCL20, IL8, or TNFα is above 0.01 pg/ml, 0.1 pg/ml, 1 pg/ml 2 pg/ml, 3 pg/ml, 4 pg/ml, 5 pg/ml, 6 pg/ml, 7 pg/ml, 8 pg/ml, 9 pg/ml, 10 pg/ml, 11 pg/ml, 12 pg/ml, 13 pg/ml, 14 pg/ml, 15 pg/ml, 17.5 pg/ml, 20 pg/ml, 22.5 pg/ml, 25 pg/ml, 30 pg/ml, or 35 pg/ml, then administering to the patient an initial dose of a SMAD7 antisense oligonucleotide.

Additionally, the method may further include the steps of: (c) analyzing the level of CCL20, IL8, or TNFα in the patient after said administering step, *i.e.,* step (b); and (d) if the level of CCL20, IL8, or TNFα is above normal levels of CCL20, IL8, or TNFα, then administering to the patient a subsequent dose that is greater than or equal to the initial dose. Alternatively, if in step (d), the level of CCL20, IL8, or TNFα is below normal levels of CCL20, IL8, or TNFα, as determined in step (c), then step (d) includes administering to the patient a subsequent dose that is equal to or smaller than the initial dose. In some instances, if the subsequent dose administered in step (d) is equal to or greater than the maximum tolerated dose (MTD), then the method includes the step of terminating the treatment.

In some embodiments, the invention comprises methods of treating or managing IBD, dependent upon establishment of a control level of CCL20, IL8, or TNFα. For example, in a particular embodiment, the method for treating or managing IBD in a patient having IBD includes the steps of (a) establishing a control level of CCL20, IL8, or TNFα for the patient; (b) administering to the patient an initial dose of a SMAD7 antisense-oligonucleotide; (c) analyzing the level of CCL20, IL8, or TNFα in the patient; and (d) if the level of CCL20, IL8, or TNFα is lower than the control level, then administering to the patient a subsequent dose that is the same as the initial dose or smaller than the initial dose. Alternatively, if the level of CCL20, IL8, or TNFα determined in step (c) is unchanged or increased compared to the control level, then the method includes a step (d) of administering to the patient a subsequent dose that is the same as the initial dose or greater than the initial dose or terminating the treatment.

In some embodiments, the method comprises the steps of (a) analyzing a first level of any of CCL20, IL8, or TNFα in the patient; (b) administering to the patient an initial dose of a SMAD7 antisense oligonucleotide (AON); and (c) analyzing a second level of CCL20, IL8, or TNFα in the patient after the administering step. In an embodiment of the invention, if the second level of CCL20, IL8, or TNFα is the same or higher than the first level of CCL20, IL8, or TNFα, then: administering to the patient a subsequent dose that is equal to or greater than the initial dose, and/or administering to the patient a subsequent dose at an equal or higher frequency than the initial dose. Alternatively, if the second level of CCL20, IL8, or TNFα is lower than the first level of CCL20, IL8, or TNFα, then administering to the patient a subsequent dose that is equal to or smaller than the initial dose, and/or administering to the patient a subsequent dose at an equal or lower frequency than the initial dose.

In some embodiments of the invention, the second level of CCL20, IL8, or TNFα is higher than the first level of CCL20, IL8, or TNFα. For example, in some embodiments, the second level of CCL20, IL8, or TNFα is about 10% higher, about 20% higher, about 30% higher, about 40% higher, about 50% higher, about 60% higher, about 70% higher, about 80% higher, about 90% higher, about 100% higher, or more than the first level of CCL20, IL8, or TNFα. In some embodiments, the second level of CCL20, IL8, or TNFα is about 10% to about 20% higher , about 20% to about 30% higher, about 30% to about 40% higher, about 40% to about 50% higher, about 50% to about 60% higher, about 60% to about 70% higher, about 70% to about 80% higher, about 80% to about 90% higher, or about 90% to about 100% higher than the first level of CCL20, IL8, or TNFα. Alternatively, in some embodiments, the second level of CCL20, IL8, or TNFα is lower than the first level of CCL20, IL8, or TNFα. For example, in some embodiments, the second level of CCL20, IL8, or TNFα is about 10% lower, about 20% lower, about 30% lower, about 40% lower, about 50% lower, about 60% lower, about 70% lower, about 80% lower, about 90% lower, or about 100% lower than the first level of CCL20, IL8, or TNFα. In some embodiments, the second level of CCL20, IL8, or TNFα is about 10% to about 20% lower, about 20% to about 30% lower, about 30% to about 40% lower, about 40% to about 50% lower, about 50% to about 60% lower, about 60% to about 70% lower, about 70% to about 80% lower, about 80% to about 90% lower, or about 90% to about 100% lower than the first level of CCL20, IL8, or TNFα.

In some embodiments, the invention comprises a method for treating or managing IBD in a patient having IBD, wherein the method comprises the steps of (a) administering to the patient an initial dose of a SMAD7 antisense-oligonucleotide; and (b) analyzing the level of CCL20, IL8, or TNFα in the patient after the administering step. In some embodiments, if the level of CCL20, IL8, or TNFα is above normal levels of CCL20, IL8, or TNFα, then the patient is administered a subsequent dose that is greater than or equal to the initial dose, and/or administering to the patient a subsequent dose at an equal or higher frequency than the initial dose. In some embodiments, if the level of CCL20, IL8, or TNFα is below normal levels of CCL20, IL8, or TNFα, then the patient is administered a subsequent dose that is equal to or smaller than the initial dose and/or administering to the patient a subsequent dose at an equal or lower frequency than the initial dose.

In some embodiments of the invention, the level of CCL20, IL8, or TNFα is higher than the normal level of CCL20, IL8, or TNFα. For example, in some embodiments, the level of CCL20, IL8, or TNFα is about 10% higher, about 20% higher, about 30% higher, about 40% higher, about 50% higher, about 60% higher, about 70% higher, about 80% higher, about 90% higher, about 100% higher, or more than the normal level of CCL20, IL8, or TNFα. In some embodiments, the level of CCL20, IL8, or TNFα is about 10% to about 20% higher , about 20% to about 30% higher, about 30% to about 40% higher, about 40% to about 50% higher, about 50% to about 60% higher, about 60% to about 70% higher, about 70% to about 80% higher, about 80% to about 90% higher, or about 90% to about 100% higher than the normal level of CCL20, IL8, or TNFα. In some embodiments, the level of CCL20, IL8, or TNFα is lower than the normal level of CCL20, IL8, or TNFα. For example, in some embodiments, the level of CCL20, IL8, or TNFα is about 10% lower, about 20% lower, about 30% lower, about 40% lower, about 50% lower, about 60% lower, about 70% lower, about 80% lower, about 90% lower, or about 100% lower than the normal level of CCL20, IL8, or TNFα. In some embodiments, the second level of CCL20, IL8, or TNFα is about 10% to about 20% lower, about 20% to about 30% lower, about 30% to about 40% lower, about 40% to about 50% lower, about 50% to about 60% lower, about 60% to about 70% lower, about 70% to about 80% lower, about 80% to about 90% lower, or about 90% to about 100% lower than the normal level of CCL20, IL8, or TNFα.

In some embodiments, the invention comprises a SMAD7 antisense-oligonucleotide for use in a method for treating or managing IBD in a patient having IBD, wherein the method comprises (a) analyzing the level of CCL20, IL8, or TNFα in the patient; and (b) if the level of CCL20, IL8, or TNFα is above normal levels of CCL20, IL8, or TNFα, then administering to the patient an initial dose of the SMAD7 antisense-oligonucleotide.

The level of CCL20, IL8, or TNFα may be analyzed at varying time points following an administering step (b). For instance, in some embodiments, following an administering step (b), the level of CCL20, IL8, or TNFα is analyzed at least 1 day, at least 3 days, at least 5 days, at least 1 week, at least 2 weeks, at least 3 weeks, at least 1 month, at least 2 months, at least 4 months, or at least 6 months after said administration step. In some embodiments, the level of CCL20, IL8, or TNFα is analyzed immediately after said administration step. In yet other embodiments, the level of CCL20, IL8, or TNFα is analyzed about 7 days, about 10 days, about 15 days, about 20 days, about 25 days, or about 28 days after said administration step.

Normal levels or a control level of CCL20, IL8, or TNFα may be determined based on numerical reference values or with respect to levels of CCL20, IL8, or TNFα in a healthy control group. For instance, in some embodiments, a control level or normal levels of CCL20, IL8, or TNFα are about 0.01 pg/ml, about 0.1 pg/ml, about 1 pg/ml, about 2 pg/ml, about 3 pg/ml, about 4 pg/ml, about 5 pg/ml, about 6 pg/ml, about 7 pg/ml, about 8 pg/ml, about 9 pg/ml, about 10 pg/ml, about 11 pg/ml, about 12 pg/ml, about 13 pg/ml, about 14 pg/ml, about 15 pg/ml, about 16 pg/ml, about 17 pg/ml, about 17.5 pg/ml, about 18 pg/ml, about 19 pg/ml, about 20 pg/ml, about 22.5 pg/ml, about 25 pg/ml, about 30 pg/ml, or about 35 pg/ml. In other embodiments of the invention, a control level or normal levels of CCL20, IL8, or TNFα are defined as median levels of CCL20, IL8, or TNFα in a healthy control group. A healthy control group may be defined based on various criteria related to genetic background, habits, and physical attributes matched to the same set of criteria in the patient. For instance, in some embodiments, the healthy control group and the patient having IBD are matched with respect to age, gender, ethnic origin, smoking habits, dietary habits, body-mass index (BMI), recreational drug use, medical drug use, drug use related to IBD, and/or exercise habits. Other factors that can be matched between the patient and control group include, but are not limited to, clinical criteria (*e.g.,* CDAI score, Mayo score, severity of IBD-related symptoms), metabolism, IBD patient's personal disease history, genetic factors, IBD patient's family disease history, exposure to environmental factors (*e.g.,* pollutants, toxins, allergens), and life-style (*e.g.,* urban, suburban, or rural place of work and/or domicile).

In various embodiments of the invention, the initial dose of a SMAD7 antisense oligonucleotide administered to a patient having IBD may vary. For instance, in some embodiments, the initial dose of a SMAD7 antisense oligonucleotide administered to a patient having IBD is less than 500 mg/day, less than 400 mg/day, less than 300 mg/day, less than 200 mg/day, less than 100 mg/day, less than 90 mg/day, less than 80 mg/day, less than 70 mg/day, less than 60 mg/day, less than 50 mg/day, less than 40 mg/day, less than 30 mg/day, less than 20 mg/day, or less than 10 mg/day. Alternatively, in other embodiments, the initial dose is at least 1 mg/day, at least 5 mg/day, at least 10 mg/day, at least 20 mg/day, at least 30 mg/day, at least 40 mg/day, at least 50 mg/day, at least 60 mg/day, at least 70 mg/day, at least 80 mg/day, at least 90 mg/day, at least 100 mg/day, at least 200 mg/day, at least 300 mg/day, at least 400 mg/day, or at least 500 mg/day. In yet other embodiments, the initial dose is about 5 mg/day, about 10 mg/day, about 20 mg/day, about 30 mg/day, about 40 mg/day, about 50 mg/day, about 60 mg/day, about 70 mg/day, about 80 mg/day, about 90 mg/day, about 100 mg/day, about 200 mg/day, about 300 mg/day, about 400 mg/day, or about 500 mg/day. In some embodiments, the initial dose is 5 mg/day, 10 mg/day, 20 mg/day, 30 mg/day, 40 mg/day, 50 mg/day, 60 mg/day, 70 mg/day, 80 mg/day, 90 mg/day, 100 mg/day, 110 mg/day, 120 mg/day, 130 mg/day, 140 mg/day, 150 mg/day, 160 mg/day, 170 mg/day, 180 mg/day, 190 mg/day, or 200 mg/day.

In some embodiments of the invention, after analyzing the level of CCL20, IL8, or TNFα in the patient in a step (b) or (c), if the level of CCL20, IL8, and/or TNFα is above normal levels of CCL20, IL8, or TNFα, then the method may include the step of administering to the patient a subsequent dose that is greater than the initial dose. In some embodiments, after analyzing the level of CCL20, IL8, or TNFα in the patient in a step (b) or (c), if the level of CCL20, IL8, or TNFα is below normal levels of CCL20, IL8, or TNFα, then the method may include the step of administering to the patient a subsequent dose that is smaller than the initial dose.

The invention also provides a method for determining the level of a subsequent dose of SMAD7 antisense oligonucleotide with respect to an initial dose of SMAD7 antisense oligonucleotide based on levels of CCL20, IL8, or TNFα in a patient having IBD. For instance, in embodiments of the invention described herein, if CCL20, IL8, or TNFα levels in a patient having IBD are above normal levels or a control level following an initial administration step (a) or (b), the subsequent dose administered in a step (c) or (d) is at least about 5 mg/day, at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, at least about 160 mg/day, at least about 170 mg/day, at least about 180 mg/day, at least about 190 mg/day, or at least about 200 mg/day greater than the initial dose.

Alternatively, in some embodiments, if CCL20, IL8, or TNFα levels in a patient having IBD are below a control level or normal levels following an initial administration step (a) or (b), the subsequent dose administered in a step (c) or (d) is at least about 5 mg/day, at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, or at least about 100 mg/day smaller than the initial dose. Furthermore, in some embodiments, the initial dose administered in an initial administration step (a) or (b) is between about 10 mg/day and 100 mg/day, about 5 mg/day and 200 mg/day, about 10 mg/day and 50 mg/day, about 50 mg/day and 100 mg/day, and about 100 mg/day and about 200 mg/day, and the subsequent dose administered in a step (c) or (d) is between about 30 mg/day and 200 mg/day, about 5 mg/day and 30 mg/day, about 20 mg/day and 50 mg/day, about 50 mg/day and 100 mg/day, or about 100 mg/day and 200 mg/day.

The invention also provides methods for modulating treatment with a SMAD7 antisense oligonucleotide in a patient with IBD based on a comparison of relative levels of CCL20, IL8, or TNFα in a patient before and after an initial administering step. The method includes the following steps: (a) analyzing the level of CCL20, IL8, or TNFα in the patient; and (b) if the level of CCL20, IL8, or TNFα is above normal levels of CCL20, IL8, or TNFα, then administering to the patient an initial dose of a SMAD7 antisense oligonucleotide; (c) analyzing the level of CCL20, IL8, or TNFα in the patient after said administering step; and (d) if the level of CCL20, IL8, or TNFα is lower after said administration step than the level of CCL20, IL8, or TNFα before said administration step, then administering to the patient a subsequent dose that is the same as the initial dose or smaller than the initial dose. Alternatively, in step (d) if the level of CCL20, IL8, or TNFα is unchanged or increased after said administration step (*i.e.,* step (b)) compared to the level of CCL20, IL8, or TNFα before said administration step, then step (d) includes administering to the patient a subsequent dose that is greater than the initial dose or terminating the treatment. Alternatively, in step (d) if the patient is in clinical remission and the level of CCL20, IL8, or TNFα is unchanged or increased after said administration step (*i.e.,* step (b)) compared to the level of CCL20, IL8, or TNFα before said administration step, then step (d) includes terminating the treatment.

According to methods of the invention, a change in CCL20, IL8, or TNFα levels observed after an initial administration step (of SMAD7 antisense oligonucleotide) compared to CCL20, IL8, or TNFα levels prior to the administration step can be compared, for example, as a change in percent of CCL20, IL8, or TNFα levels, to determine the amount of a subsequent dose of SMAD7 antisense oligonucleotide to be administered to a patient with IBD. For example, in some embodiments, if the level of CCL20, IL8, or TNFα is at least 10%, at least 20%, at least 30%, at least 40%, at least 50%, at least 60%, at least 70%, at least 80%, at least 90%, or at least 95% decreased after said administration step (*e.g.,* an administration step (b)) compared to the level of CCL20, IL8, or TNFα before said administration step, then the method includes a step (*e.g.,* an administration step (d)) of administering to the patient a subsequent dose that is the same as the initial dose or smaller than the initial dose.

The invention also provides methods for determining the probability that a patient having IBD will experience clinical remission following treatment with a SMAD7 antisense oligonucleotide based on a comparison of CCL20, IL8, or TNFα levels, for example, based on a comparison of percent change in CCL20, IL8, or TNFα levels before and after treatment with a SMAD7 antisense oligonucleotide. For example, in some embodiments, the methods described herein further comprise the step of determining that the patient having IBD has a greater than 20%, greater than 30%, greater than 40%, greater than 50%, greater than 60%, greater than 70%, greater than 80%, greater than 90% or greater than 100% chance of experiencing clinical remission of the IBD for a time period of at least 1 week, at least 2 weeks, at least 3 weeks, at least 4 weeks, at least 5 weeks, at least 6 weeks, at least 7 weeks, or at least 8 weeks, if the level of CCL20, IL8, or TNFα after an administering step (*e.g.,* an administering step (b)) is at least 5%, at least 10%, at least 20%, at least 30%, at least 40%, at least 50%, at least 60%, at least 70%, at least 80%, at least 90%, or at least 95% decreased compared to the level of CCL20, IL8, or TNFα before the administration step.

Clinical remission, as described herein, may be determined by comparison to a reference value, for example, a Crohn's Disease Activity Index (CDAI). In some embodiments of the invention, clinical remission in a patient having IBD is indicated by a CDAI score of less than 150 (CDAI <150).

In some embodiments of the invention, clinical remission or a patient CDAI score may be observed at a given time point or within a given time frame with respect to administration of the SMAD7 antisense oligonucleotide. For example, in some embodiments, clinical remission is observed about 1 day, about 3 days, about 1 week, about 2 weeks, about 3 weeks, about 4 weeks, about 5 weeks, about 6 weeks, about 7 weeks, about 8 weeks, about 9 weeks, or about 10 weeks after an administration step (for example, an administration step (b)) and maintained for a period of at least 3 days, at least 1 week, at least 2 weeks, at least 3 weeks, at least 4 weeks, at least 5 weeks, at least 6 weeks, at least 7 weeks, at least 8 weeks, at least 9 weeks, or at least 10 weeks. Similarly, some embodiments of the invention include a method of determining that the patient having IBD has a chance of experiencing clinical remission of IBD, where the patient having IBD had a CDAI of between about 220 and about 400, about 150 and about 200, about 200 and about 250, about 250 and about 300, about 300 and about 350, about 350 and about 400, about 400 and about 450, or greater than about 450 one week prior to an administration step (for example, an administration step (b)).

In some embodiments, the invention provides a method of treating or managing IBD in a patient with above normal levels of CCL20, IL8, or TNFα, where the method includes administering to the patient a dose of SMAD7 antisense oligonucleotide. Furthermore, in some embodiments, the invention provides methods for treating or managing IBD in a patient who has above normal CCL20, IL8, or TNFα levels following administration of a dose of a SMAD7 antisense oligonucleotide, where the patient is administered a further dose of the SMAD7 antisense oligonucleotide that is greater than or equal to the prior dose. Similarly, in some embodiments, the invention provides methods for treating or managing IBD in a patient having IBD who has below normal CCL20, IL8, or TNFα levels following administration of a dose of SMAD7 antisense oligonucleotide. In the latter case, the method will include administering to the patient a further dose of the SMAD7 antisense oligonucleotide that is less than or equal to the prior dose. In some embodiments, administration of the SMAD7 antisense oligonucleotide to the patient is repeated until the levels of one or more biomarkers, *e.g.,* CCL20, IL8, C-Reactive Protein (CRP), or TNFα, reach normal levels; the patient achieves a CDAI score of less than 150; or the patient achieves clinical remission.

The invention also provides methods of treating or managing IBD in a patient having above normal levels of CCL20, IL8, or TNFα, where the amount of a SMAD7 antisense oligonucleotide administered to the patient is increased until CCL20, IL8, or TNFα levels in the patient decrease. In such embodiments, levels of SMAD7 antisense oligonucleotide administered to the patient may be increased until the level of CCL20, IL8, or TNFα in the patient decreases to about a normal level of CCL20, IL8, or TNFα or a below normal level of CCL20, IL8, or TNFα.

In some embodiments, the invention provides a method of monitoring the treatment or management of IBD in a patient with IBD, that includes analyzing CCL20, IL8, or TNFα levels in the patient following each SMAD7 antisense oligonucleotide administration. Utilizing these methods, the absence of a decrease in CCL20, IL8, or TNFα levels indicates that the treatment or management is not effective. In such embodiments, CCL20, IL8, or TNFα levels may be analyzed one time or multiple times, for instance, two times, three times, four times, about five times, about 10 times, about 15 times, about 20 times, or about 30 times, after each administration of SMAD7 antisense oligonucleotide. Furthermore, the timing of the measurement of CCL20, IL8, or TNFα levels may vary with respect to the time of SMAD7 oligonucleotide administration such that CCL20, IL8, or TNFα levels may be analyzed immediately after, about 1 hour after, about 3 hours after, about 6 hours after, about 12 hours after, about 1 day after, about 3 days after, about 1 week after, about 2 weeks after, and/or about 1 month after SMAD7 antisense oligonucleotide administration.

In order to determine levels of a biomarker or analyte, for example, CCL20, IL8, or TNFα, in a patient having IBD using the methods described herein, a sample may be obtained from the patient. Therefore, in some embodiments of the invention, the level of CCL20, IL8, or TNFα in the patient having IBD is determined in a sample obtained from the patient having IBD. Analytes other than or in addition to CCL20, IL8, or TNFα, for example, but not limited to CCL20, TNFα, IL8, and/or CRP, may also be determined in methods of the invention. Thus, in some embodiments of the invention, the method includes determining a level, or multiple levels, of one or more additional analytes in the patient having IBD. Analytes of CCL20 include RNA, DNA, and protein products of or derived from the CCL20 gene, described by NCBI Reference Sequences: AC_000134.1, NC_000002.12, and NC_018913.2. Analytes of TNFα include RNA, DNA, and protein products of or derived from the TNFα gene, described by NCBI Reference Sequence: NG_007462.1. Analytes of CRP include RNA, DNA, and protein products of or derived from the CRP gene, described by NCBI Reference Sequence: NG_013007.1. Analytes of IL8 include RNA, DNA, and protein products of or derived from the IL8 gene, described by NCBI Reference Sequence: NG_029889.1.

Samples containing analytes of interest, for example, CCL20, TNFα, CRP, and/or IL8, obtained from the patient having IBD, may include blood, serum, or plasma samples. Samples may also include tissue samples such as, but not limited to, tissue, gastrointestinal, mucosal, submucosal, intestinal, esophageal, ileal, rectal, or lymphatic samples. Levels of analytes of interest in a sample from a patient having IBD may be determined using various assays. For example, in methods of the invention, the level of CCL20, IL8, or TNFα and/or another analyte may be determined by immunochemistry, for example, by an enzyme-linked immunosorbent assay (ELISA), or by nucleotide analysis.

Methods of the invention include methods for treating and managing various forms of IBD. For example, the invention includes methods for treating and managing IBD, where the IBD is Crohn's Disease (CD) or ulcerative colitis (UC). The contemplated invention also provides methods for treating different types of patients with IBD, including, for example, but not limited to, IBD patients that are steroid-dependent patients with active CD; and steroid-resistant patients with active CD.

It will be appreciated that the SMAD7 antisense oligonucleotide administered to the patient having IBD in methods of the invention described herein, may be administered by various administration routes. In various embodiments, the SMAD7 antisense oligonucleotide may be administered by one or several routes, including orally, topically, parenterally, e.g., by subcutaneous injection, by inhalation spray, or rectally. The term parenteral as used herein includes subcutaneous injections, intrapancreatic administration, and intravenous, intramuscular, intraperitoneal, and intrasternal injection or infusion techniques. In a preferred embodiment, the SMAD7 antisense oligonucleotide may be administered orally to the patient having IBD.

The contemplated invention provides methods that include administration of a SMAD7 antisense oligonucleotide capable of targeting SMAD7 RNA for degradation, interfering with RNA splicing or preventing SMAD7 gene expression or protein translation. The contemplated SMAD7 antisense oligonucleotide of the invention may target various regions of the human SMAD7 mRNA for binding. For example, the SMAD7 antisense oligonucleotide may target nucleotides 108-128 of human SMAD7 mRNA (SEQ ID NO: 1). In some embodiments, the SMAD7 antisense oligonucleotide may target nucleotides 403, 233, 294, 295, 296, 298, 299 or 533 of the human SMAD7 sequence (SEQ ID NO: 1). The human SMAD7 mRNA sequence is the sequence of NCBI Reference Sequence: NM_005904.3 (SEQ ID NO: 1).

The sequence of the contemplated SMAD7 antisense oligonucleotide may be selected from multiple sequences capable of targeting SMAD7 RNA. For example, in some embodiments of the invention, the SMAD7 antisense oligonucleotide comprises the nucleotide sequence of SEQ ID NO: 2 (5'-GTCGCCCCTTCTCCCCGCAGC-3'). In some embodiments of the invention, the antisense oligonucleotide is an antisense oligonucleotide phosphorothioate, i.e, an oligonucleotide where at least some of the internucleotide linkages are phosphorothioate linkages, suitable for delivery to cells of a patient. Additionally, antisense oligonucleotides of the invention may include modified nucleotides, for example, nucleotides containing modified bases, for example, 5-methyl-2'-deoxycytidine. For example, in some embodiments, the antisense oligonucleotide is an antisense oligonucleotide phosphorothioate against SMAD7 comprising the following sequence:
5'-GTXGCCCCTTCTCCCXGCAG-3' (SEQ ID NO: 3) wherein X is a nucleotide comprising 5-methyl-2'-deoxycytidine and wherein the internucleotide linkages are phosphorothioate linkages.
In some embodiments, the antisense oligonucleotide is an antisense oligonucleotide phosphorothioate against SMAD7 comprising the following sequence: 5'-GTXGCCCCTTCTCCCXGCAGC-3' (SEQ ID NO: 4) wherein X is a nucleotide comprising 5-methyl-2'-deoxycytidine and wherein the internucleotide linkages are phosphorothioate linkages.
In a particular embodiment, the contemplated antisense oligonucleotide is an antisense oligonucleotide comprising SEQ ID NO: 4, wherein each of the 20 internucleotide linkages is an *O*,*O*-linked phosphorothioate linkage, referred to herein as "Mongersen."

### BRIEF DESCRIPTION OF THE DRAWINGS

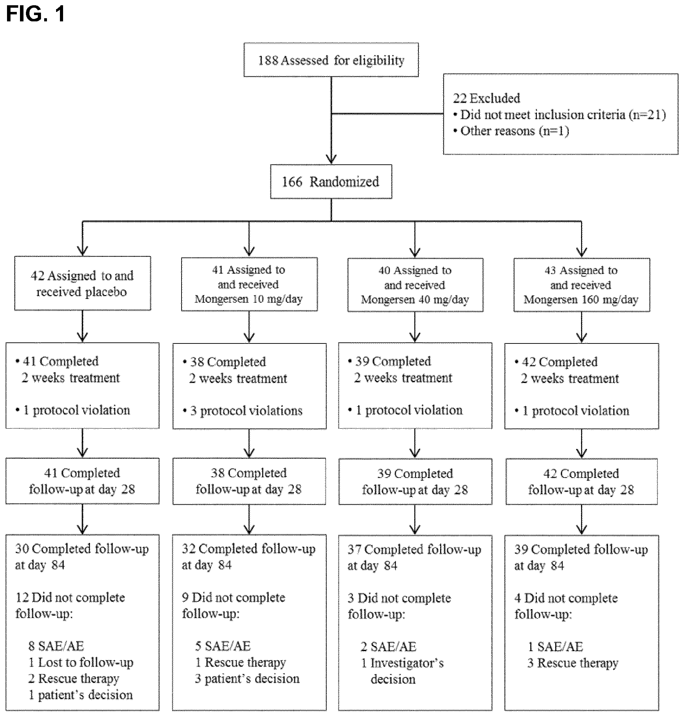

**FIGURE** 1 is a flow diagram showing the evaluation of patients assessed for eligibility in the trial and the progress of patients allowed to enter the trial (trial progression displayed from top to bottom). As indicated, prior to starting the trial 188 patients were assessed for eligibility. 22 patients failed the screening process, and 166 patients were allowed to enter the trial on day 0. As indicated 42 patients were assigned to receive placebo, 41 patients were assigned to receive Mongersen at 10 mg/day, 40 patients were assigned to receive Mongersen at 40 mg/day, and 43 patients were assigned to receive Mongersen at 160 mg/day. 41 patients completed the treatment in the placebo group, 38 patients completed the treatment in the 10 mg/day Mongersen group, 39 patients completed the treatment in the 40 mg/day Mongersen group, and 42 patients completed the treatment in the 160 mg/day Mongersen group. Of the patients who completed the assigned treatment correctly, 30 patients in the placebo group, 32 patients in the 10 mg/day Mongersen group, 37 patients in the 40 mg/day Mongersen group, and 39 patients in the 160 mg/day Mongersen group completed a follow-up procedure.

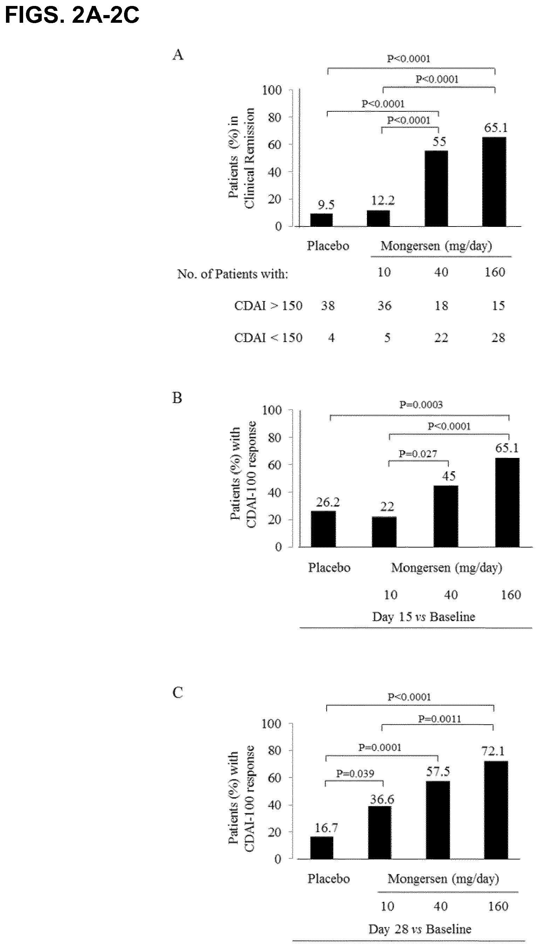

**FIGURE 2A** is a bar graph showing the percent of clinical remission in patients after the two-week clinical trial drug treatment period. As indicated, 9.5% of patients in the placebo group, 12.2% of patients in the 10 mg/day Mongersen group, 55% of patients in the 40 mg/day Mongersen group, and 65.1% of patients in the 160 mg/day Mongersen group entered remission following the end of treatment. Numbers shown below the graph show the actual number of patients in each group with a CDAI score of greater than 150 and the number of patients in each group with a CDAI score of less than 150, at the time of evaluation. Remission rates in the 40 mg/day and 160 mg/day Mongersen groups were significantly greater than the remission rates in the placebo and 10 mg/day Mongersen groups (P<0.0001 for both the 40 mg/day and 160 mg/day Mongersen groups vs. either of the 10 mg/day or placebo groups).

**FIGURE 2B** is a bar graph showing the percent of patients in each treatment group with a 100-point CDAI score decrease at day 15 of the clinical trial compared to baseline. The graph shows that 65.1% of patients in the 160 mg/day Mongersen group, 45% of patients in the 40 mg/day Mongersen group, 22% of patients in the 10 mg/day Mongersen group, and 26.2% of patients in the placebo group experienced a 100-point CDAI decrease at day 15 compared to baseline. The percentage of patients who experienced a 100-point clinical response was significantly greater in the 160 mg/day and 40 mg/day Mongersen groups compared to the 10 mg/day Mongersen group (P<0.0001 and P= 0.027 respectively). The percentage of patients who experienced a 100-point clinical response was also significantly greater in the 160 mg/day Mongersen group, compared to the placebo group (P=0.0003).

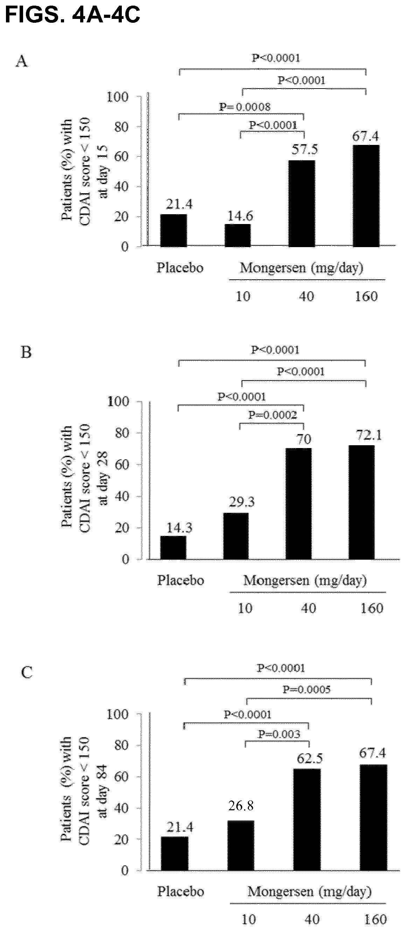

**FIGURE 2C** is a bar graph showing the percent of patients in each treatment group with a 100-point CDAI score decrease at day 28 of the clinical trial compared to baseline. The graph shows that a clinical response according to this criteria was observed in 72.1% of patients in the 160 mg/day Mongersen group, 57.5% of patients in the 40 mg/day Mongersen group, 36.6% of patients in the 10 mg/day Mongersen group, and 16.7% of patients in the placebo group. The percentage of patients who experienced a 100-point CDAI decrease on day 28 was significantly greater in the 160 mg/day, 40 mg/day, and 10 mg/day Mongersen groups compared to the placebo group (FIG. 4B; P<0.0001 for the 160 mg/day group, P=0.0001 for the 40 mg/day group, and P=0.039 for the 10 mg/day group, compared to placebo for each group, respectively).

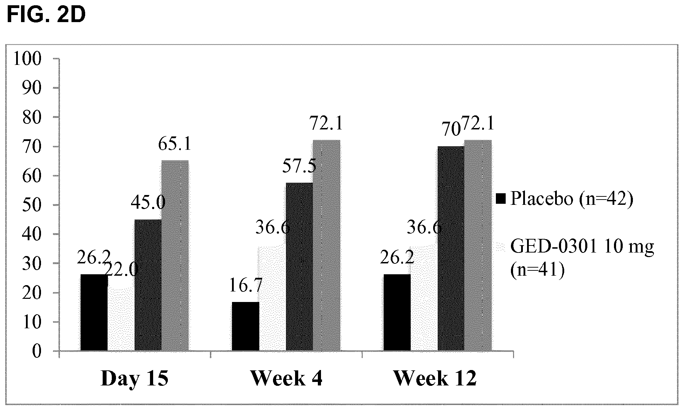

**FIGURE 2D** is a bar graph showing the data from FIGS. 2B and 2C along with 12 week (day 84) data for a 100-point CDAI score decrease.

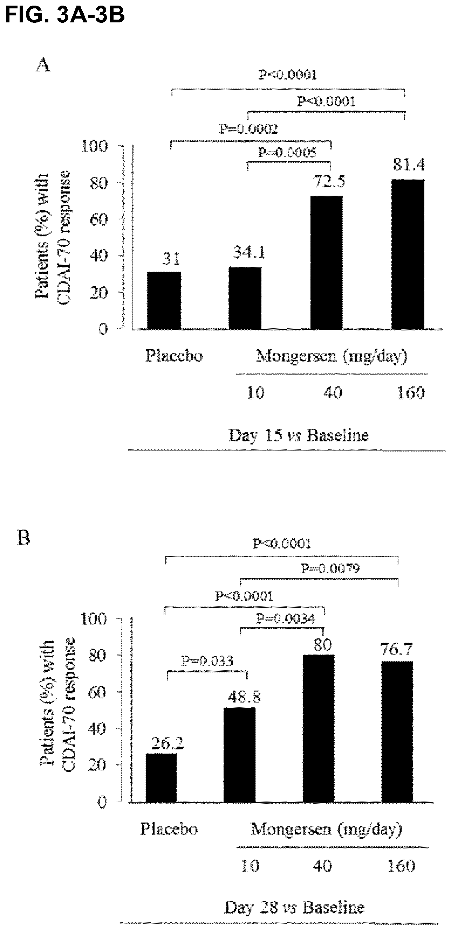

**FIGURE 3A** is a bar graph showing the percent of patients in each treatment group with a 70-point CDAI score decrease at day 15 of the clinical trial compared to baseline. The graph shows that a 70-point CDAI decrease was documented in 81.4% of patients in the 160 mg/day Mongersen group, 72.5% of patients in the 40 mg/day Mongersen group, 34.1% of patients in the 10 mg/day Mongersen group, and 31% of patients in the placebo group. The percentage of patients who experienced a 70-point CDAI score decrease on day 15 was significantly greater in the 160 mg/day and 40 mg/day Mongersen groups compared to the 10 mg/day Mongersen (P<0.0001 and P= 0.0005, respectively) and placebo groups (P<0.0001 and P= 0.0002, respectively).

**FIGURE 3B** is a bar graph showing the percent of patients in each treatment group with a 70-point CDAI score decrease at day 28 of the clinical trial compared to baseline. The graph shows that a 70-point CDAI decrease was documented in 76.7% of patients in the 160 mg/day Mongersen group, 80% of patients in the 40 mg/day Mongersen group, 48.8% of patients in the 10 mg/day Mongersen group, and 26.2% of patients in the placebo group. The percentage of patients who experienced a 70-point CDAI score decrease on day 28 was significantly greater in the 160 mg/day, 40 mg/day, and 10 mg/day Mongersen groups compared to placebo groups (P<0.0001, P<0.0001, and P= 0.033, respectively).

**FIGURE 4A** is a bar graph showing the percentage of patients in each group with CDAI scores of less than 150 on day 15 of the trial. The percentage of patients with CDAI scores of less than 150 in the 160 mg/day, 40 mg/day, and 10 mg/day Mongersen groups and placebo groups were 67.4%, 57.5%, 14.6%, and 21.4%, respectively. On day 15, the percent of patients with CDAI scores lower than 150 was significantly higher in the 160 mg/day and 40 mg/day Mongersen groups compared to the 10 mg/day Mongersen group (P<0.0001 for both groups) and the placebo group (P<0.0001 and P=0.0008, respectively).

**FIGURE 4B** is a bar graph showing the percentage of patients in each group with CDAI scores of less than 150 on day 28 of the trial. On day 28, the percentage of patients with CDAI scores of less than 150 in the 160 mg/day, 40 mg/day, and 10 mg/day Mongersen groups and placebo groups were 72.1%, 70%, 29.3%, and 14.3%, respectively. On day 28, the percent of patients with CDAI scores lower than 150 was significantly higher in the 160 mg/day and 40 mg/day Mongersen groups compared to the 10 mg/day Mongersen group (P<0.0001 and P=0.0002, respectively) and the placebo group (P<0.0001 for both groups).

**FIGURE 4C** is a bar graph showing the percentage of patients in each group with CDAI scores of less than 150 on day 84 of the trial. On day 84, the percentage of patients with CDAI scores of less than 150 in the 160 mg/day, 40 mg/day, and 10 mg/day Mongersen groups and placebo groups were 67.4%, 62.5%, 29.3%, and 21.4%, respectively. On day 84, the percent of patients with CDAI scores lower than 150 was significantly higher in the 160 mg/day and 40 mg/day Mongersen groups compared to the 10 mg/day Mongersen group (P=0.0005 and P=0.003, respectively) and the placebo group (P<0.0001 for both groups).

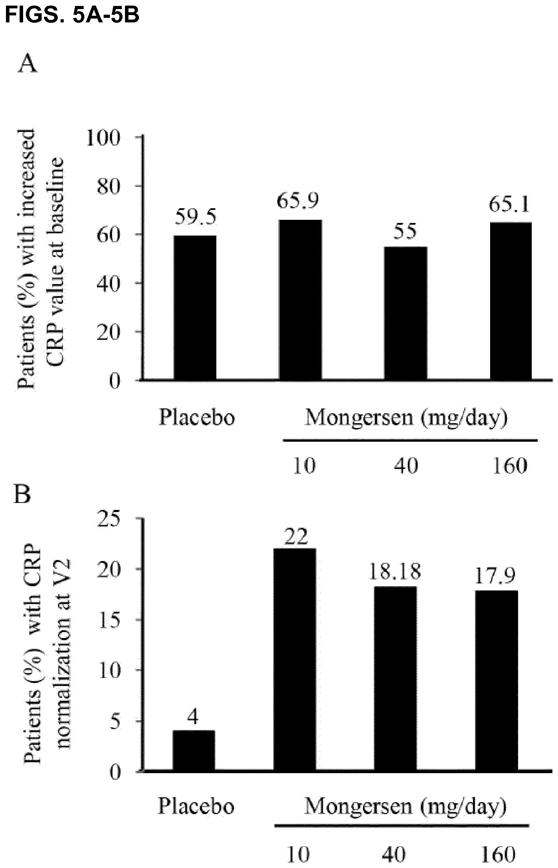

**FIGURE 5A** is a bar graph that shows the percent of patients in each group with elevated CRP levels at baseline. Elevated CRP levels were observed in 59.5% of patients in the placebo group, 65.9% of patients in the 10 mg/day Mongersen group, 55% of patients in the 40 mg/day Mongersen group, and 65.1% of patients in the 160 mg/day Mongersen group.

**FIGURE 5B** is a bar graph that shows the percent of patients in each group with normalized CRP levels on day 15 of the clinical trial. Normalized CRP levels were observed in 4% of patients in the placebo group, and 22%, 18.18%, and 17.9% of patients, respectively, in the 10 mg/day, 40 mg/day and 160 mg/day Mongersen groups.

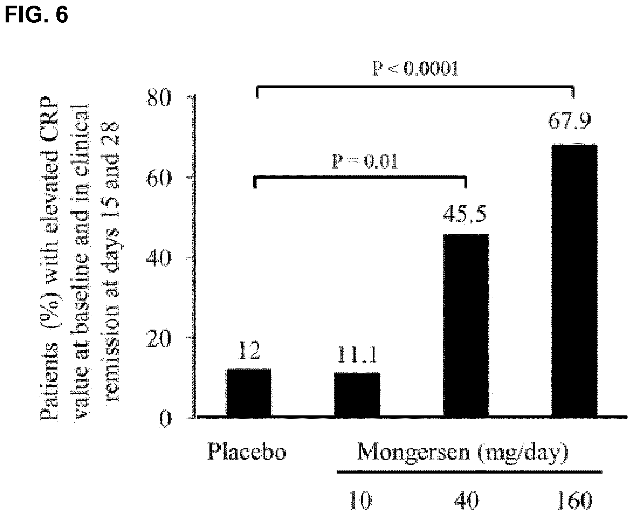

**FIGURE 6** is a bar graph showing the percent of patients in each treatment group with elevated baseline CRP levels at baseline that entered remission. In patients with elevated CRP levels at baseline, the percentage of patients that entered remission were 67.9%, 45.5%, 11.1%, and 12%, respectively, in the 160 mg/day, 40 mg/day, and 10 mg/day Mongersen groups and the placebo group. The percentage of patients with elevated CRP levels at baseline that entered remission was significantly higher in the 160 mg/day and 40 mg/day Mongersen groups compared to the placebo group (P<0.0001 and P=0.01, respectively). No significant difference was observed between the 160 mg/day and 40 mg/day Mongersen groups or between the 10 mg/day Mongersen and placebo groups.

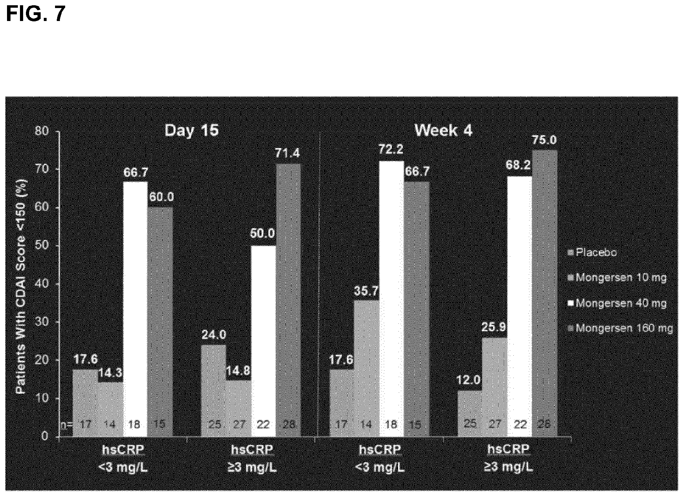

**FIGURE 7** is a bar graph showing percentage of patients with clinical remission for patients having CRP levels of less than 3 mg/l or having CRP levels of 3 mg/l or more at day 15 and week 4 of the trial.

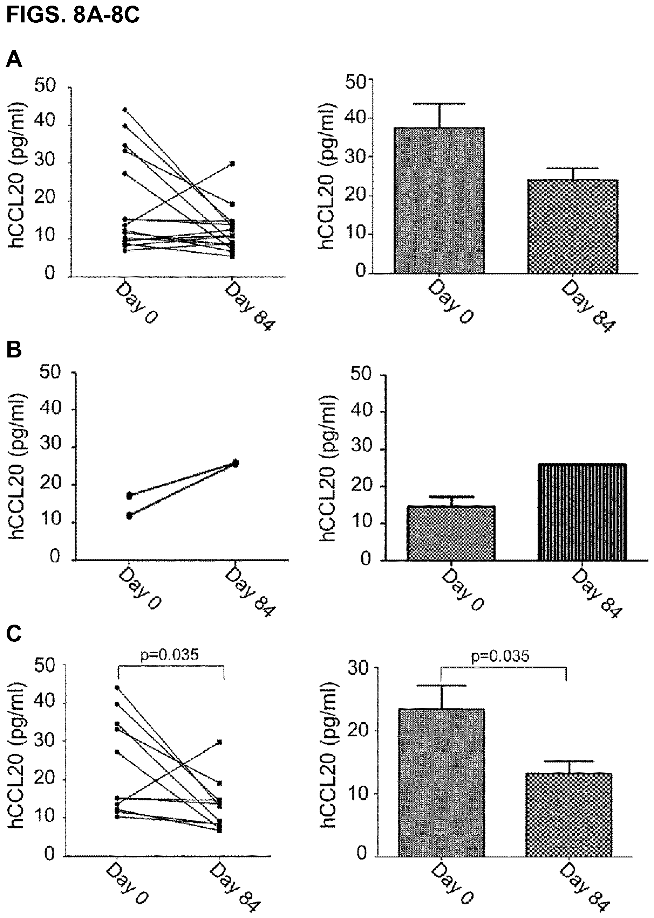

**FIGURE 8A** is a set of two graphs showing levels of serum CCL20 (hCCL20) in individuals who underwent clinical remission/response following 40 mg/day or 160 mg/day Mongersen treatment. The left panel shows individual measurements taken at day 0 and day 84, with lines connecting the data taken from individual patients at each time point. The right panel is a bar graph showing mean value ± sem for CCL20 levels at day 0 and day 84.

**FIGURE 8B** is a set of graphs showing levels of serum CCL20 (hCCL20) in individuals who did not experience clinical remission/response following 40 mg/day or 160 mg/day Mongersen treatment. The left panel shows individual measurements taken at day 0 and day 84, with lines connecting the data taken from individual patients at each time point. The right panel is a bar graph showing the mean value ± sem for CCL20 levels at day 0 and day 84.

**FIGURE 8C** is a set of two graphs showing levels of serum CCL20 (hCCL20) measured at day 0 and day 84 in individuals who underwent clinical remission/response following 40 mg/day or 160 mg/day Mongersen treatment, with baseline serum CCL20 levels greater than 10 pg/ml. The left panel shows individual measurements taken at each time point, with lines connecting the data points at day 0 and day 84 taken from individual patients. The right panel is a bar graph showing the mean value ± sem for CCL20 levels at day 0 and day 84.

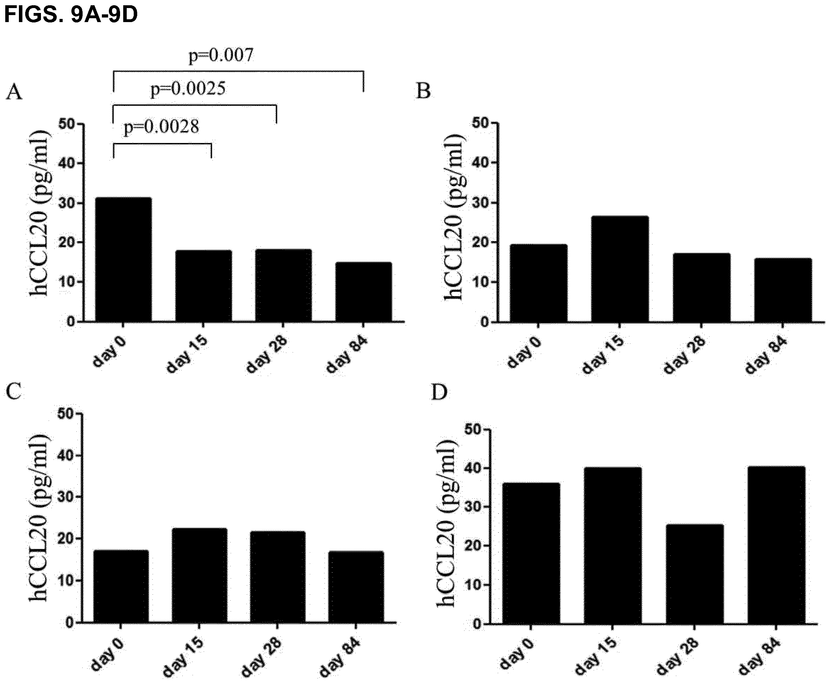

**FIGURES 9A-9D** are bar graphs showing CCL20 serum levels (hCCL20) at days 0, 15, 28, and 84 of Mongersen or placebo treatment among clinically responsive Mongersen-treated patients (**FIG. 9A**), clinically non-responsive Mongersen-treated patients **(****FIG. 9B****),** clinically responsive placebo-treated patients (**FIG. 9C****)**, and clinically non-responsive placebo-treated patients **(****FIG. 9D****).**

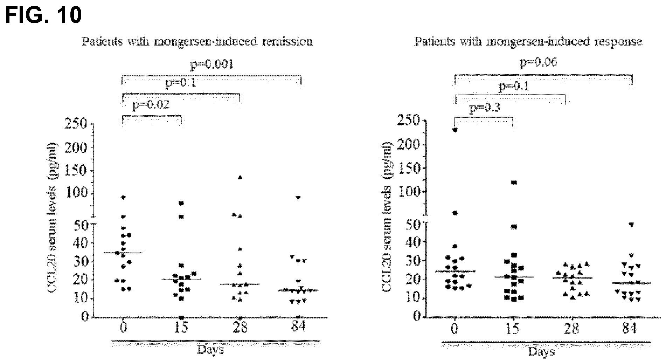

**FIGURE 10** is a pair of graphs showing CCL20 serum levels at days 0, 15, 28, and 84 of Mongersen treatment among individuals who experienced clinical remission (left) or clinical response (right). Horizontal bars represent median values for each time point, and each point represents CCL20 serum values in a single patient.

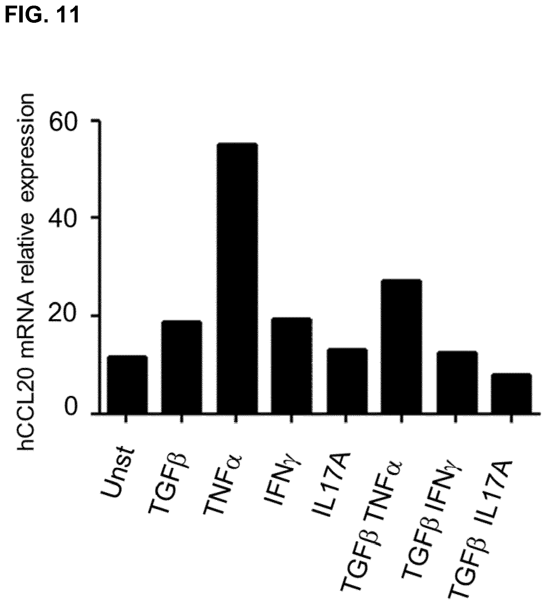

**FIGURE 11** is a bar graph showing CCL20 (hCCL20) mRNA expression in NCM460 cells following stimulation with various factors relative to unstimulated (Unst) controls.

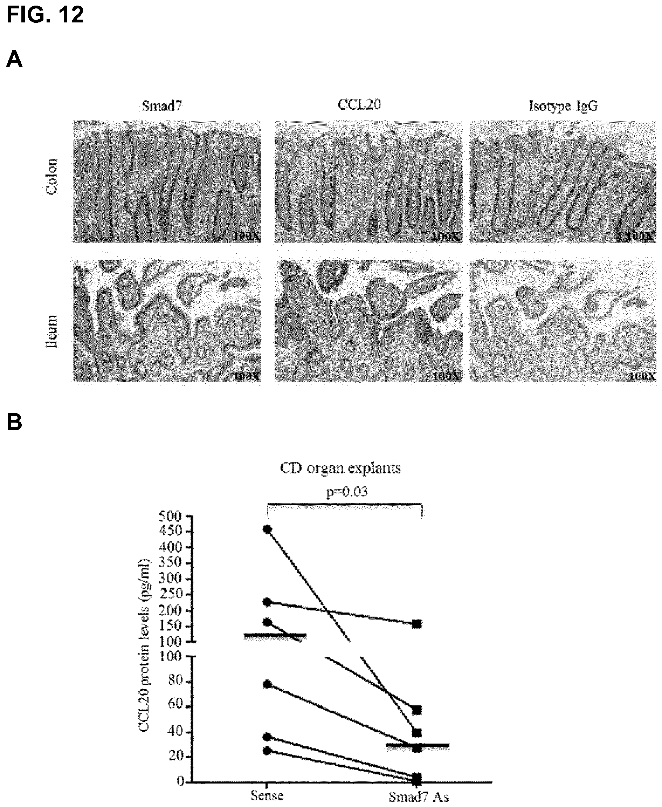

**FIGURE 12A** is a series of images showing immunohistochemical staining of SMAD7 (left panels), CCL20 (middle panels), or IgG (Isotype IgG, right panels)(brown) and haematoxylin (purple) in colon or ileum mucosal tissue sections from Crohn's disease patients. Images are representative of 4 experiments.

**FIGURE 12B** is a graph showing median and individual CCL20 protein levels in colonic tissue explants treated with either SMAD7 sense (Sense) or antisense (Smad7 As) oligonucleotides. Horizonal bars represent median CCL20 levels in each group, and each data point represents CCL20 levels in an individual explant. Each bar connecting individual data points in the Sense and Smad7 As columns connects data points for explants obtained from the same patient.

### DETAILED DESCRIPTION

The invention provides methods that are generally useful for treating and managing IBD in a patient having IBD. Patients having IBD include, but are not limited to, patients having UC and CD, including steroid-dependent and steroid-resistant forms of the latter. The method is particularly useful in terms of managing treatment in a patient being treated with an anti-SMAD7 therapy, such as a SMAD7 antisense oligonucleotide therapy. A SMAD7 antisense oligonucleotide therapy may be any therapy that includes an oligonucleotide that is capable of binding to a SMAD7 mRNA transcript and inducing degradation of the SMAD7 mRNA transcript, preventing splicing of the SMAD7 mRNA transcript, or preventing protein translation of the SMAD7 mRNA transcript.

Methods of the invention are useful for predicting and determining responsiveness of patients having IBD to treatment with SMAD7 antisense oligonucleotide. Thus, methods of the invention can be used to identify patients that are likely to respond to SMAD7 antisense oligonucleotide treatment as well as patients that are unlikely to respond to SMAD7 antisense oligonucleotide treatment. The methods described herein are also useful for determining whether a patient is or is not responsive to IBD treatment. Generally, methods of the invention can also be used to determine the level or likely level of responsiveness in a patient having IBD being treated with a SMAD7 antisense oligonucleotide. Based upon a determination of a level of responsiveness or a likely level of responsiveness, administration of the SMAD7 antisense oligonucleotide may be initiated, repeated, maintained, increased, decreased, or terminated. Responsiveness may be determined using a number of factors including, but not limited to: analysis of levels or changes in levels of biomarkers and/or other analytes (e.g., CCL20, IL8, CRP, TNFα), CDAI score or changes in CDAI score, or assessment of symptoms of IBD *(e.g.,* weight loss, tissue inflammation, bloody stool).

Similarly, the methods are useful for evaluating efficacy and safety of treatment with a SMAD7 antisense oligonucleotide in a patient having IBD. For example, methods of the invention may include determining changes in levels of biomarker expression or other indicators or manifestations of disease state that can indicate that treatment with the SMAD7 antisense oligonucleotide is effective or not effective to cause partial or complete remission or amelioration of IBD. Determining levels or changes in levels of biomarker expression, disease symptoms, tissue, blood, or systemic levels of the SMAD7 antisense oligonucleotide, or indicators of general health may also indicate a worsening of disease state or unsafe drug levels. Assessment of multiple indicators before, during, between, and/or after treatment(s) may be used to monitor disease stage, progression, and severity.

The invention is based in part on the discovery of a relationship between IBD disease state and CCL20, IL8, and TNFα levels. Specifically, the inventors have discovered that each of CCL20, IL8, and TNFα levels is a useful biomarker for determining whether a patient is responsive to, likely to be responsive to, not responsive to, or likely not responsive to treatment of IBD using a SMAD7 antisense oligonucleotide.

Furthermore, CCL20, IL8, or TNFα levels can be used to manage disease treatment using a SMAD7 antisense oligonucleotide, specifically with respect to dose amount of the SMAD7 antisense oligonucleotide. For example, levels of CCL20, IL8, or TNFα may be used to determine whether a patient having IBD should be given a specific dose amount, for example, a higher dose or a lower dose, of SMAD7 antisense oligonucleotide, for example in a subsequent dose, with respect to, for example, a previously administered dose, for example, an initial dose, of SMAD7 antisense oligonucleotide. Thus, administration of a SMAD7 antisense oligonucleotide may be adjusted in terms of, for example, dose amount or frequency, with respect to absolute levels of CCL20, IL8, or TNFα or relative levels of CCL20, IL8, or TNFα in a patient having IBD. For instance, administration of a SMAD7 antisense oligonucleotide may be adjusted based on absolute levels of CCL20, IL8, or TNFα by comparing absolute levels of CCL20, IL8, or TNFα measured in a sample from a patient having IBD with a normal level of CCL20, IL8, or TNFα, where the normal level of CCL20, IL8, or TNFα is, for instance, either a benchmark value or a median level of CCL20, IL8, or TNFα in a healthy control group matched to the patient having IBD. In some embodiments of the invention, administration of a SMAD7 antisense oligonucleotide may be adjusted based on relative levels of CCL20, IL8, or TNFα, for instance, based on a comparison of CCL20, IL8, or TNFα levels before and after SMAD7 antisense oligonucleotide administration, immediately after and later after SMAD7 antisense oligonucleotide administration, or during and after SMAD7 antisense oligonucleotide administration. In some embodiments, the SMAD7 antisense oligonucleotide may be administered multiple times between an initial detection of CCL20, IL8, or TNFα levels and a later detection of CCL20, IL8, or TNFα levels used to generate the comparison of CCL20, IL8, or TNFα levels in the patient sample.

In some embodiments of the invention the IBD patient being treated is a patient with above-normal CCL20, IL8, and/or TNFα levels. In some embodiments, a patient is known to have high CCL20, IL8, and/or TNFα levels before treatment. In some embodiments, CCL20, IL8, and/or TNFα levels in the IBD patient are determined before treatment, after treatment, before administration of an initial dose of a SMAD7 antisense oligonucleotide, after administration of an initial dose of a SMAD7 antisense oligonucleotide, before administration of a subsequent dose of a SMAD7 antisense oligonucleotide, and/or after administration of a subsequent dose of a SMAD7 antisense oligonucleotide.

### Control Levels and Control Samples

A control level of CCL20, IL8, or TNFα may be determined by determining the level of CCL20, IL8, or TNFα protein or mRNA transcript in a sample (*e.g.,* a blood sample) obtained from the subject prior to treatment with an anti-SMAD7 therapy. The control level of CCL20, IL8, or TNFα may provide a baseline for monitoring a subject's response to treatment. A control sample may be obtained from the subject on the day the anti-SMAD7 therapy is first administered (*e.g.,* Day 1 of a treatment regimen), for example, immediately after administration of at least one anti-SMAD7 therapy. In other embodiments, a control sample may be obtained from a subject one day prior to the start of an anti-SMAD7 therapy (*e.g.,* Day 0 of a treatment regimen). Alternatively, a control sample may be obtained from a subject 2, 3, 4, 5, 6, 7 or more days prior to the start of an anti-SMAD7 therapy. For example, the increase or decrease in CCL20, IL8, or TNFα concentration may be measured prior to treatment (*e.g.,* in a control sample), during treatment, and/or after treatment to monitor a subject's response to therapy, *e.g.,* an anti-SMAD7 therapy.

In some embodiments, a control level may be established for a subject based on long-term monitoring of circulating CCL20, IL8, or TNFα concentration in the subject. In such instances, it is contemplated that a subject may undergo multiple rounds of treatment with an anti-SMAD7 therapy. The circulating CCL20, IL8, or TNFα concentration detected following multiple rounds of treatment may be compared to a prior control level of CCL20, IL8, or TNFα for the subject to determine whether the subject has responded to therapy and/or is likely to respond to further treatment with an anti-SMAD7 therapy. In other embodiments, a control or baseline level for a subject may be established based on an average measurement of a circulating CCL20, IL8, or TNFα concentration determined from multiple baseline samples obtained over time (*e.g.,* obtained over the course of days, weeks, months, or years). Accordingly, any test or assay conducted as disclosed herein may be compared with a previous or established control level and it may not be necessary to obtain a new control sample from the subject for comparison, *e.g.,* if the subject is receiving more than one round of treatment with an anti-SMAD7 therapy.

Normal levels of CCL20, IL8, or TNFα may be determined based on numerical reference values or with respect to levels of CCL20, IL8, or TNFα in a healthy control group.

In other embodiments of the invention, normal levels of CCL20, IL8, or TNFα are defined as median levels of CCL20, IL8, or TNFα in a healthy control group.

A healthy control group may be defined based on various criteria related to genetic background, habits, and physical attributes matched to the same set of criteria in the patient. For instance, in some embodiments, the healthy control group and the patient having IBD are matched with respect to age, gender, ethnic origin, smoking habits, dietary habits, body-mass index (BMI), recreational drug use, medical drug use, drug use related to IBD, and/or exercise habits. Other factors that can be matched between the patient and control group include, but are not limited to, clinical criteria (*e.g.,* CDAI score, Mayo score, severity of IBD-related symptoms), metabolism, IBD patient's personal disease history, genetic factors, IBD patient's family disease history, exposure to environmental factors (*e.g.,* pollutants, toxins, allergens), and life-style (*e.g.,* urban, suburban, or rural place of work and/or domicile).

In some embodiments, the control group is the patient receiving a treatment with an SMAD7 antisense oligonucleotide prior to receiving an initial dose of the SMAD7 antisense oligonucleotide. In some embodiments, the patient is a treatment naive patient.

### Data Interpretation

In some embodiments, prior to initial administration of an anti-SMAD7 therapy, the level of CCL20, IL8, or TNFα in a patient having IBD is analyzed and compared to a threshold level. As described herein, a threshold level may be established based on CCL20, IL8, or TNFα levels in a healthy control group or a group of IBD patients. In general, a threshold level will be elevated with respect to normal CCL20, IL8, or TNFα levels, for example median CCL20, IL8, or TNFα levels in a healthy control group, or it may fall within the spectrum of CCL20, IL8, or TNFα levels in a control group, for example a control group comprised of IBD patients.

A subject's responsiveness to treatment with an anti-SMAD7 therapy can be interpreted with respect to the control level of CCL20, IL8, or TNFα in a sample obtained from the subject prior to treatment. A subject may be identified as sensitive to treatment (e.g., responsive or likely to respond to treatment) with an anti-SMAD7 therapy if there is a decrease in the concentration of CCL20, IL8, or TNFα in the sample obtained from the subject compared to the control sample. In some embodiments the sample may be obtained while the subject is receiving an anti-SMAD7 therapy treatment. In other embodiments, the sample may be obtained after the subject has stopped receiving treatment, for example, about 1 day, about 7 days (*i.e.,* about 1 week), about 14 days (*i.e.,* about 2 weeks), about 28 days, about 56 days, about 70 days and/or longer, after stopping treatment. In a preferred embodiment, the sample may be obtained about one day after stopping anti-SMAD7 therapy treatment.

In a contemplated embodiment of the invention, a decrease in the amount of CCL20, IL8, or TNFα in the sample coincides with a CDAI score indicating that the subject is responsive to therapy and/or has entered remission or is likely to enter remission. For example, in some embodiments, a decrease in the amount of CCL20, IL8, or TNFα in the sample compared to the control level coincides with a CDAI score of less than about 200, less than about 190, less than about 180, less than about 170, less than about 160, or less than about 150 in the subject. In a particular embodiment, a decrease in the amount of CCL20, IL8, or TNFα in the sample compared to the control level coincides with a CDAI score of less than about 150 in the subject. In some embodiments, the CDAI score that coincides with the decrease in CCL20, IL8, or TNFα concentration is maintained for at least one day, at least one week, at least two weeks, or at least 10 weeks in the subject.

In some embodiments, the CDAI score that coincides with the decrease in CCL20, IL8, or TNFα concentration is observable after stopping treatment with the anti-SMAD7 therapy. For example, the CDAI score that coincides with the decrease in CCL20, IL8, or TNFα concentration may be observable about 1 day, about 1 week, about 2 weeks, about 10 weeks, about 1 day and about 2 weeks, or longer after stopping treatment with an anti-SMAD7 therapy. In some embodiments, a decrease in the amount of CCL20, IL8, or TNFα in the sample coincides with a decrease in CDAI score indicating that the subject is responsive to therapy and/or has entered remission or is likely to enter remission. For example, in some embodiments of the invention, a decrease in the amount of CCL20, IL8, or TNFα in the sample compared to the control level coincides with a decrease in CDAI score of about 50, about 60, about 70, about 80, about 90, about 100, about 110, about 120, about 130, about 140, or about 150 in the subject. In particular embodiments, a decrease in the amount of CCL20, IL8, or TNFα in the sample compared to the control level coincides with a decrease in CDAI score of about 70 to about 100 in the subject. In some embodiments, the decrease in CDAI score that coincides with the decrease in the amount of CCL20, IL8, or TNFα is observable after stopping treatment with the anti-SMAD7 therapy. For example, the decrease in CDAI score that coincides with the decrease in the amount of CCL20, IL8, or TNFα may be observable about 1 day, about 1 week, about 2 weeks, about 3 weeks, about 4 weeks, about 10 weeks, or longer after stopping treatment with an anti-SMAD7 therapy. In some embodiments, the decrease in CDAI score that coincides with the decrease in the amount of CCL20, IL8, or TNFα is observable about 1 day or about 2 weeks after stopping treatment with an anti-SMAD7 therapy.

In some embodiments, patients receiving an anti-SMAD7 therapy, such as a SMAD7 antisense oligonucleotide, also receive one or more additional IBD therapies, *e.g.,* steroids. In some embodiments, patients receiving the anti-SMAD7 therapy and the one or more additional IBD therapies can taper the one or more additional IBD therapies if they respond to the anti-SMAD7 therapy and/or experience clinical remission, *e.g.,* as indicated by decreasing CDAI scores and/or decreasing CCL20, IL8, or TNFα levels. In some embodiments, patients experiencing clinical remission following the administration of an anti-SMAD7 therapy *(e.g.,* CDAI<150 at both day 15 and day 28 following completion of a 2-week treatment regimen with a SMAD7 antisense oligonucleotide) can taper steroids.

Alternatively, a subject may be identified as resistant to treatment (*e.g.,* non-responsive or unlikely to respond) with an anti-SMAD7 therapy if there is no change or an increase in circulating CCL20, IL8, or TNFα concentration in the sample obtained from the subject, compared to the control level. In one embodiment, the sample may be obtained while the subject is receiving an anti-SMAD7 therapy treatment. In other embodiments, the sample may be obtained after the subject has stopped receiving treatment, for example, about 1 day, about 7 days (*i.e.,* about 1 week), about 14 days (*i.e.,* about 2 weeks), about 28 days, about 56 days, about 70 days, and/or longer after stopping treatment. In a preferred embodiment, the sample may be obtained about one day after stopping anti-SMAD7 therapy treatment.

In some embodiments, one or more rescue therapies (*e.g.,* a biologic such as an IL8, TNFα, or CCL20 inhibitor and/or an immunosuppressive drug) is administered to patients experiencing a worsening of disease during a course of treatment with an anti-SMAD7 therapy, *e.g.,* as indicated by increasing CDAI scores (*e.g.,* >70 CDAI score increase) and/or increasing CCL20, IL8, or TNFα levels (*e.g.,* >50% increase in CCL20, IL8, or TNFα levels).

Differences in patient CCL20, IL8, or TNFα levels and threshold CCL20, IL8, or TNFα levels are indicative of a patient's potential responsiveness to anti-SMAD7 therapy. For example, patient CCL20, IL8, or TNFα levels that are elevated relative to a threshold CCL20, IL8, or TNFα level indicate that a patient may be responsive to anti-SMAD7 therapy. Threshold levels of CCL20, IL8, or TNFα can be established using different criteria. In some embodiments, the threshold level of CCL20, IL8, or TNFα is determined with respect to normal CCL20, IL8, or TNFα levels, for example median CCL20, IL8, or TNFα levels, in a control group. Control groups may be comprised of healthy/normal subjects (*e.g.,* a healthy control group) or groups of IBD patients.

For instance, in some embodiments, a CCL20, IL8, or TNFα threshold level is at least 2-fold, at least 3-fold, at least 5-fold, at least 8-fold, at least 10-fold, at least 20-fold, at least 30-fold, at least 50-fold, at least 80-fold, or at least 100-fold above normal levels. In other embodiments, the CCL20, IL8, or TNFα threshold level is in the 50^{th} percentile, 60^{th} percentile, 70^{th} percentile, 80^{th} percentile or 90^{th} percentile of CCL20, IL8, or TNFα levels with respect to CCL20, IL8, or TNFα levels, for example median CCL20, IL8, or TNFα levels, in a group of IBD patients. Additionally, in some embodiments, the threshold level of CCL20, IL8, or TNFα is at least or about 1 pg/ml, at least or about 2.5 pg/ml, at least or about 5 pg/ml, at least or about 7.5 pg/ml, at least or about 10 pg/ml, at least or about 12.5 pg/ml, at least or about 15 pg/ml, at least or about 17.5 pg/ml, at least or about 20 pg/ml, at least or about 25 pg/ml, at least or about 30 pg/ml, at least or about 35 pg/ml, at least or about 40.0 mg/L, or at least or about 45.0 mg/L (*e.g.,* as measured in blood serum).

In some embodiments, the control group may consist of the patient receiving an initial dose of a SMAD7 antisense oligonucleotide. In some embodiments, normal CCL20, IL8, or TNFα levels, or CCL20, IL8, or TNFα threshold levels, may be the CCL20, IL8, or TNFα baseline levels that are observed in a patient prior to administration of an initial dose of SMAD7 antisense oligonucleotide. CCL20, IL8, or TNFα levels can subsequently be monitored in a patient over time, following the administration of the initial dose or of subsequent doses of SMAD7 antisense oligonucleotide to the patient. CCL20, IL8, or TNFα levels in the patient following one or more administrations of a SMAD7 antisense oligonucleotide can be compared to the CCL20, IL8, or TNFα baseline level in the patient. Dosing regimens for the SMAD7 antisense oligonucleotide can be adjusted, depending on whether CCL20, IL8, or TNFα levels in the patient increase, decrease or remain constant relative to the patient's CCL20, IL8, or TNFα baseline level.

### Anti-SMAD7 Therapies

The present disclosure is directed in part to methods of treating IBD in a patient with an anti-SMAD7 therapy comprising a SMAD7 inhibitor. SMAD7 inhibitors may include, for example, small binding molecules, e.g., natural and synthetic compounds, antibodies, aptamers, intramers, RNAi (double stranded RNA, siRNA) and SMAD7 antisense oligonucleotides that bind, degrade, or otherwise interfere with SMAD7 stability, production, or function. SMAD7 inhibitors may also comprise truncated and/or mutated SMAD7 molecules which interfere with SMAD7 activity, binding partners, or substrates and which, thereby, inhibit SMAD7 function.

The present disclosure is also directed in part to methods of treating IBD in a patient with a SMAD7 antisense oligonucleotide. Antisense oligonucleotides are short synthetic oligonucleotide sequences complementary to the messenger RNA (mRNA), which encodes for the target protein (*e.g.,* SMAD7). Antisense oligonucleotide sequences hybridize to the mRNA producing a double-strand hybrid that can lead to the activation of ubiquitary catalytic enzymes, such as RNase H, which degrades DNA/RNA hybrid strands thus preventing protein translation.

The contemplated SMAD7 antisense oligonucleotide may target any region of the SMAD7 mRNA. In certain embodiments, an anti-SMAD7 antisense oligonucleotide may target site 403, 233, 294, 295, 296, 298, 299, and/or 533 (*i.e.,* nucleotides 403, 233, 294, 295, 296, 298, 299, and 533, respectively) of the human SMAD7 mRNA (*e.g.,* of SEQ ID NO: 1; NCBI Reference Sequence NM_005904.3).

In certain embodiments, an antisense oligonucleotide may be derived from the following anti-SMAD7 antisense oligonucleotide 5'-GTCGCCCCTTCTCCCCGCAGC-3' (SEQ ID NO: 2).

It is contemplated herein that an antisense oligonucleotide targeting SMAD7 may comprise a mixed-backbone wherein the cytosine residues in a CpG pair are replaced by 5'-methylcytosine (abbreviated as Me-dC). Methylphosphonate linkages may also be placed at the 5' and/or 3' ends of an antisense oligonucleotide (abbreviated as MeP).

Exemplary antisense oligonucleotide therapies that target SMAD7 include, but are not limited to, 5'-GTXYCCCCTTCTCCCXYCAG-3' (SEQ ID NO: 5), wherein X is a nucleotide comprising a nitrogenous base selected from the group consisting of cytosine and 5-methylcytosine or a 2'-O-methylcytosine nucleoside, and wherein Y is a nucleotide comprising a nitrogenous base selected from the group consisting of guanine and 5-methylguanine or a 2'-O-methylguanine nucleoside, provided that at least one of the nucleotides X or Y comprises a methylated nitrogenous base;

5'-GTXGCCCCTTCTCCCXGCAG-3' (SEQ ID NO: 3), wherein X is 5-methyl 2'-deoxycytidine (See, *e.g.,* U.S. Patent Nos. 7,807,818 and 6,159,697, which are each incorporated herein by reference.);

5'-GTXGCCCCTTCTCCCXGCAGC-3' (SEQ ID NO: 4), wherein X is 5-methyl 2'-deoxycytidine; and

Antisense oligonucleotides described in U.S. Patent No. 8,648,186 and International Patent Application Publication WO 2010/054826, each of which is incorporated herein by reference.

Contemplated antisense oligonucleotides include those comprising SEQ ID NO: 4: 5'-GTC* GCC CCT TCT CCC C*GC AGC-3', where C* represents 5-methyl-2'-deoxycytidine. In some embodiments, at least one of the internucleotide linkages of a contemplated antisense oligonucleotide is an *O*,*O*-linked phosphorothioate, for example, each of the 20 internucleotide linkages of SEQ ID NO: 4 may be an *O*,*O*-linked phosphorothioate. In a particular embodiment, the contemplated antisense oligonucleotide is an antisense oligonucleotide comprising SEQ ID NO: 4, wherein each of the 20 internucleotide linkages is an *O*,*O*-linked phosphorothioate linkage, referred to herein as "Mongersen." In some embodiments, contemplated compositions disclosed herein may include a pharmaceutically acceptable salt, *e.g.,* a sodium salt of the antisense oligonucleotide of SEQ ID NO: 4, that optionally may include 1 to 20 *O*,*O*-linked phosphorothioate internucleotide linkages. Contemplated salts of oligonucleotides include those that are fully neutralized, *e.g.,* each phosphorothioate linkage is associated with an ion such as Na⁺. Oligonucleotides may include naturally occurring nucleobases, sugars, and covalent internucleotide (backbone) linkages as well as non-naturally occurring portions. In varying embodiments, the antisense oligonucleotides described herein, for example, the antisense oligonucleotides of SEQ ID NO: 2, SEQ ID NO: 3, and SEQ ID NO: 4, may include nucleotides comprising deoxycytidine and/or 5-methyl 2'-deoxycytidine, including, but not limited to, 5-methyl-2'-deoxycytidine 5'-monophosphate and 5-methyl-2'-deoxycytidine 5' monophosphorothioate.

Contemplated SMAD7 antisense oligonucleotides include oligonucleotides that act against SMAD7 and may be administered orally. Disclosed therapies may, when administered orally to a subject suffering from IBD, deliver an effective amount of an antisense oligonucleotide to the intestinal system of a patient, *e.g.,* deliver an effective amount of an antisense oligonucleotide to the terminal ileum and/or right colon of a patient.

In some embodiments of the invention, the anti-SMAD7 therapy *(i.e.,* a therapy comprising a SMAD7 antisense oligonucleotide) may be suitable for oral delivery of an antisense oligonucleotide, *e.g.,* tablets, that include an enteric coating, *e.g.,* a gastro-resistant coating, such that the compositions may deliver the antisense compound to, *e.g.,* the terminal ileum and right colon of a patient. For example, such administration may result in a topical effect, substantially topically applying the antisense compound directly to an affected portion of the intestine of a subject. Such administration, may, in some embodiments, substantially avoid unwanted systemic absorption of the antisense compound.

For example, a tablet for oral administration may comprise granules (*e.g.,* is at least partially formed from granules) that include a disclosed antisense compound and pharmaceutically acceptable excipients. Such a tablet may be coated with an enteric coating. Contemplated tablets may include pharmaceutically acceptable excipients such as fillers, binders, disintegrants, and/or lubricants, as well as coloring agents, release agents, coating agents, sweetening, flavoring such as wintergreen, orange, xylitol, sorbitol, fructose, and maltodextrin, and perfuming agents, preservatives and/or antioxidants.

In some embodiments, contemplated pharmaceutical formulations include an intra-granular phase that includes a contemplated antisense compound or a pharmaceutically acceptable salt and a pharmaceutically acceptable filler. For example, Mongersen and a filler may be blended together, with optionally other excipients, and formed into granules. In some embodiments, the intragranular phase may be formed using wet granulation, *e.g.,* a liquid (*e.g.,* water) is added to the blended antisense compound and filler, and then combination is dried, milled and/or sieved to produce granules. One of skill in the art would understand that other processes may be used to achieve an intragranular phase.

In some embodiments, contemplated formulations include an extra-granular phase, which may include one or more pharmaceutically acceptable excipients, and which may be blended with the intragranular phase to form a disclosed formulation.

An anti-SMAD7 therapy formulation may include an intragranular phase that includes a filler. Exemplary fillers include, but are not limited to, cellulose, gelatin, calcium phosphate, lactose, sucrose, glucose, mannitol, sorbitol, microcrystalline cellulose, pectin, polyacrylates, dextrose, cellulose acetate, hydroxypropylmethyl cellulose, partially pregelatinized starch, calcium carbonate, and others including combinations thereof.

In some embodiments, an anti-SMAD7 therapy formulation may include an intragranular phase and/or an extragranular phase that includes a binder, which may generally function to hold the ingredients of the pharmaceutical formulation together. Exemplary binders include invention may be, but are not limited to, the following: starches, sugars, cellulose or modified cellulose such as hydroxypropyl cellulose, lactose, pregelatinized maize starch, polyvinyl pyrrolidone, hydroxypropyl cellulose, hydroxypropylmethyl cellulose, low substituted hydroxypropyl cellulose, sodium carboxymethyl cellulose, methyl cellulose, ethyl cellulose, sugar alcohols and others including combinations thereof.

Contemplated anti-SMAD7 therapy formulations, *e.g.,* that include an intragranular phase and/or an extragranular phase, may include a disintegrant such as but are not limited to, starch, cellulose, crosslinked polyvinyl pyrrolidone, sodium starch glycolate, sodium carboxymethyl cellulose, alginates, corn starch, crosmellose sodium, crosslinked carboxymethyl cellulose, low substituted hydroxypropyl cellulose, acacia, and others including combinations thereof. For example, an intragranular phase and/or an extragranular phase may include a disintegrant.

In some embodiments, a contemplated anti-SMAD7 therapy formulation includes an intra-granular phase comprising a disclosed antisense compound and excipients chosen from: mannitol, microcrystalline cellulose, hydroxypropylmethyl cellulose, and sodium starch glycolate or combinations thereof, and an extra-granular phase comprising one or more of: microcrystalline cellulose, sodium starch glycolate, and magnesium stearate or mixtures thereof.

In some embodiments, a contemplated anti-SMAD7 therapy formulation may include a lubricant, *e.g.,* an extra-granular phase may contain a lubricant. Lubricants include but are not limited to talc, silica, fats, stearin, magnesium stearate, calcium phosphate, silicone dioxide, calcium silicate, calcium phosphate, colloidal silicon dioxide, metallic stearates, hydrogenated vegetable oil, corn starch, sodium benzoate, polyethylene glycols, sodium acetate, calcium stearate, sodium lauryl sulfate, sodium chloride, magnesium lauryl sulfate, talc, and stearic acid.

In some embodiments, the pharmaceutical formulation comprises an enteric coating. Generally, enteric coatings create a barrier for the oral medication that controls the location at which the drug is absorbed along the digestive tract. Enteric coatings may include a polymer that disintegrates a different rates according to pH. Enteric coatings may include for example, cellulose acetate phthalate, methyl acrylate-methacrylic acid copolymers, cellulose acetate succinate, hydroxylpropylmethyl cellulose phthalate, methyl methacrylate-methacrylic acid copolymers, ethylacrylate-methacrylic acid copolymers, methacrylic acid copolymer type C, polyvinyl acetate-phthalate, and cellulose acetate phthalate.

In some embodiments, the enteric coating includes an anionic, cationic, or neutral copolymer based on methacrylic acid, methacrylic/acrylic esters or their derivatives. In some embodiments, the enteric coating includes an ethylacrylate-methacrylic acid copolymer. Commercially available enteric coatings include Opadry® AMB, Acryl-EZE®, Eudragit® grades. In some embodiments, the enteric coating makes up about 5% to about 10%, about 5% to about 20%, about 8 to about 15%, about 8% to about 18%, about 10% to about 12%, or about 12% to about 16%, of a contemplated tablet by weight.

For example, an anti-SMAD7 therapy in the form of a tablet is provided that comprises or consists essentially of about 0.5% to about 70%, *e.g.,* about 0.5% to about 10%, or about 1% to about 20%, by weight of an antisense oligonucleotide or a pharmaceutically acceptable salt thereof (*e.g.,* Mongersen). Such a tablet may include for example, about 0.5% to about 60% by weight of mannitol, *e.g.,* about 30% to about 50% by weight mannitol, *e.g.,* about 40% by weight mannitol; and/or about 20% to about 40% by weight of microcrystalline cellulose, or about 10% to about 30% by weight of microcrystalline cellulose. For example, a contemplated tablet may comprise an intragranular phase that includes about 30% to about 60%, *e.g.,* about 45% to about 65% by weight, or alternatively, about 5 to about 10% by weight Mongersen, about 30% to about 50%, or alternatively, about 5% to about 15% by weight mannitol, about 5% to about 15% microcrystalline cellulose, about 0% to about 4%, or about 1% to about 7% hydroxypropylmethyl cellulose, and about 0% to about 4%, *e.g.,* about 2% to about 4% sodium starch glycolate by weight.

Exemplary anti-SMAD7 therapy formulations include dosage forms that include or consist essentially of about 10 mg to about 500 mg of Mongersen, for example, tablets that include about 10 mg, about 15 mg, about 20 mg, about 25 mg, about 30 mg, about 35 mg, about 40 mg, about 50 mg, about 60 mg, about 70 mg, about 80 mg, about 90 mg, about 100 mg, about 150 mg, about 200 mg, or about 250 mg of Mongersen are contemplated herein. In one embodiment, the anti-SMAD7 therapy may be a tablet for oral use comprising: about 0.5% to about 10% by weight of an antisense oligonucleotide represented by SEQ ID NO: 4 or a pharmaceutically acceptable salt thereof; about 30% to about 50% by weight mannitol; and about 10% to about 30% by weight microcrystalline cellulose.

In an exemplary embodiment of the invention, a pharmaceutically acceptable tablet for oral administration is provided that includes an intra-granular phase that may comprise about 50% by weight Mongersen (or salt thereof), about 11.5% by weight mannitol, about 10% by weight microcrystalline cellulose, about 3% by weight hydroxypropylmethyl cellulose, and about 2.5% by weight sodium starch glycolate; and an extra-granular phase that may comprise about 20% by weight microcrystalline cellulose, about 2.5% by weight sodium starch glycolate, and about 0.5% by weight magnesium stearate. The tablet may also include an enteric coating.

In another exemplary embodiment, a pharmaceutically acceptable tablet for oral administration is provided that includes or consists essentially of: an intra-granular phase that may comprise or consist essentially of about 5% to about 10%, *e.g.,* about 8% by weight Mongersen (*e.g.,* wherein the internucleotide linkages are each *O*,*O*-linked phophorothioates, and/or salt thereof, *e.g.,* a sodium salt), about 40% by weight mannitol, about 8% by weight microcrystalline cellulose, about 5% by weight hydroxypropylmethyl cellulose, and about 2% by weight sodium starch glycolate; and an extra-granular phase that may comprise about 17% by weight microcrystalline cellulose, about 2% by weight sodium starch glycolate, and about 0.4% by weight magnesium stearate.

Contemplated tablets may also include an enteric coating, *e.g.,* a disclosed tablet may include about 13%, about 14%, about 15%, about 16%, or about 17% by weight of an enteric coating, *e.g.,* ethylacrylate-methacrylic acid copolymers (*e.g.,* AcrylEZE®).

For example, the anti-SMAD7 therapy may be in the form of a pharmaceutically acceptable tablet for oral use comprising an intra-granular phase and extra-granular phase, wherein for example, the intra-granular phase comprises about 5% to about 10%, by weight (for example about 8% by weight) of an antisense oligonucleotide represented by SEQ ID NO: 4 or a pharmaceutically acceptable salt thereof, about 40% by weight mannitol, about 8% by weight microcrystalline cellulose, about 5% by weight hydroxypropylmethyl cellulose, and about 2% by weight sodium starch glycolate, and for example, the extra-granular phase comprises about 17% by weight microcrystalline cellulose, about 2% by weight sodium starch glycolate, and about 0.4% by weight magnesium stearate, where the tablet may further comprise an enteric coating.

Contemplated formulations, *e.g.,* tablets, in some embodiments, when orally administered to the patient may result in minimal plasma concentration of the oligonucleotide in the patient. In another embodiment, contemplated formulations, when orally administered to a patient, topically deliver to the terminal ileum and/or right colon of a patient, *e.g.,* to an affected or diseased intestinal site of a patient.

### Inflammatory bowel disease

The invention described herein provides methods useful for treating and managing IBD. "Inflammatory bowel disease" or "IBD," as used herein, may refer to a number of chronic inflammatory diseases including Crohn's disease (CD), gastroduodenal Crohn's disease, Crohn's (granulomatous) colitis, ulcerative colitis (UC), collagenous colitis, lymphocytic colitis, ischaemic colitis, diversion colitis, Behçet's disease, microscopic colitis, ulcerative proctitis, proctosigmoiditis, jejunoileitis, left-sided colitis, pancolitis, ileocolitis, ileitis, and indeterminate colitis. CD and UC are the two most common forms of IBD. IBD is an autoimmune disease of the digestive system. CD may be localized to any portion of the gastrointestinal tract, including the terminal ileum, and may impact all cell types of the gastrointestinal tract. UC is localized to the colon and rectum, and affects cells of the mucosa only.

Both environmental and genetic factors are believed to play a role in IBD, although the identity of such factors is not well-defined. Environmental components may include alterations in flora of the gut which are affected by exposure to ingested foods and medications.

IBD is associated with symptoms including abdominal pain, vomiting, diarrhea, rectal bleeding, severe cramps, muscle spasms, weight loss, malnutrition, fever, anemia, skin lesions, joint pain, eye inflammation, liver disorders, arthritis, pyoderma gangrenosum, primary sclerosing cholangitis, and non-thyroidal illness syndrome. Children suffering from UC may suffer from growth defects.

Forms of CD include steroid-dependent and steroid-resistant forms of CD, including active CD. Patients with IBD who suffer from a steroid-dependent form of CD are responsive to treatment with steroid therapy, but cannot terminate or curtail steroid therapy without suffering from an increase in occurrence of symptoms associated with CD. Patients with IBD who suffer from a steroid-resistant form of CD are not responsive to treatment with steroid therapy. Steroid therapeutics commonly prescribed and/or administered to patients with IBD include: corticosteroids, for example, prednisone, dexamethasone, hydrocortisone, methylprednisolone, prednisone, and budesonide. A human patient suffering from active CD is a patient actively suffering from symptoms of CD, for example, but not limited to, bloody stool, weight loss, and/or abdominal cramps.

### Treatment and Management

A "patient," as described herein, refers to any animal suffering from or diagnosed for IBD, including, but not limited to, mammals, primates, and humans. In certain embodiments, the subject may be a non-human mammal such as, for example, a cat, a dog, or a horse. In a preferred embodiment, the subject is a human subject.

"A patient with IBD," as used herein, refers to a patient suffering from any of the symptoms or manifestations of IBD, a patient who may suffer from any of the symptoms or manifestations of IBD, or any patient who might benefit from a method of the invention for treating or managing IBD. A patient in need may include a patient who has suffered from IBD in the past, or a patient who has previously been treated for IBD. Of particular relevance are individuals that suffer from IBD associated with increased levels of CCL20, IL8, or TNFα expression. In some embodiments, the patient with IBD is a Crohn's disease (CD) patient. In some embodiments, the patient with IBD is an ulcerative colitis (UC) patient.

The terms "treat," "treatment," "treating," and the like are used herein to generally mean obtaining a desired pharmacological and/or physiological effect. The effect may be therapeutic in terms of partially or completely curing a disease and/or adverse effect attributed to the disease. The term "treatment" as used herein covers any treatment of a disease in a mammal, particularly a human, and includes: (a) inhibiting the disease, *i.e.,* preventing the disease from increasing in severity or scope; (b) relieving the disease, *i.e.,* causing partial or complete amelioration of the disease; or (c) preventing relapse of the disease, *i.e.,* preventing the disease from returning to an active state following previous successful treatment of symptoms of the disease or treatment of the disease.

The terms "manage," "management," "managing," and the like are used herein to generally mean controlling the severity or manifestation of symptoms of a disease, or the means of treating the disease. Generally, management is used to obtaining a desired pharmacological and/or physiological effect. The effect may be therapeutic in terms of partially or completely curing a disease and/or adverse effect attributed to the disease or ensuring that a particular symptom or manifestation of the disease does not occur or reoccur in a patient or does not rise to an undesirable or intolerable level in a patient. The term "management" as used herein covers any management of a disease in a mammal, particularly a human, and includes: (a) inhibiting the disease, *i.e.,* preventing the disease from increasing in severity or scope; (b) relieving the disease, *i.e.,* causing partial or complete amelioration of the disease; or (c) preventing relapse of the disease, *i.e.,* preventing the disease from returning to an active state following previous successful treatment of symptoms of the disease or treatment of the disease. "Management" as used herein may also be used with reference to administration of a specific treatment for the disease, for example, a SMAD7 antisense oligonucleotide.

In some embodiments of the invention, a patient having IBD will be administered an initial dose of an anti-SMAD7 therapy, for instance, a SMAD7 antisense oligonucleotide. As used herein, "initial dose" refers to a dose of an anti-SMAD7 therapy administered to a patient having IBD, in a series of doses. A series of doses may include one or more doses. For instance, a series of doses may comprise a single dose of an anti-SMAD7 therapy or more than a single dose of an anti-SMAD7 therapy. An initial dose may be a dose of an anti-SMAD7 therapy administered to a patient prior to any later dose administered to the patient. For instance, an initial dose may be, but is not limited to, the first dose of an anti-SMAD7 therapy administered to a treatment-naive patient. An initial dose may also be a first dose in any treatment cycle of the anti-SMAD7 therapy. For example, an initial dose may be the first dose of a first treatment cycle, of a second treatment cycle, or of any subsequent treatment cycles. Alternatively, an "initial dose" may be the first dose administered to a patient after analyzing levels of CCL20, IL8, or TNFα and/or another biomarker or biomarkers in a patient, or may be the most recently administered dose before a determination of the levels of CCL20, IL8, or TNFα and/or another biomarker or biomarkers in a patient.

In some embodiments of the invention, a patient having IBD will be administered a subsequent dose of an anti-SMAD7 therapy, for instance, a SMAD7 antisense oligonucleotide. As used herein, "subsequent dose" refers to a dose of an anti-SMAD7 therapy administered to a patient having IBD, after administration of a prior dose, for example, an initial dose. Thus, a subsequent dose may be administered to a patient having IBD in a series of doses comprising two or more doses. Furthermore, in some instances, the amount of a subsequent dose may be calibrated with respect to an initial dose or a prior dose, such that a subsequence dose is greater, equal to, or lesser than a prior dose. Calibration of the amount of a subsequent dose may be based on levels or changes in levels of CCL20, IL8, or TNFα and/or another biomarker or biomarkers in a patient having IBD, for instance: levels of CCL20, IL8, or TNFα in a patient having IBD analyzed prior to or after a prior dose, for instance, an initial dose; or changes in CCL20, IL8, or TNFα levels in a patient having IBD before and after a prior dose, for instance, an initial dose. A subsequent dose may be a dose administered to a patient having IBD after a first dose, for instance, an initial dose, of an anti-SMAD7 therapy administered to a patient having IBD. A subsequent dose may also be a dose administered after a prior dose of an anti-SMAD7 therapy administered to a patient having IBD, for instance, a dose administered after a prior dose in the same round of treatment or a different round of treatment, for instance, a previous round of treatment. A subsequent dose may be a subsequent dose with respect to any prior dose, for instance, a prior dose immediately preceding the subsequent dose or a prior dose followed by one or more doses administered prior to administration of the subsequent dose.

As used herein, "Crohn's Disease Activity Index" or "CDAI" refers to a measurement or index used to assess the progress of patients suffering from CD as described by Best et al., GASTROENTEROLOGY, 70:439-44 (1976). CDAI scores of 150 or below are generally associated with inactive disease and are indicative of better prognosis than higher scores. Values above 150 are generally associated with active disease and values above 450 are associated with extremely severe disease. CDAI scores may be used to determine how well a patient is responding to therapy and may be used to identify patients in remission. In certain embodiments, a benchmark clinical response means that the subject displays a decrease in CDAI score by at least 100 points. In a clinical trial, a CDAI score of 150 or below is generally associated with remission.

As used herein, "Ulcerative Colitis Disease Activity Index" or "UCDAI" refers to a measurement or index used to assess the progress of patients suffering from UC as described by Sutherland et al., Gastroenterology, 92:1894-98 (1987). The UCDAI is a series of qualifiers about the symptoms of UC including stool frequency, rectal bleeding, the appearance of the colon lining, and a physician's rating of disease activity. Each of these qualifiers is given a number from 0 to 3, with 3 being the highest disease activity. In a clinical trial, remission is often defined as a UCDAI score of 1 or less, and improvement is a reduction of 3 or more points from the score at the beginning of the trial. UCDAI may be used in clinical trials to determine how well a patient is responding to therapy and may be used to identify patients in remission. Other commonly used indices for measuring disease severity in UC patients include the Truelove and Witts Index, the St. Mark's Index, the Simple Clinical Colitis Activity Index (SCCAI), the Lichtiger Index, the Ulcerative Colitis Symptom Score (UCSS), and the Mayo Clinic Score.

As used herein, "remission" or "clinical remission" refers to a reduction - partial or complete - of a clinical manifestation or manifestations or symptoms of IBD. For instance, clinical remission may include an easing of the severity of symptoms associated with IBD, for example, bloody stool, weight loss, or tissue inflammation, or a complete disappearance of symptoms of IBD from the patient. In embodiments of the invention, clinical remission may be indicated by changes in or measurement of CDAI score. For example, a CDAI score of less than about 150, less than about 155, less than about 160, less than about 165, less than about 170, or less than about 175 may indicate clinical remission. Clinical remission may be observed following administration of the SMAD7 antisense oligonucleotide to the patient having IBD. Clinical remission may be observed by measuring factors that contribute to a disease activity index score, such as a CDAI score, or by measurement of objective factors, for instance, but not limited to, levels of analytes in a sample from a patient. Furthermore, in some embodiments of the invention, clinical remission is both observed after an administration step and maintained for a period of time following the initial observation. For instance, in some embodiments clinical remission is observed about one week, about two weeks, or about three weeks after administration of the SMAD7 antisense oligonucleotide and maintained for a period of at least 1 week, at least 2 weeks, at least 3 weeks, at least 4 weeks, at least 6 weeks or at least 8 weeks following the initial observation of clinical remission.

As used herein, "SMAD7" (also known as CRCS3, FLJ16482, MADH7, MADH8, MAD (mothers against decapentaplegic, Drosophila) homolog 7, MAD homolog 8, SMAD, mothers against DPP homolog 7, mothers against DPP homolog 8) means the human protein or any of the mRNA transcripts encoded by the gene identified by Entrez GenelD No. 4092 and allelic variants thereof.

As used herein, "IL8" (also known as Interleukin-8 (IL-8); Tumor Necrosis Factor-Induced Gene 1; NAF; Granulocyte Chemotactic Protein 1 (GCP1); LECT; LUCT; Protein 3-10C; Beta-Thromboglobulin-Like Protein; Neutrophil-Activating Peptide 1; Neutrophil-Activating Protein 1 (NAP1; NAP-1); Emoctakin; GCP-1; LYNAP; Lymphocyte Derived Neutrophil Activating Peptide; Lung Giant Cell Carcinoma-Derived Chemotactic Protein; Small Inducible Cytokine Subfamily B, Member 8; Beta Endothelial Cell-Derived Neutrophil Activating Peptide; Monocyte-Derived Neutrophil Chemotactic Factor (MDNCF); Monocyte-Derived Neutrophil-Activating Peptide (MONAP); Alveolar Macrophage Chemotactic Factor I; C-X-C Motif Chemokine 8; and Chemokine (C-X-C Motif) Ligand 8 (CXCL8)) means the human protein or any of the mRNA transcripts encoded by the gene identified by Entrez GenelD No. 3576 and allelic variants thereof.

As used herein, "CRP" (also known as C-reactive protein, pentraxin-related; Pentraxin; and PTX1) means the human protein or any of the mRNA transcripts encoded by the gene identified by Entrez GenelD No. 1401 and allelic variants thereof.

As used herein, "TNFα" (also known as Tumor Necrosis Factor, DIF, Tumor Necrosis Factor Ligand Superfamily Member 2 (TNFSF2), APC1 Protein, cachectin, Tumor Necrosis Factor A (TNFA), Tumor Necrosis Factor-a (TNF-a), and Tumor Necrosis Factor-alpha (TNF-alpha)) means the human protein or any of the mRNA transcripts encoded by the gene identified by Entrez GenelD No. 7124 and allelic variants thereof.

As used herein, "CCL20" (also known as Chemokine (C-C Motif) Ligand 20; CKb4; LARC; ST38; MIP3A; Exodus; Macrophage Inflammatory Protein 3 Alpha (MIP-3a, MIP-3-alpha); SCYA20; Small Inducible Cytokine Subfamily A (Cys-Cys), Member 20; Liver And Activation-Regulated Chemokine; Small-Inducible Cytokine A20; CC Chemokine LARC; ST38; Beta Chemokine Exodus-1; and C-C Motif Chemokine 20) means the human protein or any of the mRNA transcripts encoded by the gene identified by Entrez GenelD No. 6364 and allelic variants thereof.

### Methods of Monitoring Treatment

In some embodiments, the methods described herein entail monitoring the treatment, disease state, or biomarkers associated with a disease state of a patient having IBD. Monitoring treatment may be useful in terms of assessing treatment efficacy and safety, as well as evaluating the need to modulate treatment. Monitoring treatment may also be useful for evaluating whether the amount of SMAD7 antisense oligonucleotide being administered to a patient or which will be administered to a patient should be increased or decreased. Furthermore, monitoring treatment may be useful in terms of determining the amount or relative amount by which a dose of SMAD7 antisense oligonucleotide should be modulated, *i.e.,* increased or decreased.

Monitoring, for example, monitoring of CCL20, IL8, or TNFα levels in a patient having IBD, may commence prior to, during, or after an initial dose of a SMAD7 antisense oligonucleotide. Furthermore, monitoring may continue after an initial dose. For example monitoring may be performed after administration of an initial dose. Monitoring may also be performed before, during, or after a subsequent dose of SMAD7 antisense oligonucleotide. Monitoring may be continuous or discontinuous such that monitoring may be performed at regular intervals, for example, after each dose of a SMAD7 antisense oligonucleotide is administered to a patient, before each dose of a SMAD7 antisense oligonucleotide is administered to a patient, or before and after each dose of a SMAD7 antisense oligonucleotide is administered to a patient. Monitoring may be performed multiple times in a single day (for instance, 2 times, 3 times, 4 times, about five times, or about 10 times in a single day), once a day, multiple times in a single week (for instance, 2 times, 3 times, 4 times, about five times, or about 10 times in a single week), once a week, multiple times in a single month (for instance, 2 times, 3 times, 4 times, about five times, or about 10 times in a single month), or once a month. In methods of the invention, monitoring may be performed at various times relative to an administering step. For instance, in some embodiments, monitoring may be performed immediately after, or at least 1 day, at least 3 days, at least 5 days, at least 1 week, at least 2 weeks, at least 3 weeks, at least 1 month, at least 2 months, at least 4 months, or at least 6 months after an administration step. In some embodiments, monitoring is performed about 15 days or about 28 days after an administration step.

As described above, the invention is based in part on the discovery that levels of CCL20, IL8, or TNFα can be used to evaluate and modify management and treatment with a SMAD7 antisense oligonucleotide in a patient having IBD. Thus, in embodiments of the invention, it is useful to know, determine, analyze, or compare levels of CCL20, IL8, or TNFα in a patient or a sample from a patient having IBD. For example, in some instances it will be useful to know a threshold value for normal or abnormal levels of CCL20, IL8, or TNFα in order to determine whether levels of the SMAD7 antisense oligonucleotide should be increased, decreased, or left untouched. In the methods described herein, a normal level of CCL20 may be tied to a specific value, for instance, a value of 0.01 pg/ml, about 0.1 pg/ml, about 1 pg/ml, about 2 pg/ml, about 3 pg/ml, about 4 pg/ml, about 5 pg/ml, about 6 pg/ml, about 7 pg/ml, about 8 pg/ml, about 9 pg/ml, about 10 pg/ml, about 11 pg/ml, about 12 pg/ml, about 13 pg/ml, about 14 pg/ml, about 15 pg/ml, about 17.5 pg/ml, about 20 pg/ml, about 22.5 pg/ml, about 25 pg/ml, about 30 pg/ml, or about 35 pg/ml. In the methods described herein, a normal level of IL8 may be tied to a specific value, for instance, a value of about 10 pg/ml, about 12.5 pg/ml, about 15 pg/ml, about 16 pg/ml, about 17 pg/ml, or about 18 pg/ml, about 19 pg/ml, about 20 pg/ml, or about 25 pg/ml. In the methods described herein, a normal level of TNFα may be tied to a specific value, for instance, a value of about 10 pg/ml, about 12.5 pg/ml, about 15 pg/ml, about 16 pg/ml, about 17 pg/ml, or about 18 pg/ml, about 19pg/ml, about 20 pg/ml, or about 25 pg/ml. In some embodiments, a normal level of CCL20, IL8, or TNFα may be determined by comparison to median levels of CCL20, IL8, or TNFα in a healthy control group that is matched to the patient with respect to various factors, for example, age, gender, ethnic origin, smoking habits, dietary habits, body-mass index (BMI), and/or exercise habits.

Levels of CCL20, IL8, or TNFα and/or other analytes may be determined by obtaining a sample from the patient. According to the methods described herein, a sample may be a tissue sample (*e.g.,* a gastrointestinal tissue sample) or a bodily fluid sample (*e.g.,* a saliva sample, a stool, or a urine sample). A sample can be a sample obtained from a patient tissue biopsy, for example, a mucosal tissue biopsy, for example, an intestinal mucosal tissue biopsy. Furthermore, the sample may be a blood, serum, or plasma sample. A blood sample from a subject may be obtained using techniques well-known in the art. Blood samples may include peripheral blood mononuclear cells (PMBCs), RBC-depleted whole blood, or blood serum. PBMCs can be separated from whole blood samples using different density gradient (*e.g.,* Ficoll density gradient) centrifugation procedures. For example, whole blood (*e.g.,* anticoagulated whole blood) is layered over the separating medium and centrifuged. At the end of the centrifugation step, the following layers are visually observed from top to bottom: plasma/platelets, PBMC, separating medium and erythrocytes/granulocytes.

Methods of monitoring treatment may also include methods of monitoring other factors, including, but not limited to levels of other analytes (*e.g.,* CCL20, IL8, CRP, TNFα), CDAI score, clinical remission, and presence or severity of IBD symptoms.

In embodiments of the invention where levels of an analyte (*e.g.,* CCL20, IL8, or TNFα) are measured, various methods may be used to measure the analyte. For example, the level of an analyte, for example, CCL20, IL8, TNFα, or CRP, may be determined by immunochemistry and/or by nucleotide analysis. Methods of determining analyte concentration by immunochemistry include, but are not limited to, Western blotting, ELISA, and immunostaining methods. In some embodiments, a method of determining analyte concentration by immunochemistry is performed using an antibody that can bind to the analyte of interest, for instance, an anti- CCL20 antibody, an anti-IL8 antibody, or an anti-TNFa antibody. Methods of determining analyte concentration by immunochemistry may also involve the use of buffers, blocking reagents, unconjugated primary antibodies, and primary and/or secondary antibodies conjugated to tags that allow for antibody detection, such as fluorescent probes or substrate-specific enzymes.

Methods of determining analyte concentration by nucleotide analysis include, but are not limited to, methods of analyzing analyte mRNA transcript levels such as Northern blotting and polymerase chain reaction methods, for example, quantitative polymerase chain reaction methods. Nucleotide analysis may be performed using an oligonucleotide probe that binds an analyte nucleotide sequence (*e.g.,* a CCL20, IL8, or TNFα nucleotide sequence) or a pair of oligonucleotide primers capable of amplifying an analyte nucleotide sequence via a polymerase chain reaction, for example, by a quantitative polymerase chain reaction. Oligonucleotide probes and oligonucleotide primers may be linked to a detectable tag, such as, for example, a fluorescent tag. In determining analyte concentration by nucleotide analysis, the practitioner may evaluate a particular analyte's mRNA transcript concentration in a sample. Alternatively, in determining analyte concentration by nucleotide analysis, the practitioner may establish a correlation between a particular analyte's mRNA transcript abundance and the particular analyte's protein abundance in order to extrapolate analyte protein concentration based on a measure of analyte mRNA transcript abundance.

Methods of the claimed invention include steps that may be carried out *in vitro.* For instance, it is contemplated that the steps of measuring CCL20, IL8, or TNFα levels in the subject, determining the levels of CCL20, IL8, or TNFα in a sample, and/or determining CDAI score or taking measurements necessary to determine CDAI score may be carried out *in vitro.* For example, the level of CCL20, IL8, or TNFα in a sample may be determined by performing immunochemistry or nucleotide analysis on the sample *in vitro.* Alternatively, in some embodiments of the invention, the steps of determining and analyzing the CCL20, IL8, or TNFα levels in a patient having IBD, determining and analyzing the CCL20, IL8, or TNFα levels in a sample, and/or determining CDAI score or taking measurements necessary to determine CDAI score may be carried out *in vivo.*

Anti-IL8 antibodies suitable for immunochemistry are commercially available, including, but not limited to, goat anti-human IL8 from Abcam (Cat. No. ab 10769), mouse anti-human IL8 from Santa Cruz (Cat. Nos. sc-73321, sc-52870, and sc-7302), mouse anti-human IL8 (3IL8-H10) from Pierce (Cat. No. M801), and a mouse anti-human IL8 from Sigma-Aldrich (Cat. No. WH0003576M5) antibody.

Anti-TNFα antibodies suitable for immunochemistry are commercially available, including, but not limited to, rabbit anti-human TNFα from Abcam (Cat. No. ab9635), rabbit anti-human TNFα from Cell Signaling Technology (Cat. No. 3707), mouse anti-human TNFα from affymetrix eBioscience (Cat. No. 14-7348-81), and rabbit anti-human TNFα from Rockland Antibodies & Assays (Cat. No. 209-401-306S) antibody.

Anti-CRP antibodies suitable for immunochemistry are commercially available, such as, for example, goat anti-human CRP polyclonal antibodies from Santa Cruz Biotechnology (Catalog Numbers sc-18304 and sc-18306), a rabbit anti-human CRP polyclonal antibody from from Santa Cruz Biotechnology (Catalog Number sc-30047), a mouse anti-human CRP monoclonal antibody from Santa Cruz Biotechnology (Catalog Number sc-70883), a mouse anti-human CRP monoclonal antibody from Sigma-Aldrich (Catalog Number C1688-.2ML), a rabbit anti-human monoclonal antibody from abcam (Catalog Number ab32412), a mouse anti-human CRP monoclonal antibody from abcam (Catalog Number ab 13426), and a goat anti-human CRP polyclonal antibody from Thermo Scientific (Catalog Number G0301-1B).

Anti-CCL20 antibodies suitable for immunochemistry are commercially available, for example, a mouse anti-human CCL20 monoclonal antibody from R&D Systems (Catalog Number MAB360), a goat anti-human CCL20 polyclonal antibody from Sigma Aldrich (Catalog Number SAB2501804), a mouse anti-human CCL20 monoclonal antibody from Origene (Catalog Number TA316597), a goat anti-human CCL20 polyclonal antibody from Origene (Catalog Number TA316596), a goat anti-human CCL20 polyclonal antibody from Abnova (Catalog Number PAB17268), a rabbit anti-human CCL20 polyclonal antibody from Abnova (Catalog Number PAB16925), a mouse anti-human CCL20 monoclonal antibody from Abnova (Catalog Number MAB1314), a goat anti-human CCL20 polyclonal antibody from Santa Cruz Biotechnology (Catalog Number sc-9775), and a rabbit anti-human CCL20 polyclonal antibody from Abcam (Catalog Number ab9829).

### Enzyme-Linked Immunosorbent Assay

In some embodiments, CCL20, IL8, or TNFα and/or other analyte concentration may be determined by Enzyme-linked immunosorbent assay (ELISA). Specifically, levels of CCL20, IL8, or TNFα and/or other analytes in a sample, especially a blood sample, for example, a blood serum sample, can be determined by ELISA. Assaying analyte concentration by ELISA requires at least one antibody against the analyte protein, *e.g.,* at least one anti-CCL20 antibody, anti-IL8 antibody, or anti-TNFa antibody, and/or at least one secondary antibody, *e.g.,* at least one labeled secondary antibody. In some embodiments, the primary antibody is labeled with, *e.g.,* a fluorescent label. In certain embodiments, the primary antibody is not labeled and a secondary antibody capable of binding the species isotype of the primary antibody is labeled, *e.g.,* with a fluorescent probe or enzyme capable of reacting with a specific substrate, thereby providing a detectable signal.

Performing an ELISA requires at least one capture antibody, at least one detection antibody, and/or at least one enzyme-linked or fluorescent labeled secondary antibody. For example, assaying CCL20, IL8, or TNFα levels by ELISA may require an anti-CCL20 antibody, anti-IL8 antibody, or anti-TNFα antibody, respectively, as the capture antibody. The anti-CCL20 antibody, anti-IL8 antibody, or anti-TNFα antibody is immobilized on a solid support such as a polystyrene microtiter plate. A sample, for example, a blood serum sample is then added and allowed to complex with the bound antibody. Unbound serum components are removed with a wash. A detection antibody, *e.g.,* a different anti-CCL20 antibody, anti-IL8 antibody, or anti-TNFα antibody, *e.g.,* an anti-CCL20 antibody, anti-IL8 antibody, or anti-TNFα antibody, that binds to a different portion of the CCL20, IL8, or TNFα protein, respectively, than the capture antibody, is added and is allowed to bind to the captured CCL20, IL8, or TNFα. The detection antibody is linked to a detectable tag, such as an enzyme, either directly or indirectly, *e.g.,* through a secondary antibody that specifically recognizes the detection antibody. Typically between each step, the plate, with bound protein, is washed with a wash buffer, *e.g.,* a mild detergent solution. Typical ELISA protocols also include one or more blocking steps, which involve use of a non-specifically-binding protein such as bovine serum albumin to block unwanted non-specific binding of protein reagents to the plate. After a final wash step, the plate is developed by addition of an appropriate enzyme substrate, to produce a visible signal, which indicates the amount of CCL20, IL8, or TNFα protein in the sample. The substrate can be, *e.g.,* a chromogenic substrate or a fluorogenic substrate. ELISA methods, reagents and equipment are well-known in the art and commercially available.

### Nucleotide Analysis

In some embodiments, levels of CCL20, IL8, or TNFα and/or other analytes may be determined by performing a "nucleotide analysis." A nucleotide analysis may include analysis of analyte nucleotide transcript levels (*e.g.,* CCL20, IL8, or TNFα mRNA transcript levels) in a sample, for example, a blood sample. Analyte transcript levels may be determined by Northern blot, for example, a quantitative Northern blot; or polymerase chain reaction, for example, a quantitative polymerase chain reaction. Reagents necessary to perform Northern blot include oligonucleotide probes, for example, oligonucleotide probes linked to a detectable label. Detectable labels may include fluorescent labels or enzymes capable of reacting with a specific substrate. Reagents necessary to perform polymerase chain reaction include oligonucleotide primers capable of specifically binding to a particular analyte mRNA transcript and amplifying the number of analyte mRNA transcripts by polymerase chain reaction. Oligonucleotide primers may be linked to a detectable label to enable, for example, quantitative polymerase chain reaction. Other reagents necessary to perform quantitative polymerase chain reaction include, but are not limited to, primers capable of amplifying a control transcript signal, for instance, a beta tubulin transcript signal. Buffers, reagents (including oligonucleotide primers and probes), techniques, and equipment necessary for performing Northern blotting and polymerase chain reactions are readily available and are well-known in the art.

### Methods of Selecting Patients

The invention described herein provides methods of treating patients in part by selecting patients that show some likelihood of responsiveness to SMAD7-antisense therapy. Likeliness of responsiveness to anti-SMAD7 therapy is premised in part on determining levels of CCL20, IL8, or TNFα in a patient with IBD, for example, preexisting levels of CCL20, IL8, or TNFα (*i.e.,* levels of CCL20, IL8, or TNFα in a patient prior to administration of an initial dose of a SMAD7 antisense oligonucleotide) or levels of CCL20, IL8, or TNFα determined after an initial dose or one or more subsequent doses of SMAD7 antisense oligonucleotide. For instance, in some embodiments of the invention, a patient will be selected for treatment or further treatment with a SMAD7 antisense oligonucleotide after detecting or analyzing absolute or relative CCL20, IL8, or TNFα levels or changes in CCL20, IL8, or TNFα levels. Levels of CCL20, IL8, or TNFα in a patient with IBD may be compared to a normal level of CCL20, IL8, or TNFα, for example, normal levels of CCL20, IL8, or TNFα as defined by median CCL20, IL8, or TNFα levels in a matched control group or absolute levels of CCL20, IL8, or TNFα.

In some embodiments, a patient will be selected for treatment or further treatment with a SMAD7 antisense oligonucleotide if the levels of CCL20, IL8, or TNFα in the patient are more than 10%, 20%, 30%, 40%, 50%, 60%, 70%, 80%, 90%, or 100% elevated relative to the average, median or mean levels of CCL20, IL8, or TNFα in a matched control group.

In some embodiments, a patient will be selected for treatment or further treatment with a SMAD7 antisense oligonucleotide if the level of CCL20, IL8, or TNFα in the patient are more than 2-fold, more than 3-fold, more than 4-fold, more than 5-fold, more than 6-fold, more than 7-fold, more than 8-fold, more than 9-fold or more than 10-fold elevated relative to the average, median or mean levels of CCL20, IL8, or TNFα in a matched control group.

Typically CCL20, IL8, or TNFα levels will be measured in terms of a concentration, for instance, mass of CCL20, IL8, or TNFα protein, peptide, or RNA per volume of sample, for example, volume of blood or tissue. Thus selection of patients for initial or continued treatment is tied to CCL20, IL8, or TNFα levels in the patient, such that, for example, high initial levels of CCL20, IL8, or TNFα may indicate a potential for responsiveness to SMAD7 antisense oligonucleotide treatment. Furthermore, high levels of CCL20, IL8, or TNFα (*i.e.,* above normal levels of CCL20, IL8, or TNFα) may indicate a need for increased doses of SMAD7 antisense oligonucleotide, whereas normal or below normal levels of CCL20, IL8, or TNFα may indicate a need for decreased or unchanged doses of SMAD7 antisense oligonucleotide, especially following one or more doses. Alternatively, continued above normal levels of CCL20, IL8, or TNFα after repeated doses may indicate that the patient is not responsive to treatment.

Thus, if levels of CCL20, IL8, or TNFα are above normal levels of CCL20, IL8, or TNFα, a patient may be administered an initial and/or subsequent dose of SMAD7 antisense oligonucleotide. In some embodiments, CCL20, IL8, or TNFα levels are already known to be above normal CCL20, IL8, or TNFα levels prior to administration of an initial dose. In some embodiments, CCL20, IL8, or TNFα levels in a patient with IBD will be determined prior to administration of an initial dose. In some embodiments, after an initial dose of SMAD7 antisense oligonucleotide, if CCL20, IL8, or TNFα levels are analyzed and determined to be above normal levels of CCL20, IL8, or TNFα, the patient will be administered a subsequent dose of SMAD7 antisense oligonucleotide, for instance a greater dose than the initial dose or a dose equal to the initial dose. Alternatively, if CCL20, IL8, or TNFα levels are analyzed and determined to be below normal levels of CCL20, IL8, or TNFα, the patient may be administered a subsequent dose of SMAD7 antisense oligonucleotide, for instance an equal or smaller dose than the initial dose.

In yet other embodiments, CCL20, IL8, or TNFα levels may be analyzed and determined in a patient with IBD, and then an initial dose of SMAD7 antisense oligonucleotide may be administered to the patient if the CCL20, IL8, or TNFα levels are above normal levels of CCL20, IL8, or TNFα. Furthermore, in some embodiments, after an initial dose of SMAD7 antisense oligonucleotide, levels of CCL20, IL8, or TNFα may be determined, and if the levels of CCL20, IL8, or TNFα are above normal levels of CCL20, IL8, or TNFα then a subsequent dose of SMAD7 antisense oligonucleotide that is greater than or equal to the initial dose may be administered to the patient. Alternatively, after an initial dose of SMAD7 antisense oligonucleotide, levels of CCL20, IL8, or TNFα may be determined, and if the levels of CCL20, IL8, or TNFα are below normal levels of CCL20, IL8, or TNFα then a subsequent dose of SMAD7 antisense oligonucleotide that is smaller than or equal to the initial dose may be administered to the patient.

In yet other embodiments, the invention provides methods whereby: CCL20, IL8, or TNFα levels may be analyzed and determined in a patient with IBD; an initial dose of SMAD7 antisense oligonucleotide may be administered to the patient if the CCL20, IL8, or TNFα levels are above normal levels of CCL20, IL8, or TNFα; the levels of CCL20, IL8, or TNFα are analyzed after the initial administration; and if the level of CCL20, IL8, or TNFα after the initial dose is administered is lower than the level of CCL20, IL8, or TNFα before the initial dose is administered, then the patient is administered a subsequent dose that is the same as the initial dose or smaller than the initial dose. Alternatively, if the level of CCL20, IL8, or TNFα is unchanged or increased after the initial dose is administered compared to the level of CCL20, IL8, or TNFα before the initial dose is administered, then the patient is administered a subsequent dose that is the same as the initial dose or greater than the initial dose or treatment is terminated.

Thus, the contemplated invention provides different methods for treating and managing IBD in a patient by accounting for multiple treatment scenarios based on analysis and determination of CCL20, IL8, or TNFα levels and patient responsiveness to SMAD7 antisense oligonucleotide administration.

For instance, if after administration of a SMAD7 antisense oligonucleotide CCL20, IL8, or TNFα levels in a patient are above normal CCL20, IL8, or TNFα levels, treatment can continue at the same dose or at an increased dose of the SMAD7 antisense oligonucleotide.

If after administration of a SMAD7 antisense oligonucleotide CCL20, IL8, or TNFα levels in a patient are below normal CCL20, IL8, or TNFα levels, treatment can continue at the same dose or at a decreased dose of the SMAD7 antisense oligonucleotide.

If after an initial dose and one or more subsequent doses of a SMAD7 antisense oligonucleotide CCL20, IL8, or TNFα levels continue to be above or below normal CCL20, IL8, or TNFα levels, the treatment may be terminated. For example, treatment may be terminated either because the patient is in remission, because the patient is not responsive to treatment, or the patient has been administered the maximum tolerated dose.

In some instances, if CCL20, IL8, or TNFα levels decrease in a patient following one or more doses of the SMAD7 antisense oligonucleotide, this may indicate that a patient is responsive to treatment. In these patients, subsequent doses of the SMAD7 antisense oligonucleotide may be administered but at the same dose or a smaller dose compared to the previous dose(s).

In some instances, if CCL20, IL8, or TNFα levels are stable or increase following an initial or one or more subsequent doses of the SMAD7 antisense oligonucleotide, this may indicate that a patient is not responsive to treatment. In these patients, subsequent doses of the SMAD7 antisense oligonucleotide may be administered but at a greater dose compared to the previous dose(s). Alternatively, the treatment can be discontinued, for example, if the dose approaches the maximum tolerated dose.

In some instances, if a patient achieves clinical remission, as determined by clinical factors other than CCL20, IL8, or TNFα levels, but CCL20, IL8, or TNFα levels remain essentially unchanged or above normal after administration of a SMAD7 antisense oligonucleotide, then the SMAD7 antisense oligonucleotide treatments may be terminated. In such a case, CCL20, IL8, or TNFα levels may not be indicative of IBD progression, but may be elevated due to other factors, e.g., another inflammatory disease.

### Examples

The invention is further illustrated by the following examples. The examples are provided for illustrative purposes only, and are not to be construed as limiting the scope or content of the invention in any way.

### Example 1: Design of a Phase 2 Clinical Trial to Evaluate Safety and Efficacy of an Anti-SMAD7 Antisense Treatment in CD Patients

A phase 2 clinical trial was conducted to evaluate the safety and efficacy of an anti-SMAD7 antisense treatment in patients with CD. Male or female CD patients eligible for the trial were 18-75 years old and had a CDAI score ranging from 220 to 400 for at least one week prior to enrollment. Patients enrolled in the trial had inflammatory lesions in the terminal ileum and/or right colon and were steroid-dependent and/or steroid-resistant. Concomitant mesalamine and steroid therapy was maintained during the clinical trial at a stable dose in patients already receiving those drugs. Eligible patients had the option of continuing to take immunomodulators (*e.g.,* azathioprine, mercaptopurine, methotrexate) if such therapy had begun at least 6 months before the treatment phase of the study commenced, and the patients did not receive treatment with anti-TNF-a antibodies or other biologics during the 90 days prior to enrollment in the trial.

The anti-SMAD7 therapy evaluated in the trial was an oral dosage form that included an enteric coating which allowed targeted topical delivery of the anti-SMAD7 antisense oligonucleotide Mongersen to the terminal ileum and right colon. Because the active compound of the oral dosage form of Mongersen was released in the terminal ileum and right colon, patients with lesions in the stomach and/or the proximal small intestine and/or the transverse and/or left colon were excluded from the trial. Patients were also excluded if they had strictures, fistulae or perianal disease or if they had extraintestinal manifestations. Patients were also excluded if they had active or recent infections or a history of malignancy. Patients were also excluded from the trial if they had undergone proctocolectomy or intestinal resection resulting in the short-bowel syndrome, or had experienced a clinically significant abnormality on an electrocardiogram or clinically significant laboratory abnormalities. Women who were pregnant or breast-feeding were also excluded. Female patients enrolled in the trial were required to use two forms of contraception throughout the study period.

The study was conducted as a multicenter, randomized, placebo-controlled, double-blind, phase 2 clinical trial. After providing written informed consent, patients entered a 9-day screening phase to determine eligibility and pre-treatment measurements. Patients were screened for eligibility at 17 centers in Italy and Germany after the local institutional review boards and ethics committees had approved the clinical trial protocol. Patients who were determined eligible for the trial were randomly assigned into active treatment and placebo groups. The trial included 1 placebo group and 3 experimental groups receiving varying doses of Mongersen (10 mg/day, 40 mg/day, and 160 mg/day). Patients were assigned to these groups in a 1:1:1:1 ratio, using an independent, computer-generated randomization schedule without stratification or block allocation. The three doses of the active drug were selected based on data generated by preclinical, toxicology and phase 1 studies. The four groups of patients received treatment of either placebo or one of the three doses of Mongersen for two weeks (*i.e.,* days 1-14). Following the two week treatment, patients were evaluated on days 15, 28, and 84 (*i.e.,* 1 day after stopping treatment, 2 weeks after stopping treatment, or 10 weeks after stopping treatment).

The study used different measurements to determine efficacy of treatment with Mongersen in IBD patients enrolled in the trial. Rates of remission, defined as a CDAI score of less than 150 at day 15 and maintained for at least 2 weeks, were assessed in patients. Rates of clinical response - defined as a decrease in patient CDAI score of at least 100 or 70 points as evaluated on day 15 or day 28 (*i.e.,* 1 day or 2 weeks, respectively, after stopping drug treatment) - were also evaluated. The number of patients with a CDAI score less than 150 at each time point (*i.e.,* day 15, 28, and 84) was also evaluated. Patient CDAI scores were measured before treatment and during the week preceding days 15, 28, and day 84. The percentage of patients who achieved normalization of CRP levels (*i.e.,* CRP concentration of less than 3.0 mg/liter) at the end of treatment was also determined. Finally, the percentage of patients who had elevated CRP levels (*i.e.,* greater than 3.0 mg/liter) before treatment and who also reached clinical remission was evaluated.

Patients who experienced disease worsening (≥70 CDAI score increase) could receive rescue therapy with biologics and/or immunosuppressive drugs after the 2-week treatment period. Six patients (two in the placebo group, one in the 10 mg/day Mongersen group, and three in the 160 mg/day Mongersen group) received rescue therapy with biologics and/or immunosuppressive drugs due to a worsening of disease after day 28 and were considered non-responders (CDAI > 150) in the analysis of the secondary endpoints. Patients who were in clinical remission after the 2-week treatment period (CDAI <150 at both day 15 and day 28) could taper steroids.

Safety of Mongersen treatment was also assessed. Changes in clinical, biochemical, and hematologic variables were assessed on days 1, 7, 15, 28, and 84. ELISA was used to monitor complement activation in patients. Instances of adverse events (AE) that occurred during the trial were evaluated for severity, and the cause (*i.e.,* study drug or procedure) of each AE was determined. The analysis of AE included all 166 patients who underwent randomization.

The following considerations were taken into account when establishing clinical trial parameters and performing statistical analysis of the data obtained from the clinical trial. Sample size was determined using a one-sided testing framework with an alpha error of 0.1 and a beta error of 0.1, as the prospective primary hypothesis was that 14-day treatment with the highest doses of Mongersen would result in a higher proportion of patients in clinical remission than 14-day placebo treatment. An assumption was made that the group receiving the highest dose of Mongersen would have a rate of remission of 50% and that the placebo group would have a remission rate of 20%. It was estimated that 40 patients would be needed in each group in order to detect a significant difference in remission rates between each group at a power of 90%. All efficacy analyses were conducted according to the intention-to-treat principle on all randomized patients who received at least one dose of the assigned treatment. Patients with missing primary endpoint data at day 15 and/or day 28 were classified as failure. Remission and response rates were compared using the Pearson's chi-square test or Fisher's exact test. The proportions of patients with changes from baseline CRP levels between each of the three Mongersen groups and the placebo group were also described.

188 patients were screened for participation in the clinical trial and 166 were deemed eligible for enrollment (FIG. 1). Eligible patients were randomly assigned to receive Mongersen at a dose of either 10 mg/day (N=41), 40 mg/day (N=40) or 160 mg/day (N=43), or placebo (N=42). Demographic and clinical characteristics of the patients in each treatment group were evaluated and noted to be similar (Table 1).

**Table 1: Baseline Demographic and Clinical Characteristics**

| Characteristic | | | | |
|---|---|---|---|---|
| | Placebo (N=42) | Mongersen 10 mg (N=41) | Mongersen 40 mg (N=40) | Mongersen 160 mg (N=43) |
| Age - yr | 41 | 43 | 43 | 43 |
| mean | 19-74 | 20-71 | 19-69 | 22-70 |
| range | | | | |
| Sex - no. (%) | 23 (54.76) | 17 (41.46) | 21 (52.5) | 20 (46.51) |
| male | 19 (45.23) | 24 (58.53) | 19 (47.5) | 23 (53.48) |
| female | | | | |
| Body Mass Index | 23.24 | 22.23 | 23.63 | 23.60 |
| mean | 15.76-39.84 | 15.89-29.94 | 18.29-38.45 | 15.06-36.36 |
| range | | | | |
| Crohn's Disease | 264 (222-392) | 246 (221-399) | 240 (223-368) | 243 (221-396) |
| Activity | | | | |
| Index score median (range) | | | | |
| CRP-mg/L median (range) | 5.1(0-102) | 4.3(0-78) | 4.9(0-47) | 4.6(0-51) |
| Steroid-Dependent - no. (%) | 36 (85.7) | 32 (78.0) | 38 (95.0) | 36 (83.7) |
| Steroid-Resistant - no. (%) | 6 (14.3) | 9(22.0) | 2(5.0) | 7 (16.3) |
| Concomitant medication with immunomodulators - no. (%) | 12 (28.6) | 6 (14.6) | 7 (17.5) | 12 (27.9) |
| Smokers - no. (%) | 14 (33.3) | 18 (43.9) | 13 (32.5) | 17 (39.5) |
| Steroid use at baseline n (%) | 9(21.4) | 7 (17.1) | 13 (32.5) | 9 (20.9) |
| Previous history of CDrelated intestinal resection: - no. (%) | 14 (33.3) | 21 (51.2) | 15 (37.5) | 19 (44.2) |
| Duration of CD: yr- mean (SEM) | 10.9 (1.44) | 12.3 (1.58) | 7(1.3) | 9.3 (1.51) |

Of this group of 166 patients, 1 patient in the 160 mg/day Mongersen group, 1 patient in the 40 mg/day Mongersen group, 3 patients in the 10 mg/day Mongersen group, and 1 patient in the placebo group did not adhere to the drug administration protocol (FIG. 1). 160 patients out of 166 patients that entered the trial (96.38%) completed the 2-week treatment correctly, and 138 patients (83.1%; 39 patients in the 160 mg/day Mongersen group, 37 patients in the 40 mg/day Mongersen group, 32 patients in the 10 mg/day Mongersen group, and 30 patients in the placebo group) completed the entire protocol, including the 84-day follow-up period (FIG. 1). Some patients were withdrawn from the trial during the follow-up period due to AE, disease worsening, requests to be withdrawn by the patients, disappearance of the patient, or based on an investigator's decision.

No statistically significant differences in median steroid consumption were detected between the groups at baseline (Table 1). One patient receiving placebo and one patient receiving 10 mg/day Mongersen were taking 25 mg/day prednisone, and 1 patient receiving 40 mg/day Mongersen was taking 5 mg/day prednisone. Budesonide was taken by 8 patients receiving placebo (median dose 6 mg/day), six patients receiving 10 mg/day Mongersen (median dose 6 mg/day), 12 patients receiving 40 mg/day Mongersen (median dose 6 mg/day), and nine patients receiving 160 mg/day Mongersen (median dose 7.5 mg/day). At day 84, the percentage of patients who reached a steroid-free remission was significantly greater with 160 mg/day Mongersen (6/9, 67%) than with placebo (1/9, 11%, P=0.04), while there was no difference between 10 mg/day (3/7, 43%) or 40 mg/day groups (6/13, 46%) and placebo.

### Example 2: Treatment with an Anti-SMAD7 Therapy Induces Clinical Remission and Response in IBD Patients

In order to determine whether treatment with Mongersen resulted in remission in patients with CD, CDAI scores were evaluated after the two week treatment period ended. Patients with CDAI scores of less than 150 at both day 15 (1 day after stopping drug treatment) and day 28 (2 weeks after stopping drug treatment) were considered to have entered remission. In the placebo group, 4 out of 42 patients (9.5%) entered remission. By contrast, 22 out of 40 patients (55%) and 28 out of 43 patients (65.1%), respectively, in the 40 mg/day and 160 mg/day Mongersen groups entered remission. 5 out of 41 patients (12.2%) in the 10 mg/day Mongersen group entered remission (FIG. 2A). The remission rates in the 160 mg/day and 40 mg/day Mongersen groups were significantly higher than those in the 10 mg/day Mongersen (P<0.0001 for both groups) and placebo groups (P<0.0001 for both groups). There was no significant difference in remission rates between the 160 mg/day and 40 mg/day Mongersen groups or between the 10 mg/day Mongersen and placebo groups. These results demonstrated that administration of an anti-SMAD7 antisense therapy was effective to induce clinical remission in IBD patients.

In order to determine whether treatment with an anti-SMAD7 therapy was effective to cause a clinical response in patients with CD, patient CDAI scores were evaluated after the two week treatment period ended (*i.e.,* on day 15). Clinical response was defined as a 100-point decrease in CDAI score at day 15 compared to baseline (*i.e.,* prior to drug treatment). A clinical response according to this criteria was documented in 65.1% of patients in the 160 mg/day Mongersen group, 45% of patients in the 40 mg/day Mongersen group, 22% of patients in the 10 mg/day Mongersen group, and 26.2% of patients in the placebo group (FIG. 2B). The percentage of patients who experienced a 100-point CDAI decrease was significantly greater in the 160 mg/day and 40 mg/day Mongersen groups compared to the 10 mg/day Mongersen group (P<0.0001 and P= 0.027 respectively). The percentage of patients who experienced a 100-point clinical response was also significantly greater in the 160 mg/day Mongersen group, compared to the placebo group (P=0.0003).

Additionally, patient CDAI scores were evaluated on day 28 of the clinical trial (2 weeks after the end of treatment). Clinical response was defined in this case as a 100-point decrease in CDAI score at day 28 compared to baseline (*i.e.,* prior to drug treatment). A clinical response according to this criteria was documented in 72.1% of patients in the 160 mg/day Mongersen group, 57.5% of patients in the 40 mg/day Mongersen group, 36.6% of patients in the 10 mg/day Mongersen group, and 16.7% of patients in the placebo group (FIG. 2C). The percentage of patients who experienced a 100-point CDAI decrease was significantly greater in the 160 mg/day, 40 mg/day, and 10 mg/day Mongersen groups compared to the placebo group (FIG. 2C; P<0.0001 for the 160 mg/day group, P=0.0001 for the 40 mg/day group, and P=0.039 for the 10 mg/day group, compared to placebo for each group, respectively).

Similarly, patient CDAI scores were evaluated on day 84 of the clinical trial (10 weeks after the end of treatment). Clinical response was defined in this case as a 100-point decrease in CDAI score at day 84 compared to baseline (*i.e.,* prior to drug treatment). A clinical response according to this criteria was documented in 72.1% of patients in the 160 mg/day Mongersen group, 70.0% of patients in the 40 mg/day Mongersen group, 36.6% of patients in the 10 mg/day Mongersen group, and 26.2% of patients in the placebo group (FIG. 2D). The percentage of patients who experienced a 100-point CDAI decrease was significantly greater in the 160 mg/day and 40 mg/day Mongersen groups compared to the 10 mg/day Mongersen and placebo groups. These results demonstrated that both immediately after (*i.e.,* day 15) and two weeks after (*i.e.,* day 28) stopping drug treatment, treatment with an anti-SMAD7 therapy induced significant rates of clinical remission, defined as a CDAI decrease of at least 100 points compared to baseline, in IBD patients. These results demonstrated that both immediately after (*i.e.,* day 15) and two and ten weeks after (*i.e.,* day 28 and day 84) stopping drug treatment, treatment with an anti-SMAD7 therapy induced significant rates of clinical remission, defined as a CDAI decrease of at least 100 points compared to baseline, in IBD patients.

Clinical response defined as a decrease in CDAI score in patients of 70 points compared to baseline was also evaluated on days 15 and 28 of the clinical trial. On day 15 a 70-point CDAI decrease was documented in 81.4% of patients in the 160 mg/day Mongersen group, 72.5% of patients in the 40 mg/day Mongersen group, 34.1% of patients in the 10 mg/day Mongersen group, and 31% of patients in the placebo group (FIG. 3A). The percentage of patients who experienced a 70-point CDAI score decrease on day 15 was significantly greater in the 160 mg/day and 40 mg/day Mongersen groups compared to the 10 mg/day Mongersen (P<0.0001 and P= 0.0005, respectively) and placebo groups (P<0.0001 and P= 0.0002, respectively). On day 28, a 70-point CDAI decrease was documented in 76.7% of patients in the 160 mg/day Mongersen group, 80% of patients in the 40 mg/day Mongersen group, 48.8% of patients in the 10 mg/day Mongersen group, and 26.2% of patients in the placebo group (FIG. 3B). The percentage of patients who experienced a 70-point CDAI score decrease on day 28 was significantly greater in the 160 mg/day, 40 mg/day, and 10 mg/day Mongersen groups compared to placebo groups (P<0.0001, P<0.0001, and P= 0.033, respectively). These results demonstrated that both immediately after (*i.e.,* at day 15) and two weeks after (*i.e.,* day 28) stopping drug treatment, treatment with an anti-SMAD7 therapy induced significant rates of clinical remission, defined as a CDAI decrease of at least 70 points compared to baseline, in IBD patients.

CDAI scores were also analyzed in each group at day 15, day 28, and day 84 of the trial in order to determine whether treatment with the anti-SMAD7 therapy resulted in maintenance of CDAI scores of less than 150 over a longer period of time. On day 15, the percentage of patients with CDAI scores of less than 150 in the 160 mg/day, 40 mg/day, and 10 mg/day Mongersen groups and placebo groups were 67.4%, 57.5%, 14.6%, and 21.4%, respectively (FIG. 4A). On day 15, the percent of patients with CDAI scores lower than 150 was significantly higher in the 160 mg/day and 40 mg/day Mongersen groups compared to the 10 mg/day Mongersen group (P<0.0001 for both groups) and the placebo group (P<0.0001 and P=0.0008, respectively).

On day 28, the percentage of patients with CDAI scores of less than 150 in the 160 mg/day, 40 mg/day, and 10 mg/day Mongersen groups and placebo groups were 72.1%, 70%, 29.3%, and 14.3%, respectively (FIG. 4B). On day 28, the percent of patients with CDAI scores lower than 150 was significantly higher in the 160 mg/day and 40 mg/day Mongersen groups compared to the 10 mg/day Mongersen group (P<0.0001 and P=0.0002, respectively) and the placebo group (P<0.0001 for both groups).

On day 84, the percentage of patients with CDAI scores of less than 150 in the 160 mg/day, 40 mg/day, and 10 mg/day Mongersen groups and placebo groups were 67.4%, 62.5%, 26.8%, and 21.4%, respectively (FIG. 4C). On day 84, the percent of patients with CDAI scores lower than 150 was significantly higher in the 160 mg/day and 40 mg/day Mongersen groups compared to the 10 mg/day Mongersen group (P=0.0005 and P=0.003, respectively) and the placebo group (P<0.0001 for both groups). These results demonstrated that treatment of IBD patients with an anti-SMAD7 therapy was effective to induce a CDAI score of less than 150 in patients at least 70 days following the end of drug treatment.

Table 2 provides the median values and value range for CDAI scores of patients in each treatment group at baseline, day 15, day 28, and day 84 of the trial. As indicated in Table 2, median changes in CDAI scores in the 40 mg/day and 160 mg/day Mongersen-treated groups were significantly greater compared with those in the placebo group at each time point. No statistical difference was observed between the 10 mg/day Mongersen and placebo groups at any time point.

**Table 2. Crohn's Disease Activity Index (CDAI) Score at Baseline and Days 15, 28, and 84.**

| | Placebo | Mongersen 10 mg | Mongersen 40 mg | Mongersen 160 mg |
|---|---|---|---|---|
| CDAI score | | | | |
| Baseline | 264 (222 -392) | 246 (221 - 399) | 240 (223 - 368) | 243 (222 - 392) |
| Day 15 | 217 (66 - 421) | 201 (95 - 484) | 147 (20 - 316)† | 142 (32 - 271)† |
| Day 28 | 235 (71 - 421) | 181 (67 - 484) | 137 (0 - 436)† | 137 (31 - 348)† |
| Day 84 | 222 (37 - 431) | 187 (49 - 484) | 124 (16 - 436)† | 121 (18 - 306)† |

| | | | | |
|---|---|---|---|---|
| Data indicate median values (range). | | | | |

All P-values are for the change from baseline in each Mongersen group as compared with the placebo group. †P<0.01.

58.1% of patients receiving 160 mg/day Mongersen achieved remission at Day 15, which was maintained at both week 4 (28 days) and week 12 (84 days), compared to 9.5% of placebo patients (p<0001).

### Example 3: Treatment with an Anti-SMAD7 Therapy Induces Steroid-Free Remission in IBD Patients

The number of patients who achieved steroid-free clinical remission at the end of the trial was analyzed in order to determine if treatment with a SMAD7 antisense oligonucleotide therapy was able to facilitate steroid-free remission. At the beginning of the trial, 9 out of 42 (21.4%) of individuals in the placebo group, 7 out of 41 (17.1 %) of individuals in the 10 mg/day Mongersen group, 13 out of 40 (32.5%) of individuals in the 40 mg/day Mongersen group, and 9 out of 43 (20.9%) of individuals in the 160 mg/day Mongersen group were actively taking steroids. No significant differences in median steroid consumption existed between the groups at the beginning of the trial. On day 84 of the trial, a significantly greater portion of those individuals in the 160 mg/day group taking steroids at the start of the trial had achieved steroid-free remission as compared to the same group of individuals in the placebo group (P=0.04; 160 mg/day, 6/9 individuals (67%) achieved steroid-free remission vs. placebo, 1/9 individuals achieved steroid-free remission). Steroid-free remission was defined as clinical remission (defined above) with the equivalent of 0 mg prednisone ingestion. No significant differences were observed between the placebo group and the other Mongersen groups. These results demonstrate that treatment with a SMAD7 antisense oligonucleotide resulted in steroid-free remission in a cohort of individuals with IBD ingesting steroids prior to SMAD7 antisense oligonucleotide treatment.

### Example 4: Treatment with an Anti-SMAD7 Therapy Reduces Plasma Biomarker Expression

In order to determine if SMAD7 antisense oligonucleotide treatment resulted in changes in biomarkers associated with IBD, levels of IL8 and TNFα in patients were assessed at baseline, day 15, and day 28 of the trial by ELISA. As shown in Table 3, plasma concentrations of IL8 and TNFα were significantly reduced at days 15 and 28 compared to baseline in the 40 mg/day and 160 mg/day Mongersen groups (P<0.05 at days 15 and 28 compared to baseline in the 40 mg/day and 160 mg/day Mongersen groups). No significant difference was observed in the placebo or 10 mg/day Mongersen groups. These results demonstrate that treatment of patients with IBD with a SMAD7 antisense oligonucleotide for two weeks or less resulted in significantly reduced levels of IL8 and TNFα.

**Table 3. Cytokine Levels in Plasma Samples of Patients with Crohn's Disease Before and After Treatment with 10 mg/day, 40 mg/day and 160 mg/day Mongersen.**

| | Mongersen 10 | Mongersen 40 | Mongersen 160 |
|---|---|---|---|
| **IL8 (pg/mL)** | | | |
| Baseline | 28 (5.6) | 27 (7.2) | 30.6 (6.4) |
| Day 15 | 24 (9.2) | 17 (2.5)† | 17.9 (5.1)† |
| Day 28 | 23 (7.3) | 16 (3.2)t | 16.8 (5.6)† |
| **TNF (pg/mL)** | | | |
| Baseline | 15.4(8) | 19.2(7.3) | 22,4 (8.6) |
| Day 15 | 16 (5.4) | 11.4 (3.2)† | 14.2 (4.1)† |
| Day 28 | 14 (6.6) | 12.8 (2.6)† | 11.9 (3.3)† |

| | | | |
|---|---|---|---|
| Data indicate mean (SD). | | | |

† P<0.05 versus baseline.

Additionally, levels of CRP were evaluated in patients enrolled in the trial both prior to beginning the anti-SMAD7 therapy treatment and after treatment *(i.e.,* day 15) in order to determine if the anti-SMAD7 therapy was able to induce a change in circulating CRP levels. Screening of patients prior to beginning Mongersen treatment demonstrated that 102 out of 166 patients (61.4%) had elevated CRP levels *(i.e.,* greater than 3.0 mg/liter). Specifically, elevated CRP levels were observed in 25 out of 42 patients (59.5%) in the placebo group, 27 out of 41 patients (65.9%) in the 10 mg/day Mongersen group, 22 out of 40 patients (55%) in the 40 mg/day Mongersen group, and 28 out of 43 patients (65.1%) in the 160 mg/day Mongersen group (FIG. 5A). Evaluation of patients on day 15 demonstrated that the percentage of patients with normalized levels of CRP (*i.e.,* <3 mg/liter) was 4% in the placebo group, and 22%, 18.18%, and 17.9%, respectively, in the 10 mg/day, 40 mg/day and 160 mg/day Mongersen groups (FIG. 5B). Table 4 shows the median CRP levels and the range of CRP levels in patients with elevated CRP levels (>3 mg/L) at baseline in each treatment group. Among patients with elevated CRP levels at baseline, neither placebo nor Mongersen treatment significantly reduced the median values of CRP at days 15, 28, or 84 compared to baseline.

**Table 4. CRP Levels at Baseline and Days 15, 28, and 84 in Placebo and Mongersen-treated Patients with Elevated CRP Levels at Baseline**

| | Placebo (n = 25) | Mongersen 10 mg (n = 27) | Mongersen 40 mg (n = 22) | Mongersen 160 mg (n = 28) |
|---|---|---|---|---|
| C-reactive protein (mg/L) | | | | |
| Baseline | 10.6 (3.7-102) | 5.9 (3.02-78) | 8.02 (4.5-47.2) | 11.1 (3.17-51) |
| Day 15 | 20.6 (1.1-92.2) | 6.9 (0.4-104) | 6.2 (1.0-26.2) | 9.1 (1.4-52.3) |
| Day 28 | 17 (1.7-160.5) | 6.9 (1.0-101) | 5.6 (1.6-39.7) | 11.3 (0.9-65.1) |
| Day 84 | 8.5 (1.0-127.7) | 5.1 (1.4-44.6) | 4.8 (1.8-39) | 8.1 (1.5-54.3) |

| | | | | |
|---|---|---|---|---|
| Data indicate median values (range). | | | | |

These results indicate that treatment of IBD patients with an anti-SMAD7 therapy, even at low concentrations, resulted in an increase in the percentage of patients with normalized CRP levels compared to a placebo treatment.

Additionally, elevated baseline CRP levels and remission data from individual patients were analyzed to determine whether treatment with the anti-SMAD7 therapy resulted in remission in patients with initially elevated CRP levels (*i.e*., greater than 3.0 mg/liter). At day 15, the proportion of remitters (as defined by CDAI scores <150 and normalized CRP) among patients with elevated baseline CRP was 1/6 (16.7%), 0/4 (0%), 2/11 (18.2%), and 5/20 (25%) in the placebo, 10 mg/day, 40mg/day, and 160mg/day Mongersen groups, respectively. The analysis also showed that in patients with elevated CRP levels at baseline, the rates of remission at days 15 and 28 in the 160 mg/day and 40 mg/day Mongersen groups (67.9% and 45.5%, respectively) were significantly higher than the rates of remission for the same category of patients in the placebo group (12%; P<0.0001 and P=0.01, respectively; FIG. 6). No significant difference was observed between the 160 mg/day and 40 mg/day Mongersen groups or between the 10 mg/day Mongersen group (11.1%) and placebo group. These results demonstrated that anti-SMAD7 treatment of IBD patients with elevated CRP levels resulted in a significant increase in remission compared to treatment with placebo. These results further demonstrated that elevated CRP levels are a useful criterion for determining whether a patient will respond or is likely to respond to treatment with an anti-SMAD7 therapy.

CCL20 serum levels from patients enrolled in the trial were also analyzed to investigate the relationship between CCL20 and clinical response and remission following Mongersen treatment. CCL20 was measured using a commercial ELISA kit (R&D Catalog Number DM3A00). Serum levels of CCL20 were measured in patients in the 40 mg/day and 160 mg/day Mongersen groups who underwent clinical remission/response following treatment with Mongersen. Serum levels of CCL20 were measured at both day 0 and day 84 (FIG. 8A). Treatment with Mongersen both in individual patients (FIG. 8A, left panel) and, on average (FIG. 8A, right panel), resulted in decreased serum levels of CCL20 in patients who experienced clinical remission/response.

CCL20 levels were also measured in those patients who did not experience clinical remission/response following treatment with 40 mg/day or 160 mg/day Mongersen. Among these patients, no decrease in serum CCL20 levels was observed at day 84 with respect to baseline either on an individual basis (FIG. 8B, left panel) or on average (FIG. 8B, right panel).

Within the group of patients experiencing clinical remission/response following treatment with 40 mg/day or 160 mg/day Mongersen, CCL20 levels were analyzed in the subset of patients with baseline serum levels of CCL20 greater than 10 pg/ml. Treatment with Mongersen in individual patients (FIG. 8C, left panel) resulted in a decrease in serum levels of CCL20 at day 84 compared to serum CCL20 levels measured at day 0. Treatment also resulted in a significant decrease in average serum levels of CCL20 at day 84 compared to serum CCL20 levels measured at day 0 (FIG. 8C, right panel; p=0.035).

These results demonstrated that treatment with an anti-SMAD7 therapeutic can result in both clinical remission/response and decreased CCL20 serum levels in patients with IBD. They also demonstrated that decreased CCL20 levels are correlated with patient clinical remission/response.

Serum CCL20 levels were also measured in a larger group of patients receiving either 10 mg/day, 40 mg/day, or 160 mg/day Mongersen or placebo to further investigate the relationship between CCL20 levels and clinical response and remission following treatment with Mongersen. Serum CCL20 levels were measured in a group of 64 patients either at baseline (day 0), 2 weeks (day 15), 4 weeks (day 28), or 12 weeks (day 84) after commencing drug treatment. Following drug treatment, 25 patients from this group were in clinical remission, 14 patients exhibited a clinical response, and 13 patients did not respond to drug treatment. After placebo treatment, 2 patients were in clinical remission, 4 patients exhibited a clinical response, and 6 patients did not respond to placebo treatment.

Median baseline serum levels of CCL20 were calculated for patients that exhibited clinical remission or response ("responders") and patients that did not exhibit clinical remission or response ("non-responders") following treatment with either Mongersen or placebo. The median serum CCL20 level at baseline in responders treated with Mongersen was 13 pg/ml, with a range of 2-55 pg/ml, while the median serum CCL20 level in non-responders treated with Mongersen was 15 pg/ml, with a range of 5-27 pg/ml (data not shown). Among placebo-treated patients, median serum CCL20 levels at baseline were 15 pg/ml in responders, with a range of 13-25 pg/ml, and 9 pg/ml in non-responders, with a range of 10-50 pg/ml.

Within this group of 64 patients, CCL20 serum levels were evaluated at all time points in those patients that displayed baseline serum levels of CCL20 of 15 pg/ml or more. Non-parametric methods were used for statistical analysis. The Mann-Whitney U test for two independent samples was used to compare various patient groups. Among Mongersen-treated patients, 15 responders and 7 non-responders had baseline serum levels of CCL20 of at least 15 pg/ml. Among placebo-treated patients, 4 responders and 4 non-responders had baseline serum levels of CCL20 of at least 15 pg/ml. Among this subset of Mongersen-treated responders, serum CCL20 levels decreased significantly at all time points compared to baseline (FIG. 9A; day 0: median=31 pg/ml, range=15-55 pg/ml; day 15: median=17 pg/ml, range=6-32 pg/ml; day 28: median=17 pg/ml, range=6-36 pg/ml; and day 84: median=14 pg/ml, range=7-90 pg/ml; day 0 v. day 15, p=0.0028; day 0 v. day 28, p=0.0025; day 0 v. day 84, p=0.007). In contrast, no significant decrease was observed in serum CCL20 levels from baseline among Mongersen-treated non-responders (FIG. 9B; day 0: median=19 pg/ml, range=15-27 pg/ml; day 15: median=26 pg/ml, range=14-149 pg/ml; day 28: median=16 pg/ml, range=12-30 pg/ml; and day 84: median=17 pg/ml, range=12-31 pg/ml), placebo-treated responders (FIG. 9C; day 0: median=16 pg/ml, range=15-25 pg/ml; day 15: median=22 pg/ml, range=14-33 pg/ml; day 28: median=21 pg/ml, range=12-27 pg/ml; and day 84: median=16 pg/ml, range=10-23 pg/ml), or placebo-treated non-responders (FIG. 9D; day 0: median=35 pg/ml, range=27-134 pg/ml; day 15: median=39 pg/ml, range=17-95 pg/ml; day 28: median=25 pg/ml, range=7-33 pg/ml; and day 84: median=40 pg/ml, range=16-80 pg/ml). These results demonstrated that a significant decrease in CCL20 serum levels following SMAD7 antisense oligonucleotide treatment is correlated with clinical remission and responsiveness, and that no such correlation was found in non-responsive patients treated with the SMAD7 antisense oligonucleotide or in placebo-treated responders and non-responders.

Additionally, CCL20 serum levels were separately evaluated among those patients that displayed baseline serum levels of CCL20 of 15 pg/ml or more in each of the group of patients that displayed clinical remission and the group of patients that displayed clinical response following Mongersen treatment. Serum CCL20 levels were measured in patients receiving either 10 mg/day, 40 mg/day, or 160 mg/day Mongersen or placebo either at baseline (day 0), 2 weeks (day 15), 4 weeks (day 28), or 12 weeks (day 84) after commencing drug treatment. Treatment with Mongersen resulted in a significant decrease in CCL20 levels at day 15 and day 84 compared to baseline among patients who experienced clinical remission (FIG. 10, left panel; p=0.02, day 0 vs, day 15; p=0.1, day 0 vs, day 28; and p=0.001, day 0 vs, day 84). Median CCL20 levels also declined in patients who experienced clinical response following Mongersen treatment (FIG. 10, right panel; p=0.3, day 0 vs, day 15; p=0.1, day 0 vs, day 28; and p=0.06, day 0 vs, day 84). Horizontal bars in Figure 10 represent median values for each time point, and each point represents CCL20 serum values in a single patient. These results demonstrated that a significant decrease in CCL20 serum levels following SMAD7 antisense oligonucleotide treatment is correlated with clinical remission, and that a decrease in median CCL20 serum levels is observed in patients experiencing clinical responsiveness following SMAD7 antisense oligonucleotide treatment.

### Example 5: Treatment with an Anti-SMAD7 Therapy Is Tolerated in Patients with IBD

In order to determine whether treatment with Mongersen was associated with safety issues or AEs, instances of such issues and events were tracked throughout the trial. Nine serious adverse events (SAE) were registered in 6 patients. 2 SAE were documented in 1 patient in the placebo group, 4 SAE were documented in 3 patients in the 10 mg/day Mongersen group, 2 SAE were documented in 1 patient in the 40 mg/day Mongersen group, and 1 SAE was documented in 1 patient in the 160 mg/day Mongersen group (Table 5). Two SAE were not considered by physicians to be related to treatment. Most SAE consisted of hospital admissions for complications or symptoms associated with CD. One patient in the group given 160 mg of Mongersen experienced a thermal burn of his legs while cooking. No patients died during the clinical trial.

**Table 5. Serious adverse events registered during the study.**

| | Placebo (n=42) | Mongersen 10 mg (n=41) | Mongersen 40 mg (n=40) | Mongersen 160 mg (n=43) |
|---|---|---|---|---|
| Total pts with at least one SAE | 1 (2.4) | 3 (7.3) | 1 (2.5) | 1 (2.3) |
| Total number of SAEs | 2 | 4 | 2 | 1 |
| Abdominal pain | - | 2 (4.9) | - | - |
| Seton placement for perianal fistula | - | - | 1 (2.5) | - |
| Crohn's disease worsening | - | 1 (2.4) | - | - |
| Surgery for hemorrhoid thrombosis | - | - | 1 (2.5) | - |
| Pyrexia | 1 (2.4) | 1 (2.4) | - | - |
| Thermal burn | - | - | - | 1 (2.3) |
| Cough | 1 (2.4) | - | - | - |

| | | | | |
|---|---|---|---|---|
| Data indicate number (%) of patients (pts) with at least one serious adverse event (SAE) and total number of SAEs. | | | | |

Differences between groups were not statistically significant.

28 patients in the placebo group and 20, 25, and 21 patients, respectively, in the 10 mg/day, 40 mg/day and 160 mg/day Mongersen groups experienced an AE during or after treatment (Table 6). Overall, 200 AE were document during the clinical trial. These included 64 AE in the placebo group, 39 AE in the 10 mg/day Mongersen group, 50 in the 40 mg/day Mongersen group, and 47 in the 160 mg/day Mongersen group. The majority of AE were mild and considered not related to treatment by the investigators. No change in vital signs was noted in any patient during the study. No increase in serum complement factors was observed in patients during the trial. These results demonstrate that, overall, treatment of IBD with the anti-SMAD7 therapy is not associated with AE and is well-tolerated by patients.

**Table 6. Adverse Events Reported by >5% of Patients in a Single Arm.**

| | Placebo (n=42) | Mongersen 10 mg (n=41) | Mongersen 40 mg (n=40) | Mongersen 160 mg (n=43) |
|---|---|---|---|---|
| Total pts with at least one AE (%) | 28 (66.7) | 20 (48.8) | 25 (62.5) | 21 (48.8) |
| Total number of AEs | 64 | 39 | 50 | 47 |
| Abdominal pain | 6 (14.28) | 4(9.8) | 4 (10.0) | 5 (11.6) |
| Crohn's disease worsening | 14 (33.3) | 6 (14.6) | 4 (10.0) | 5 (11.6) |
| C-reactive protein increased | 4 (9.5) | 2(4.9) | 2 (5.0) | 4 (9.3) |
| Pyrexia | 4 (9.5) | 3 (7.3) | 2 (5.0) | 2(4.7) |
| Abdominal mass | 2 (4.8) | 1 (2.4) | 3 (7.5) | 3 (7.0) |
| Diarrhoea | 1 (2.4) | - | 2 (5.0) | 3 (7.0) |
| Arthralgia | 1 (2.4) | 2 (4.9) | 2 (5.0) | 1 (2.3) |
| Urinary tract infection | 1 (2.4) | 6 (14.6) | 2 (5.0) | 2(4.7) |
| Asthenia | 1 (2.4) | - | 2 (5.0) | 1 (2.3) |
| Flu-like illness | 3 (7.1) | - | 1 (2.5) | 3 (6.97) |
| Headache | 3 (7.1) | - | - | 1 (2.3) |
| Transaminases increased | - | - | 2 (5.0) | - |

| | | | | |
|---|---|---|---|---|
| Data indicate number (%) of patients with at least one adverse event (AE) and total number of AEs. | | | | |

### Example 6: Clinical remission correlated with baseline CRP levels

The relationship between baseline CRP levels and clinical remission at day 15 and week 4 was studied, as shown in FIG. 7. Patients with CRP levels lower than 3 mg/L showed increased rates of remission after 15 days when receiving 40 mg/day or 160 mg/day Mongersen (66.7% and 60.0%, respectively), as compared to those receiving placebo or 10 mg/day Mongersen (17.6% and 14.3%, respectively). The rates of remission for patients with CRP levels of 3 mg/L or greater who received 40 mg/day or 160 mg/day Mongersen (50.0% and 71.4%, respectively) were greater than those receiving placebo or 10 mg/day Mongersen (24.0% and 14.8%, respectively).

After four weeks, patients with CRP levels lower than 3 mg/L and receiving placebo or 10, 40, or 160 mg/day Mongersen showed remission rates of 17.6%, 35.7%, 72.2%, and 66.7%, respectively. Patients with CRP levels of 3 mg/L or greater and receiving placebo or 10, 40, or 160 mg/day Mongersen showed remission rates of 12.0%, 25.9%, 68.2%, and 75.0%, respectively.

### Example 7: TGFβ Reduces TNFα-Mediated CCL20 Induction

To evaluate the effect of various factors on CCL20 expression in colonic epithelial cells, human normal colonic epithelial cells from the NCM 460 cell line were either left unstimulated (Unst), exposed to TGFβ, TNFα, Interferon γ (IFNγ), or Interleukin-17A (IL17A) individually, or exposed to a combination of TGFβ and TNFα, TGFβ and IFNγ, or TGFβ and IL17A (FIG. 11). CCL20 (hCCL20) mRNA expression was measured in each group. While TNFα exposure resulted in a large increase in CCL20 expression relative to the unstimulated control, exposure to a combination of TGFβ and TNFα resulted in much lower expression of CCL20. This result indicates that TGFβ exposure inhibited the ability of TNFα to induce CCL20 mRNA expression.

### Example 8: SMAD7 and CCL20 Expression is Correlated in Gastrointestinal Tissue of Crohn's Disease Patients

To evaluate whether SMAD7 and CCL20 are co-expressed in gastrointestinal tissue of Crohn's disease patients, CCL20 and SMAD7 expression were analyzed by immunohistochemistry in ileal and colonic tissue sections from patients with Crohn's disease. Samples were embedded in cryostat mounting medium, snap-frozen, and sectioned. Tissue sections mounted on slides were then fixed and washed. Tissue sections were incubated with a rabbit polyclonal antibody directed against human CCL20 (final dilution 1:100; Abcam, Cambridge, UK) and a mouse monoclonal antibody against human SMAD7 (final dilution 1:50; R&D Systems, Minneapolis, MN) at room temperature for 30 minutes and 1 hour, respectively, followed by a biotin-free horse radish peroxidase polymer detection technology (Ultravision Detection System, Cat. No. TL-060-HL, Thermo Scientific, Waltham, MA) with 3,3'diaminobenzidine (Dako, Carpinteria, CA) as a chromogen. Sections were counterstained with haematoxylin and dehydrated. Isotype control IgG-stained sections were prepared under identical immunohistochemical conditions, using mouse and rabbit normal IgG control antibodies (R&D Systems, Minneapolis, MN) instead of SMAD7 or CCL20 primary antibodies. Stained sections were analyzed by light microscopy. Isotype control IgG-stained sections revealed no significant background signal (FIG. 12A, right panels). Staining of tissue from Crohn's disease patients demonstrated that both SMAD7 and CCL20 were expressed in epithelial cells of the ileum and colon (FIG. 12A, left and middle panels).

In order to determine whether SMAD7 expression affects CCL20 protein expression, CCL20 protein levels were analyzed in mucosal colonic explants from Crohn's disease patients exposed to either SMAD7 sense or antisense oligonucleotides. Inflamed colonic tissue from 6 patients undergoing surgical resection for active Crohn's disease was harvested, cut into pieces of 3-4mm in length, and placed on sterile filters (EMD Millipore, Milan, Italy) immersed in AQIX medium (Liquid life, London, United Kingdom) in an organ culture chamber (37°C, 5% CO₂/95% O₂ environment). SMAD7 antisense or control sense oligonucleotides (10 µg/ml) were added to the explants for 36 hours, and samples were collected for CCL20 protein analysis. CCL20 levels were measured in sera and culture supernatants by ELISA (R&D Systems, Minneapolis, MN). Absorbance signal was measured with the spectrophotometer Multimode detector DTX 880 (Beckman Coulter, Milan, Italy). A comparison of CCL20 protein levels in explants from individual patients treated with either SMAD7 antisense oligonucleotide (Smad7 As) or SMAD7 sense oligonucleotide (Sense) demonstrated that CCL20 levels were significantly reduced following Smad7 As treatment compared to Sense treatment (FIG. 12B, p=0.03). In Figure 12, each point represents CCL20 protein levels in a single explant, and horizontal bars indicate median CCL20 protein values across all explants treated with Sense or Smad7 AS oligonucleotides. Each bar connecting individual data points in the Sense and Smad7 As columns connects data points for explants obtained from the same patient. These results demonstrated that CCL20 levels in colonic tissue from Crohn's disease patients are decreased following treatment with a SMAD7 antisense oligonucleotide that decreases SMAD7 protein expression.

### SEQUENCES

SEQ ID NO: 1 (Coding Sequence CDS (288-1568) of NM_005904.3; Homo sapiens SMAD family member 7 (SMAD7), transcript variant 1, mRNA) - Target sequence of Mongersen target and its derivatives underlined (108-128); 
ATG TTCAGGACCA AACGATCTGC GCTCGTCCGG CGTCTCTGGA GGAGCCGTGC
GCCCGGCGGC GAGGACGAGG AGGAGGGCGC AGGGGGAGGT GGAGGAGGAG GCGAGCTGCG
GGGAGAAGGG GCGACGGACA GCCGAGCGCA TGGGGCCGGT GGCGGCGGCC CGGGCAGGGC
TGGATGCTGC CTGGGCAAGG CGGTGCGAGG TGCCAAAGGT CACCACCATC CCCACCCGCC
AGCCGCGGGC GCCGGCGCGG CCGGGGGCGC CGAGGCGGAT CTGAAGGCGC TCACGCACTC
GGTGCTCAAG AAACTGAAGG AGCGGCAGCT GGAGCTGCTG CTCCAGGCCG TGGAGTCCCG
CGGCGGGACG CGCACCGCGT GCCTCCTGCT GCCCGGCCGC CTGGACTGCA GGCTGGGCCC
GGGGGCGCCC GCCGGCGCGC AGCCTGCGCA GCCGCCCTCG TCCTACTCGC TCCCCCTCCT
GCTGTGCAAA GTGTTCAGGT GGCCGGATCT CAGGCATTCC TCGGAAGTCA AGAGGCTGTG
TTGCTGTGAA TCTTACGGGA AGATCAACCC CGAGCTGGTG TGCTGCAACC CCCATCACCT
TAGCCGACTC TGCGAACTAG AGTCTCCCCC CCCTCCTTAC TCCAGATACC CGATGGATTT
TCTCAAACCA ACTGCAGACT GTCCAGATGC TGTGCCTTCC TCCGCTGAAA CAGGGGGAAC
 
GAATTATCTG GCCCCTGGGG GGCTTTCAGA TTCCCAACTT CTTCTGGAGC CTGGGGATCG
GTCACACTGG TGCGTGGTGG CATACTGGGA GGAGAAGACG AGAGTGGGGA GGCTCTACTG
TGTCCAGGAG CCCTCTCTGG ATATCTTCTA TGATCTACCT CAGGGGAATG GCTTTTGCCT
CGGACAGCTC AATTCGGACA ACAAGAGTCA GCTGGTGCAG AAGGTGCGGA GCAAAATCGG
CTGCGGCATC CAGCTGACGC GGGAGGTGGA TGGTGTGTGG GTGTACAACC GCAGCAGTTA
CCCCATCTTC ATCAAGTCCG CCACACTGGA CAACCCGGAC TCCAGGACGC TGTTGGTACA
CAAGGTGTTC CCCGGTTTCT CCATCAAGGC TTTCGACTAC GAGAAGGCGT ACAGCCTGCA
GCGGCCCAAT GACCACGAGT TTATGCAGCA GCCGTGGACG GGCTTTACCG TGCAGATCAG
CTTTGTGAAG GGCTGGGGCC AGTGCTACAC CCGCCAGTTC ATCAGCAGCT GCCCGTGCTG
GCTAGAGGTC ATCTTCAACA GCCGGTAG
SEQ ID NO: 2
   5'-GTCGCCCCTTCTCCCCGCAGC-3'
SEQ ID NO: 3
   5'-GTXGCCCCTTCTCCCXGCAG-3'
   X is 5-methyl 2'-deoxycytidine
SEQ ID NO: 4
   5'-GTXGCCCCTTCTCCCXGCAGC-3'
   X is 5-methyl-2'-deoxycytidine
SEQ ID NO: 5
   5'-GTXYCCCCTTCTCCCXYCAG-3'
X is a nucleotide comprising a nitrogenous base selected from the group consisting of cytosine and 5-methylcytosine or a 2'-O-methylcytosine nucleoside, and wherein Y is a nucleotide comprising a nitrogenous base selected from the group consisting of guanine and 5-methylguanine or a 2'-O-methylguanine nucleoside, provided that at least one of the nucleotides X or Y comprises a methylated nitrogenous base

### INCORPORATION BY REFERENCE

The entire disclosure of each of the patent documents and scientific articles cited herein is incorporated by reference for all purposes.

### EQUIVALENTS

The invention can be embodied in other specific forms with departing from the essential characteristics thereof. The foregoing embodiments therefore are to be considered illustrative rather than limiting on the invention described herein. The scope of the invention is indicated by the appended claims rather than by the foregoing description, and all changes that come within the meaning and range of equivalency of the claims are intended to be embraced therein.

Aspects and features of the present disclosure are set out in the following numbered clauses which contain the subject matter of the claims of the parent application as filed.
1. A method for treating or managing inflammatory bowel disease (IBD) in a patient having IBD, wherein the method comprises (a) administering to the patient an initial dose of a SMAD7 antisense-oligonucleotide; (b) analyzing the level of Chemokine (C-C motif) ligand 20 (CCL20) in the patient; and (c) if the level of CCL20 is above normal levels of CCL20, then administering to the patient a subsequent dose that is greater than or equal to the initial dose, or, if the level of CCL20 is below normal levels of CCL20, then administering to the patient a subsequent dose that is equal to or smaller than the initial dose.
2. A method for treating or managing inflammatory bowel disease (IBD) in a patient having IBD, wherein the method comprises (a) analyzing the level of CCL20 in the patient; and (b) if the level of CCL20 is above normal levels of CCL20, then administering to the patient an initial dose of a SMAD7 antisense-oligonucleotide.
3. The method of clause 2, wherein the method further comprises: (c) analyzing the level of CCL20 in the patient after said administering step; and (d) if the level of CCL20 is above normal levels of CCL20 then administering to the patient a subsequent dose that is greater than or equal to the initial dose, or, if the level of CCL20 is below normal levels of CCL20 then administering to the patient a subsequent dose that is equal to or smaller than the initial dose.
4. The method of clause 2, wherein the method further comprises: (c) analyzing the level of CCL20 in the patient after said administering step; and (d) if the level of CCL20 is lower after said administration step than the level of CCL20 before said administration step, then administering to the patient a subsequent dose that is the same as the initial dose or smaller than the initial dose, or, if the level of CCL20 is unchanged or increased after said administration step compared to the level of CCL20 before said administration step, then administering to the patient a subsequent dose that is the same as the initial dose or greater than the initial dose or terminating the treatment.
5. A method for treating or managing IBD in a patient having IBD, wherein the method comprises: (a) establishing a control level of CCL20 for the patient; (b) administering to the patient an initial dose of a SMAD7 antisense-oligonucleotide; (c) analyzing the level of CCL20 in the patient; and (d) if the level of CCL20 is lower than the control level, then administering to the patient a subsequent dose that is the same as the initial dose or smaller than the initial dose, or, if the level of CCL20 is unchanged or increased compared to the control level, then administering to the patient a subsequent dose that is the same as the initial dose or greater than the initial dose or terminating the treatment.
6. The method of clause 4 or 5, wherein if the patient is in clinical remission and the level of CCL20 is unchanged or increased after said administration step compared to the level of CCL20 before said administration step, then terminating the treatment.
7. The method of clause 4 or 5, wherein, if the level of CCL20 is at least 10%, at least 20%, at least 30%, at least 40%, at least 50%, or at least 70% decreased after said administration step compared to the level of CCL20 before said administration step, then administering to the patient a subsequent dose that is the same as the initial dose or smaller than the initial dose.
8. The method of clause 4 or 5, further comprising determining that the patient having IBD has a greater than 20%, greater than 30%, greater than 40%, greater than 50%, greater than 60%, greater than 70%, greater than 80%, greater than 90% or greater than 100% chance of experiencing clinical remission of the IBD for a time period of at least 1 week, at least 2 weeks, at least 3 weeks, at least 4 weeks, at least 6 weeks or at least 8 weeks, if the level of CCL20 after said administering step is at least 10%, at least 20%, at least 30%, at least 40%, at least 50%, at least 60%, or at least 70% decreased compared to the level of CCL20 before said administration step.
9. The method of clause 8, wherein the clinical remission in the patient having IBD is indicated by a Crohn's Disease Activity Index (CDAI) <150.
10. The method of clause 8, wherein the clinical remission is observed about one week, about two weeks, or about three weeks after said administration step and maintained for a period of at least 1 week, at least 2 weeks, at least 3 weeks, at least 4 weeks, at least 6 weeks or at least 8 weeks.
11. The method of clause 8, wherein the patient having IBD had a CDAI of between about 220 and about 400 one week prior to said administration step.
12. The method of clause 1 or clause 3, wherein if the level of CCL20 is above normal levels of CCL20, then administering to the patient a subsequent dose that is greater than the initial dose, or, if the level of CCL20 is below normal levels of CCL20 then administering to the patient a subsequent dose that is smaller than the initial dose.
13. The method of clause 3 or 5, wherein, if the subsequent dose is equal to or greater than the maximum tolerated dose (MTD), then terminating the treatment.
14. The method of clause 3 or 5, wherein the level of CCL20 is analyzed at least 1 day, at least 3 days, at least 5 days, at least 1 week, at least 2 weeks, at least 3 weeks, at least 1 month, at least 2 months, at least 4 months, or at least 6 months after said administration step.
15. The method of clause 3 or 5, wherein the level of CCL20 is analyzed immediately after said administration step.
16. The method of clause 3 or 5, wherein the level of CCL20 is analyzed about 15 days or about 28 days after said administration step.
17. The methods of clause 1 or clause 2, wherein the normal levels of CCL20 are median levels of CCL20 in a healthy control group.
18. The methods of clause 5, wherein the control level of CCL20 is a median level of CCL20 in a healthy control group.
19. The method of clause 17 or 18, wherein the healthy control group and the patient having IBD are matched with respect to age, gender, ethnic origin, smoking habits, dietary habits, body-mass index (BMI), and/or exercise habits.
20. The method of clause 1 or clause 2, wherein normal levels of CCL20 are about 6 pg/ml, about 7 pg/ml, about 8 pg/ml, about 9 pg/ml, about 10 pg/ml, about 11 pg/ml, or about 12 pg/ml.
21. The method of clause 5, wherein control level of CCL20 is about 6 pg/ml, about 7 pg/ml, about 8 pg/ml, about 9 pg/ml, about 10 pg/ml, about 11 pg/ml, or about 12 pg/ml.
22. The method of clause 1, 2, or 5, wherein the initial dose is less than 100 mg/day, less than 90 mg/day, less than 80 mg/day, less than 70 mg/day, less than 60 mg/day, less than 50 mg/day, less than 40 mg/day or less than 30 mg/day.
23. The method of clause 1, 2, or 5, wherein the initial dose is at least 10 mg/day, at least 20 mg/day, at least 30 mg/day, at least 40 mg/day, at least 50 mg/day, at least 60 mg/day, at least 70 mg/day, at least 80 mg/day, or at least 90 mg/day.
24. The method of clause 1, 2, or 5, wherein the initial dose is about 10 mg/day, about 20 mg/day, about 30 mg/day, about 40 mg/day, about 50 mg/day, about 60 mg/day, about 70 mg/day, about 80 mg/day, about 90 mg/day, or about 100 mg/day.
25. The method of clause 1, 2, or 5, wherein the initial dose is 10 mg/day, 40 mg/day, 80 mg/day, or 160 mg/day.
26. The method of clause 1 or 3, wherein, if CCL20 levels are above normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
27. The method of clause 5, wherein, if CCL20 levels are above a control level, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
28. The method of clause 1 or 3, wherein, if CCL20 levels are below normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
29. The method of clause 5, wherein, if CCL20 levels are below a control level, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
30. The method of clause 1, 3, or 5, wherein the initial dose is between about 10 mg/day and 100 mg/day and the subsequent dose is between about 30 mg/day and 200 mg/day.
31. The method of clause 1, 2, or 5, wherein the level of CCL20 in the patient having IBD is determined in a sample obtained from the patient having IBD.
32. The method of clause 31, wherein the sample is a blood, serum or plasma sample.
33. The method of clause 1, 2, or 5, wherein the level of CCL20 is determined by immunochemistry or by nucleotide analysis.
34. The method of clause 33, wherein the level of CCL20 is determined by an enzyme-linked immunosorbent assay (ELISA).
35. The method of clause 1, 2, or 5, further comprising determining a level of one or more additional analytes in the patient having IBD.
36. The method of clause 35, wherein the one or more additional analytes comprise Interleukin-8 (IL8), Tumor Necrosis Factor α(TNFα), or C-reactive protein (CRP).
37. The method of clause 1, 2, or 5, wherein the IBD is Crohn's Disease (CD) or ulcerative colitis (UC).
38. The method of clause 37, wherein the patient having IBD is a steroid-dependent patient with active CD.
39. The method of clause 37, wherein the patient having IBD is a steroid-resistant patient with active CD.
40. The method of clause 1, 2, or 5, wherein the SMAD7 antisense-oligonucleotide is administered orally to the patient having IBD.
41. The method of clause 1, 2, or 5, wherein the SMAD7 antisense oligonucleotide targets region 108-128 of human SMAD7 (SEQ ID NO: 1).
42. The method of clause 1, 2, or 5, wherein the SMAD7 antisense oligonucleotide targets nucleotides 403, 233, 294, 295, 296, 298, 299 or 533 of human SMAD7 (SEQ ID NO: 1).
43. The method of clause 1, 2, or 5, wherein the SMAD7 antisense oligonucleotide comprises the nucleotide sequence of SEQ ID NO: 2 (5'-GTCGCCCCTTCTCCCCGCAGC-3').
44. The method of clause 1, 2, or 5, wherein the antisense oligonucleotide is an antisense oligonucleotide phosphorothioate against SMAD7 comprising the following sequence: 5'-GTXGCCCCTTCTCCCXGCAG-3' (SEQ ID NO: 3) wherein X is a nucleotide comprising 5-methyl-2'-deoxycytidine and wherein the internucleotide linkages are phosphorothioate linkages.
45. The method of clause 44, wherein the antisense oligonucleotide is an antisense oligonucleotide phosphorothioate against SMAD7 comprising the following sequence: 5'-GTXGCCCCTTCTCCCXGCAGC-3' (SEQ ID NO: 4) wherein X is a nucleotide comprising 5-methyl-2'-deoxycytidine and wherein the internucleotide linkages are phosphorothioate linkages.
46. A method for treating or managing IBD in a patient with IBD having above normal CCL20 levels following administration of a dose of a SMAD7 antisense oligonucleotide, said method comprising administering to said patient a further dose of said oligonucleotide that is greater than or equal to the prior dose.
47. A method for treating or managing IBD in a patient with IBD having below normal CCL20 levels following administration of a dose of SMAD7 antisense oligonucleotide, said method comprising administering to said patient a further dose of said oligonucleotide that is less than or equal to the prior dose.
48. A method of treating or managing IBD in a patient with IBD having above normal CCL20 levels, said method comprising administering to said patient a dose of a SMAD7 antisense oligonucleotide.
49. The method of clause 48, wherein the administering is repeated until any of CCL20 levels, IL8 levels, CRP levels, and/or TNFα levels reach a normal level.
50. The method of clause 48, wherein the administering is repeated until the patient achieves a CDAI score of less than 150.
51. The method of clause 48, wherein the administering is repeated until the patient achieves clinical remission.
52. A method of monitoring the treatment or management of IBD in a patient with IBD, the method comprising analyzing CCL20 levels in the patient following each SMAD7 antisense oligonucleotide administration, wherein the absence of a decrease in CCL20 levels indicates that the treatment or management is not effective.
53. The method of clause 52, wherein CCL20 levels are analyzed one time, two times, three times, four times, about five times, about 10 times, about 15 times, about 20 times, or about 30 times after each administration of SMAD7 antisense oligonucleotide.
54. The method of clause 52, wherein the CCL20 levels are analyzed immediately after, about 1 hour after, about 3 hours after, about 6 hours after, about 12 hours after, about 1 day after, about 3 days after, about 1 week after, about 2 weeks after, and/or about 1 month after SMAD7 antisense oligonucleotide administration.
55. A method of treating or managing IBD in a patient with IBD having above normal levels of CCL20, comprising increasing the amount of a SMAD7 antisense oligonucleotide administered to the patient until CCL20 levels in the patient decrease.
56. The method of clause 55, wherein CCL20 decreases to about a normal level of CCL20 or a below normal level of CCL20.
57. A SMAD7 antisense-oligonucleotide for use in a method for treating or managing IBD in a patient having IBD, wherein the method comprises analyzing the level of CCL20 in the patient to determine appropriate levels of SMAD7 antisense oligonucleotide administration.
58. The SMAD7 antisense-oligonucleotide for use of clause 57, wherein the method comprises the steps of: (a) administering to the patient an initial dose of the SMAD7 antisense-oligonucleotide; (b) analyzing the level of CCL20 in the patient; and (c) if the level of CCL20 is above normal levels of CCL20, then administering to the patient a subsequent dose of the SMAD7 antisense-oligonucleotide that is greater than or equal to the initial dose, or, if the level of CCL20 is below normal levels of CCL20 then administering to the patient a subsequent dose of the SMAD7 antisense-oligonucleotide that is equal to or smaller than the initial dose.
59. A SMAD7 antisense-oligonucleotide for use in a method for treating or managing IBD in a patient having IBD, wherein the method comprises (a) analyzing the level of CCL20 in the patient; and (b) if the level of CCL20 is above normal levels of CCL20, then administering to the patient an initial dose of the SMAD7 antisense-oligonucleotide.
60. A method for treating or managing inflammatory bowel disease (IBD) in a patient having IBD, wherein the method comprises (a) administering to the patient an initial dose of a SMAD7 antisense-oligonucleotide; (b) analyzing the level of interleukin 8 (IL8) in the patient; and (c) if the level of IL8 is above normal levels of IL8, then administering to the patient a subsequent dose that is greater than or equal to the initial dose, or, if the level of IL8 is below normal levels of IL8, then administering to the patient a subsequent dose that is equal to or smaller than the initial dose.
61. A method for treating or managing inflammatory bowel disease (IBD) in a patient having IBD, wherein the method comprises (a) analyzing the level of IL8 in the patient; and (b) if the level of IL8 is above normal levels of IL8, then administering to the patient an initial dose of a SMAD7 antisense-oligonucleotide.
62. The method of clause 61, wherein the method further comprises: (c) analyzing the level of IL8 in the patient after said administering step; and (d) if the level of IL8 is above normal levels of IL8, then administering to the patient a subsequent dose that is greater than or equal to the initial dose, or, if the level of IL8 is below normal levels of IL8 then administering to the patient a subsequent dose that is equal to or smaller than the initial dose.
63. A method for treating or managing IBD in a patient having IBD, wherein the method comprises: (a) establishing a control level of IL8 for the patient; (b) administering to the patient an initial dose of a SMAD7 antisense-oligonucleotide; (c) analyzing the level of IL8 in the patient; and (d) if the level of IL8 is lower than the control level, then administering to the patient a subsequent dose that is the same as the initial dose or smaller than the initial dose, or, if the level of IL8 is unchanged or increased compared to the control level, then administering to the patient a subsequent dose that is the same as the initial dose or greater than the initial dose or terminating the treatment.
64. The method of clause 60 or 62, wherein if the level of IL8 is above normal levels of IL8, then administering to the patient a subsequent dose that is greater than the initial dose, or, if the level of IL8 is below normal levels of IL8 then administering to the patient a subsequent dose that is smaller than the initial dose.
65. The method of clause 62 or 63, wherein, if the subsequent dose is equal to or greater than the maximum tolerated dose (MTD), then terminating the treatment.
66. The method of clause 62 or 63, wherein the level of IL8 is analyzed at least 1 day, at least 3 days, at least 5 days, at least 1 week, at least 2 weeks, at least 3 weeks, at least 1 month, at least 2 months, at least 4 months, or at least 6 months after said administration step.
67. The method of clause 62 or 63, wherein the level of IL8 is analyzed immediately after said administration step.
68. The method of clause 62 or 63, wherein the level of IL8 is analyzed about 15 days or about 28 days after said administration step.
69. The methods of clause 60 or clause 61, wherein the normal levels of IL8 are median levels of IL8 in a healthy control group.
70. The methods of clause 63, wherein the control level of IL8 is a median level of IL8 in a healthy control group.
71. The method of clause 69 or 70, wherein the healthy control group and the patient having IBD are matched with respect to age, gender, ethnic origin, smoking habits, dietary habits, body-mass index (BMI), and/or exercise habits.
72. The method of clause 60 or clause 61, wherein normal levels of IL8 are about 15 pg/ml, about 16 pg/ml, about 17 pg/ml, or about 18 pg/ml.
73. The method of clause 63, wherein control level of IL8 is about 15 pg/ml, about 16 pg/ml, about 17 pg/ml, or about 18 pg/ml.
74. The method of clause 60, 61, or 63, wherein the initial dose is less than 100 mg/day, less than 90 mg/day, less than 80 mg/day, less than 70 mg/day, less than 60 mg/day, less than 50 mg/day, less than 40 mg/day or less than 30 mg/day.
75. The method of clause 60, 61, or 63, wherein the initial dose is at least 10 mg/day, at least 20 mg/day, at least 30 mg/day, at least 40 mg/day, at least 50 mg/day, at least 60 mg/day, at least 70 mg/day, at least 80 mg/day, or at least 90 mg/day.
76. The method of clause 60, 61, or 63, wherein the initial dose is about 10 mg/day, about 20 mg/day, about 30 mg/day, about 40 mg/day, about 50 mg/day, about 60 mg/day, about 70 mg/day, about 80 mg/day, about 90 mg/day, or about 100 mg/day.
77. The method of clause 60, 61, or 63, wherein the initial dose is 10 mg/day, 40 mg/day, 80 mg/day, or 160 mg/day.
78. The method of clause 60 or clause 62, wherein, if IL8 levels are above normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
79. The method of clause 63, wherein, if IL8 levels are above a control level, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
80. The method of clause 60 or clause 62, wherein, if IL8 levels are below normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
81. The method of clause 63, wherein, if IL8 levels are below a control level, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
82. The method of clause 60, 62, or 63, wherein the initial dose is between about 10 mg/day and 100 mg/day and the subsequent dose is between about 30 mg/day and 200 mg/day.
83. The method of clause 61, wherein the method further comprises: (c) analyzing the level of IL8 in the patient after said administering step; and (d) if the level of IL8 is lower after said administration step than the level of IL8 before said administration step, then administering to the patient a subsequent dose that is the same as the initial dose or smaller than the initial dose, or, if the level of IL8 is unchanged or increased after said administration step compared to the level of IL8 before said administration step, then administering to the patient a subsequent dose that is the same as the initial dose or greater than the initial dose or terminating the treatment.
84. The method of clause 63 or 83, wherein if the patient is in clinical remission and the level of IL8 is unchanged or increased after said administration step compared to the level of IL8 before said administration step, then terminating the treatment.
85. The method of clause 63 or 83, wherein, if the level of IL8 is at least 10%, at least 20%, at least 30%, at least 40%, at least 50%, or at least 70% decreased after said administration step compared to the level of IL8 before said administration step, then administering to the patient a subsequent dose that is the same as the initial dose or smaller than the initial dose.
86. The method of clause 63 or 83, further comprising determining that the patient having IBD has a greater than 20%, greater than 30%, greater than 40%, greater than 50%, greater than 60%, greater than 70%, greater than 80%, greater than 90% or greater than 100% chance of experiencing clinical remission of the IBD for a time period of at least 1 week, at least 2 weeks, at least 3 weeks, at least 4 weeks, at least 6 weeks or at least 8 weeks, if the level of IL8 after said administering step is at least 10%, at least 20%, at least 30%, at least 40%, at least 50%, at least 60%, or at least 70% decreased compared to the level of IL8 before said administration step.
87. The method of clause 86, wherein the clinical remission in the patient having IBD is indicated by a Crohn's Disease Activity Index (CDAI) <150.
88. The method of clause 86, wherein the clinical remission is observed about one week, about two weeks, or about three weeks after said administration step and maintained for a period of at least 1 week, at least 2 weeks, at least 3 weeks, at least 4 weeks, at least 6 weeks or at least 8 weeks.
89. The method of clause 86, wherein the patient having IBD had a CDAI of between about 220 and about 400 one week prior to said administration step.
90. The method of clause 60, 61, or 63, wherein the level of IL8 in the patient having IBD is determined in a sample obtained from the patient having IBD.
91. The method of clause 90, wherein the sample is a blood, serum or plasma sample.
92. The method of clause 60, 61, or 63, wherein the level of IL8 is determined by immunochemistry or by nucleotide analysis.
93. The method of clause 92, wherein the level of IL8 is determined by an enzyme-linked immunosorbent assay (ELISA).
94. The method of clause 60, 61, or 63, further comprising determining a level of one or more additional analytes in the patient having IBD.
95. The method of clause 94, wherein the one or more additional analytes comprise tumor necrosis factor alpha (TNFα) or C-reactive protein (CRP).
96. The method of clause 60, 61, or 63, wherein the IBD is Crohn's Disease (CD) or ulcerative colitis (UC).
97. The method of clause 96, wherein the patient having IBD is a steroid-dependent patient with active CD.
98. The method of clause 96, wherein the patient having IBD is a steroid-resistant patient with active CD.
99. The method of clause 60, 61, or 63, wherein the SMAD7 antisense-oligonucleotide is administered orally to the patient having IBD.
100. The method of clause 60, 61, or 63, wherein the SMAD7 antisense oligonucleotide targets region 108-128 of human SMAD7 (SEQ ID NO: 1).
101. The method of clause 60, 61, or 63, wherein the SMAD7 antisense oligonucleotide targets nucleotides 403, 233, 294, 295, 296, 298, 299 or 533 of human SMAD7 (SEQ ID NO: 1).
102. The method of clause 60, 61, or 63, wherein the SMAD7 antisense oligonucleotide comprises the nucleotide sequence of SEQ ID NO: 2 (5'-GTCGCCCCTTCTCCCCGCAGC-3').
103. The method of clause 60, 61, or 63, wherein the antisense oligonucleotide is an antisense oligonucleotide phosphorothioate against SMAD7 comprising the following sequence: 5'-GTXGCCCCTTCTCCCXGCAG-3' (SEQ ID NO: 3) wherein X is a nucleotide comprising 5-methyl-2'-deoxycytidine and wherein the internucleotide linkages are phosphorothioate linkages.
104. The method of clause 103, wherein the antisense oligonucleotide is an antisense oligonucleotide phosphorothioate against SMAD7 comprising the following sequence: 5'-GTXGCCCCTTCTCCCXGCAGC-3' (SEQ ID NO: 4) wherein X is a nucleotide comprising 5-methyl-2'-deoxycytidine and wherein the internucleotide linkages are phosphorothioate linkages.
105. A method for treating or managing IBD in a patient with IBD having above normal IL8 levels following administration of a dose of a SMAD7 antisense oligonucleotide, said method comprising administering to said patient a further dose of said oligonucleotide that is greater than or equal to the prior dose.
106. A method for treating or managing IBD in a patient with IBD having below normal IL8 levels following administration of a dose of SMAD7 antisense oligonucleotide, said method comprising administering to said patient a further dose of said oligonucleotide that is less than or equal to the prior dose.
107. A method of treating or managing IBD in a patient with IBD having above normal IL8 levels, said method comprising administering to said patient a dose of a SMAD7 antisense oligonucleotide.
108. The method of clause 107, wherein the administering is repeated until IL8 levels, CRP levels, or TNFα levels reach a normal level.
109. The method of clause 107, wherein the administering is repeated until the patient achieves a CDAI score of less than 150.
110. The method of clause 107, wherein the administering is repeated until the patient achieves clinical remission.
111. A method of monitoring the treatment or management of IBD in a patient with IBD, the method comprising analyzing IL8 levels in the patient following each SMAD7 antisense oligonucleotide administration, wherein the absence of a decrease in IL8 levels indicates that the treatment or management is not effective.
112. The method of clause 111, wherein IL8 levels are analyzed one time, two times, three times, four times, about five times, about 10 times, about 15 times, about 20 times, or about 30 times after each administration of SMAD7 antisense oligonucleotide.
113. The method of clause 111, wherein the IL8 levels are analyzed immediately after, about 1 hour after, about 3 hours after, about 6 hours after, about 12 hours after, about 1 day after, about 3 days after, about 1 week after, about 2 weeks after, and/or about 1 month after SMAD7 antisense oligonucleotide administration.
114. A method of treating or managing IBD in a patient with IBD having above normal levels of IL8, comprising increasing the amount of a SMAD7 antisense oligonucleotide administered to the patient until IL8 levels in the patient decrease.
115. The method of clause 114, wherein IL8 decreases to about a normal level of IL8 or a below normal level of IL8.
116. A method for treating or managing inflammatory bowel disease (IBD) in a patient having IBD, wherein the method comprises (a) administering to the patient an initial dose of a SMAD7 antisense-oligonucleotide; (b) analyzing the level of Tumor Necrosis Factor α (TNFα) in the patient; and (c) if the level of TNFα is above normal levels of TNFα, then administering to the patient a subsequent dose that is greater than or equal to the initial dose, or, if the level of TNFα is below normal levels of TNFα, then administering to the patient a subsequent dose that is equal to or smaller than the initial dose.
117. A method for treating or managing inflammatory bowel disease (IBD) in a patient having IBD, wherein the method comprises (a) analyzing the level of TNFα in the patient; and (b) if the level of TNFα is above normal levels of TNFα, then administering to the patient an initial dose of a SMAD7 antisense-oligonucleotide.
118. The method of clause 117, wherein the method further comprises: (c) analyzing the level of TNFα in the patient after said administering step; and (d) if the level of TNFα is above normal levels of TNFα then administering to the patient a subsequent dose that is greater than or equal to the initial dose, or, if the level of TNFα is below normal levels of TNFα then administering to the patient a subsequent dose that is equal to or smaller than the initial dose.
119. A method for treating or managing IBD in a patient having IBD, wherein the method comprises: (a) establishing a control level of TNFα for the patient; (b) administering to the patient an initial dose of a SMAD7 antisense-oligonucleotide; (c) analyzing the level of TNFα in the patient; and (d) if the level of TNFα is lower than the control level, then administering to the patient a subsequent dose that the same as the initial dose or smaller than the initial dose, or, if the level of TNFα is unchanged or increased compared to the control level, then administering to the patient a subsequent dose that is the same as the initial dose or greater than the initial dose or terminating the treatment.
120. The method of clause 116 or clause 118, wherein if the level of TNFα is above normal levels of TNFα, then administering to the patient a subsequent dose that is greater than the initial dose, or, if the level of TNFα is below normal levels of TNFα then administering to the patient a subsequent dose that is smaller than the initial dose.
121. The method of clause 118 or 119, wherein, if the subsequent dose is equal to or greater than the maximum tolerated dose (MTD), then terminating the treatment.
122. The method of clause 118 or 119, wherein the level of TNFα is analyzed at least 1 day, at least 3 days, at least 5 days, at least 1 week, at least 2 weeks, at least 3 weeks, at least 1 month, at least 2 months, at least 4 months, or at least 6 months after said administration step.
123. The method of clause 118 or 119, wherein the level of TNFα is analyzed immediately after said administration step.
124. The method of clause 118 or 119, wherein the level of TNFα is analyzed about 15 days or about 28 days after said administration step.
125. The methods of clause 116 or clause 117, wherein the normal levels of TNFα are median levels of TNFα in a healthy control group.
126. The methods of clause 119, wherein the control level of TNFα is a median level of TNFα in a healthy control group.
127. The method of clause 125 or 126, wherein the healthy control group and the patient having IBD are matched with respect to age, gender, ethnic origin, smoking habits, dietary habits, body-mass index (BMI), and/or exercise habits.
128. The method of clause 116 or clause 117, wherein normal levels of TNFα are about 10 pg/ml, about 15 pg/ml, about 16 pg/ml, about 17 pg/ml, or about 18 pg/ml.
129. The method of clause 119, wherein control level of TNFα is about 10 pg/ml, about 15 pg/ml, about 16 pg/ml, about 17 pg/ml, or about 18 pg/ml.
130. The method of clause 116, 117, or 119, wherein the initial dose is less than 100 mg/day, less than 90 mg/day, less than 80 mg/day, less than 70 mg/day, less than 60 mg/day, less than 50 mg/day, less than 40 mg/day or less than 30 mg/day.
131. The method of clause 116, 117, or 119, wherein the initial dose is at least 10 mg/day, at least 20 mg/day, at least 30 mg/day, at least 40 mg/day, at least 50 mg/day, at least 60 mg/day, at least 70 mg/day, at least 80 mg/day, or at least 90 mg/day.
132. The method of clause 116, 117, or 119, wherein the initial dose is about 10 mg/day, about 20 mg/day, about 30 mg/day, about 40 mg/day, about 50 mg/day, about 60 mg/day, about 70 mg/day, about 80 mg/day, about 90 mg/day, or about 100 mg/day.
133. The method of clause 116, 117, or 119, wherein the initial dose is 10 mg/day, 40 mg/day, 80 mg/day, or 160 mg/day.
134. The method of clause 116 or clause 118, wherein, if TNFα levels are above normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
135. The method of clause 119, wherein, if TNFα levels are above a control level, the subsequent dose is at least about 10 mg/day, at least about 20 mg/day, at least about 30 mg/day, at least about 40 mg/day, at least about 50 mg/day, at least about 60 mg/day, at least about 70 mg/day, at least about 80 mg/day, at least about 90 mg/day, at least about 100 mg/day, at least about 110 mg/day, at least about 120 mg/day, at least about 130 mg/day, at least about 140 mg/day, at least about 150 mg/day, or at least about 160 mg/day greater than the initial dose.
136. The method of clause 116 or clause 118, wherein, if TNFα levels are below normal levels, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
137. The method of clause 119, wherein, if TNFα levels are below a control level, the subsequent dose is at least about 10 mg/day, at least about 20 mg/day, at least about 30 mg/day, at least about 40 mg/day, at least about 50 mg/day, at least about 60 mg/day, at least about 70 mg/day, or at least about 80 mg/day smaller than the initial dose.
138. The method of clause 116, 118, or 119, wherein the initial dose is between about 10 mg/day and 100 mg/day and the subsequent dose is between about 30 mg/day and 200 mg/day.
139. The method of clause 117, wherein the method further comprises: (c) analyzing the level of TNFα in the patient after said administering step; and (d) if the level of TNFα is lower after said administration step than the level of TNFα before said administration step, then administering to the patient a subsequent dose that is the same as the initial dose or smaller than the initial dose, or, if the level of TNFα is unchanged or increased after said administration step compared to the level of TNFα before said administration step, then administering to the patient a subsequent dose that is the same as the initial dose or greater than the initial dose or terminating the treatment.
140. The method of clause 119 or 139, wherein if the patient is in clinical remission and the level of TNFα is unchanged or increased after said administration step compared to the level of TNFα before said administration step, then terminating the treatment.
141. The method of clause 119 or 139, wherein, if the level of TNFα is at least 10%, at least 20%, at least 30%, at least 40%, at least 50%, or at least 70% decreased after said administration step compared to the level of TNFα before said administration step, then administering to the patient a subsequent dose that is the same as the initial dose or smaller than the initial dose.
142. The method of clause 119 or 139, further comprising determining that the patient having IBD has a greater than 20%, greater than 30%, greater than 40%, greater than 50%, greater than 60%, greater than 70%, greater than 80%, greater than 90% or greater than 100% chance of experiencing clinical remission of the IBD for a time period of at least 1 week, at least 2 weeks, at least 3 weeks, at least 4 weeks, at least 6 weeks or at least 8 weeks, if the level of TNFα after said administering step is at least 10%, at least 20%, at least 30%, at least 40%, at least 50%, at least 60%, or at least 70% decreased compared to the level of TNFα before said administration step.
143. The method of clause 142, wherein the clinical remission in the patient having IBD is indicated by a Crohn's Disease Activity Index (CDAI) <150.
144. The method of clause 142, wherein the clinical remission is observed about one week, about two weeks, or about three weeks after said administration step and maintained for a period of at least 1 week, at least 2 weeks, at least 3 weeks, at least 4 weeks, at least 6 weeks or at least 8 weeks.
145. The method of clause 142, wherein the patient having IBD had a CDAI of between about 220 and about 400 one week prior to said administration step.
146. The method of clause 116, 117, or 119, wherein the level of TNFα in the patient having IBD is determined in a sample obtained from the patient having IBD.
147. The method of clause 146, wherein the sample is a blood, serum or plasma sample.
148. The method of clause 116, 117, or 119, wherein the level of TNFα is determined by immunochemistry or by nucleotide analysis.
149. The method of clause 148, wherein the level of TNFα is determined by an enzyme-linked immunosorbent assay (ELISA).
150. The method of clause 116, 117, or 119, further comprising determining a level of one or more additional analytes in the patient having IBD.
151. The method of clause 150, wherein the one or more additional analytes comprise Interleukin-8 (IL8) or C-reactive protein (CRP).
152. The method of clause 116, 117, or 119, wherein the IBD is Crohn's Disease (CD) or ulcerative colitis (UC).
153. The method of clause 152, wherein the patient having IBD is a steroid-dependent patient with active CD.
154. The method of clause 152, wherein the patient having IBD is a steroid-resistant patient with active CD.
155. The method of clause 116, 117, or 119, wherein the SMAD7 antisense-oligonucleotide is administered orally to the patient having IBD.
156. The method of clause 116, 117, or 119, wherein the SMAD7 antisense oligonucleotide targets region 108-128 of human SMAD7 (SEQ ID NO: 1).
157. The method of clause 116, 117, or 119, wherein the SMAD7 antisense oligonucleotide targets nucleotides 403, 233, 294, 295, 296, 298, 299 or 533 of human SMAD7 (SEQ ID NO: 1).
158. The method of clause 116, 117, or 119, wherein the SMAD7 antisense oligonucleotide comprises the nucleotide sequence of SEQ ID NO: 2 (5'-GTCGCCCCTTCTCCCCGCAGC-3').
159. The method of clause 116, 117, or 119, wherein the antisense oligonucleotide is an antisense oligonucleotide phosphorothioate against SMAD7 comprising the following sequence: 5'-GTXGCCCCTTCTCCCXGCAG-3' (SEQ ID NO: 3) wherein X is a nucleotide comprising 5-methyl-2'-deoxycytidine and wherein the internucleotide linkages are phosphorothioate linkages.
160. The method of clause 159, wherein the antisense oligonucleotide is an antisense oligonucleotide phosphorothioate against SMAD7 comprising the following sequence: 5'-GTXGCCCCTTCTCCCXGCAGC-3' (SEQ ID NO: 4) wherein X is a nucleotide comprising 5-methyl-2'-deoxycytidine and wherein the internucleotide linkages are phosphorothioate linkages.
161. A method for treating or managing IBD in a patient with IBD having above normal TNFα levels following administration of a dose of a SMAD7 antisense oligonucleotide, said method comprising administering to said patient a further dose of said oligonucleotide that is greater than or equal to the prior dose.
162. A method for treating or managing IBD in a patient with IBD having below normal TNFα levels following administration of a dose of SMAD7 antisense oligonucleotide, said method comprising administering to said patient a further dose of said oligonucleotide that is less than or equal to the prior dose.
163. A method of treating or managing IBD in a patient with IBD having above normal TNFα levels, said method comprising administering to said patient a dose of a SMAD7 antisense oligonucleotide.
164. The method of clause 163, wherein the administering is repeated until IL8 levels, CRP levels, or TNFα levels reach a normal level.
165. The method of clause 163, wherein the administering is repeated until the patient achieves a CDAI score of less than 150.
166. The method of clause 163, wherein the administering is repeated until the patient achieves clinical remission.
167. A method of monitoring the treatment or management of IBD in a patient with IBD, the method comprising analyzing TNFα levels in the patient following each SMAD7 antisense oligonucleotide administration, wherein the absence of a decrease in TNFα levels indicates that the treatment or management is not effective.
168. The method of clause 167, wherein TNFα levels are analyzed one time, two times, three times, four times, about five times, about 10 times, about 15 times, about 20 times, or about 30 times after each administration of SMAD7 antisense oligonucleotide.
169. The method of clause 167, wherein the TNFα levels are analyzed immediately after, about 1 hour after, about 3 hours after, about 6 hours after, about 12 hours after, about 1 day after, about 3 days after, about 1 week after, about 2 weeks after, and/or about 1 month after SMAD7 antisense oligonucleotide administration.
170. A method of treating or managing IBD in a patient with IBD having above normal levels of TNFα, comprising increasing the amount of a SMAD7 antisense oligonucleotide administered to the patient until TNFα levels in the patient decrease.
171. The method of clause 170, wherein TNFα decreases to about a normal level of TNFα or a below normal level of TNFα.

## Claims

1. A SMAD7 antisense-oligonucleotide for use in a method for treating or managing Inflammatory Bowel Disease (IBD) in a patient having IBD, wherein the method comprises the steps of:
(a) analyzing the level of interleukin 8 (IL8) in the patient; (b) if the level of the IL8 is above normal or control levels of IL8, then administering to the patient an initial dose of a SMAD7 antisense-oligonucleotide; (c) analyzing the level of the IL8, and if the level of the IL8 is above normal or control levels of IL8, then administering to the patient a subsequent dose that is greater than or equal to the initial dose, or, if the level of IL8 is below normal or control levels of IL8, then administering to the patient a subsequent dose that is equal to or smaller than the initial dose;
or,
(a) administering to the patient an initial dose of a SMAD7 antisense-oligonucleotide; (b) analyzing the level of the IL8 in the patient; and (c) if the level of the IL8 is above normal or control levels of IL8, then administering to the patient a subsequent dose that is greater than or equal to the initial dose, or, if the level of IL8 is below normal or control levels of IL8, then administering to the patient a subsequent dose that is equal to or smaller than the initial dose;
or,
(a) analyzing the level of IL8 in the patient after administering an initial dose of a SMAD7 antisense-oligonucleotide; (b) if the level of the IL8 is lower after said administration step than the level of IL8 before said administration step, then administering to the patient a subsequent dose that is the same as the initial dose or smaller than the initial dose, or, if the level of the IL8 is unchanged or increased after said administration step compared to the level of the IL8 before said administration step, then administering to the patient a subsequent dose that is the same as the initial dose or greater than the initial dose or terminating treatment.

2. A SMAD7 antisense-oligonucleotide for use in a method for treating or managing Inflammatory Bowel Disease (IBD) in a patient having IBD, wherein the method comprises the steps of:
(a) analyzing the level of Tumor Necrosis Factor α (TNFα) in the patient; (b) if the level of the TNFα is above normal or control levels of TNFα, then administering to the patient an initial dose of a SMAD7 antisense-oligonucleotide; (c) analyzing the level of the TNFα, and if the level of the TNFα is above normal or control levels of TNFα, then administering to the patient a subsequent dose that is greater than or equal to the initial dose, or, if the level of TNFα is below normal or control levels of TNFα, then administering to the patient a subsequent dose that is equal to or smaller than the initial dose;
or,
(a) administering to the patient an initial dose of a SMAD7 antisense-oligonucleotide; (b) analyzing the level of the TNFα in the patient; and (c) if the level of the TNFα is above normal or control levels of TNFα, then administering to the patient a subsequent dose that is greater than or equal to the initial dose, or, if the level of TNFα is below normal or control levels of TNFα, then administering to the patient a subsequent dose that is equal to or smaller than the initial dose;
or,
(a) analyzing the level of TNFα in the patient after administering an initial dose of a SMAD7 antisense-oligonucleotide; (b) if the level of the TNFα is lower after said administration step than the level of TNFα before said administration step, then administering to the patient a subsequent dose that is the same as the initial dose or smaller than the initial dose, or, if the level of the TNFα is unchanged or increased after said administration step compared to the level of the TNFα before said administration step, then administering to the patient a subsequent dose that is the same as the initial dose or greater than the initial dose or terminating treatment.

3. The SMAD7 antisense-oligonucleotide for use according to claim 1 or 2, wherein if the patient is in clinical remission and the level of IL8 or TNFα, respectively, is unchanged or increased after said initial administration step compared to the level of IL8 or TNFα, respectively, before said initial administration step, then terminating the treatment.

4. The SMAD7 antisense-oligonucleotide for use according to claim 1 or 2, wherein if the level of IL8 or TNFα, respectively, is at least 10%, at least 20%, at least 30%, at least 40%, at least 50%, or at least 70% decreased after said administration step compared to the level of IL8 or TNFα, respectively, before said administration step, then administering to the patient a subsequent dose that is the same as the initial dose or smaller than the initial dose.

5. The SMAD7 antisense-oligonucleotide for use according to claim 1 or 2, wherein if the level of IL8 or TNFα, respectively, is above normal level of IL8 or TNFα, respectively, then administering to the patient a subsequent dose that is greater than the initial dose, or if the level of IL8 or TNFα, respectively, is below normal level of IL8 or TNFα, respectively, then administering to the patient a subsequent dose that is smaller than the initial dose.

6. The SMAD7 antisense-oligonucleotide for use according to claims 1-5, wherein the level of IL8 or TNFα, respectively, is analyzed at least 1 day, at least 3 days, at least 5 days, at least 1 week, at least 2 weeks, at least 3 weeks, at least 1 month, at least 2 months, at least 4 months, or at least 6 months after said administration step.

7. The SMAD7 antisense-oligonucleotide for use according to claims 1-6, wherein the initial dose is 10 mg/day, 40 mg/day, 80 mg/day, or 160 mg/day.

8. The SMAD7 antisense-oligonucleotide for use according to claims 1-7, wherein the level of IL8 or TNFα, respectively, in the patient having IBD is determined in a sample obtained from the patient having IBD.

9. The SMAD7 antisense-oligonucleotide for use according to claim 8, wherein the sample is a blood, serum, or plasma sample.

10. The SMAD7 antisense-oligonucleotide for use according to claims 1-9, further comprising determining a level of C-reactive protein (CRP) in the patient having IBD.

11. The SMAD7 antisense-oligonucleotide for use according to claims 1-10, wherein the IBD is Crohn's Disease (CD) or ulcerative colitis (UC); or wherein the patient having IBD is a steroid-dependent patient with active CD; or wherein the patient having IBD is a steroid-resistant patient with active CD.

12. The SMAD7 antisense-oligonucleotide for use according to claims 1-11, wherein the antisense oligonucleotide is an antisense oligonucleotide phosphorothioate against SMAD7 comprising the following sequence: 5' - GTXGCCCCTTCTCCCXGCAGC - 3' (SEQ ID NO: 4), wherein X is a nucleotide comprising 5-methyl-2'-deoxycytidine and wherein the internucleotide linkages are phosphorothioate linkages.

13. The SMAD7 antisense-oligonucleotide for use according to claims 1-12, wherein the SMAD7 antisense oligonucleotide is administered orally to the patient having IBD.
